(19) Europäisches Patentamt
European Patent Office
Office européen des brevets

(11) Numéro de publication : **0 004 512**
**B1**

(12) # FASCICULE DE BREVET EUROPÉEN

(45) Date de publication du fascicule du brevet : **18.01.84**

(51) Int. Cl.[3] : **C 07 H 15/04**, A 61 K 31/70, A 61 K 9/00

(21) Numéro de dépôt : **79400183.4**

(22) Date de dépôt : **20.03.79**

(54) **Composés esters de muramyl-peptide et leurs applications dans les compositions pharmaceutiques et réactifs de laboratoire.**

(30) Priorité : **20.03.78 FR 7808049**
**23.11.78 FR 7833126**

(43) Date de publication de la demande :
**03.10.79 Bulletin 79/20**

(45) Mention de la délivrance du brevet :
**18.01.84 Bulletin 84/03**

(84) Etats contractants désignés :
**BE CH DE GB IT LU NL SE**

(56) Documents cités :
**DE-A- 2 710 454**
**FR-A- 2 325 387**
**FR-A- 2 349 600**
**FR-A- 2 361 902**

(73) Titulaire : **ANVAR Agence Nationale de Valorisation de la Recherche**
**13, rue Madeleine Michelis**
**F-92522 Neuilly-sur-Seine (FR)**

(72) Inventeur : **Lefrancier, Pierre**
**20, rue des Bleuets**
**F-91440 Bures sur Yvette (FR)**
Inventeur : **Parant, Monique**
**33, rue Croulebarbe**
**F-75013 Paris (FR)**
Inventeur : **Audibert, Françoise**
**44, rue Ybry**
**F-92200 Neuilly sur Seine (FR)**
Inventeur : **Sache, Edgar**
**115, rue Charles de Gaulle**
**F-91440 Bures sur Yvette (FR)**
Inventeur : **Chedid, Louis**
**16-18, rue Gaston de Caillavet**
**F-75015 Paris (FR)**
Inventeur : **Choay, Jean**
**130, Faubourg Saint-Honoré**
**F-75008 Paris (FR)**
Inventeur : **Lederer, Edgar**
**9, Boulevard Colbert**
**F-92330 Sceaux (FR)**

(74) Mandataire : **Gutmann, Ernest et al**
**Cabinet Plasseraud 84, rue d'Amsterdam**
**F-75009 Paris (FR)**

**EP 0 004 512 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Composés esters de muramyl-peptide et leurs applications dans les compositions pharmaceutiques et réactifs de laboratoire

L'invention est relative à des produits nouveaux doués de propriétés biologiques et pharmacologiques de grande valeur, lesquelles peuvent être mises à profil soit pour la constitution de réactifs de laboratoire standardisés pour l'étude comparative des propriétés biologiques semblables d'autres composés, soit pour la constitution de médicaments nouveaux à usage humain ou vétérinaire.

L'invention concerne plus particulièrement des produits nouveaux capables de modifier les réponses immunitaires chez les êtres vivants à sang chaud. Plus spécifiquement encore, elle concerne des produits nouveaux capables de stimuler des réponses immunitaires faisant intervenir au moins l'un et de préférence l'ensemble des mécanismes consituant les réponses immunitaires à support humoral (opsonines, anticorps) ou à support cellulaire. Ces mécanismes peuvent être spécifiques (leur mise en jeu dépendant d'une vaccination préalable par un mélange antigénique donné, et plus spécialement provenant d'agents pathogènes) ou non spécifiques (tels qu'ils sont induits par des agents comme le BCG, les corynebactéries ou les endotoxines).

On sait que les organismes des êtres vivants à sang chaud comportent plusieurs types de cellules constituant ce qui pourrait être appelé plusieurs lignes de défense à l'égard des différentes agressions dont ces organismes peuvent être l'objet.

La première ligne de défense met en jeu, comme il est bien connu, les leucocytes et des macrophages dans le sang circulant, capables, lorsqu'ils rencontrent un agent étranger tel qu'un antigène, de le phagocyter et souvent de la détruire. Cette phagocytose peut être favorisée notamment par des consituants non spécifiques du sérum (opsonines), voire même par des interventions extérieures visant à une activation des macrophages.

Une seconde ligne de défense de l'organisme met en jeu des mécanismes déclenchés par les constituants antigéniques de l'agent étranger. Ces mécanismes immunitaires font intervenir des différenciations cellulaires dans au moins l'une des deux directions principales suivantes.

Un premier type de mécanisme immunitaire met en effet en jeu des cellules spécialisées (lymphocytes B) qui sont des précurseurs des cellules qui sécrètent des immunoglobulines ou anticorps. Ces immunoglobulines ou anticorps jouent un rôle important dans la lutte contre les infections causées par des bactéries ou autres micro-organismes qui se répliquent dans les fluides humoraux, ou encore pour neutraliser les toxines ou analogues. A ce type d'action, spécifique de l'antigène considéré, s'ajoute une action indirecte par des médiateurs circulants, et qui se traduit par une activation des cellules responsables de la phagocytose, renforçant ainsi les défenses non spécifiques de l'organisme.

Le second type de mécanisme immunitaire fait intervenir des cellules spécifiquement sensibilisées appartenant à la lignée lymphocytaire (cellules T), lesquelles interagissent sur les cellules B indiquées précédemment, ou sur les macrophages conduisant à l'activation de ces derniers ou d'autres cellules non spécifiques.

Face à ces divers mécanismes schématiquement présentés ci-dessus, on peut distinguer deux sortes d'infections suivant la façon dont elles se développent et les mécanismes sollicités pour les combattre. Ainsi, pour certaines infections, une simple phagocytose, favorisée éventuellement par des facteurs humoraux spécifiques ou non, permet la destruction des agents infectieux. Pour d'autres types d'infection, la phagocytose ne suffit pas. L'agent infectieux phagocyté n'est pas détruit et même continue de se répliquer à l'intérieur de la cellule phagocytante, entraînant au contraire la destruction de cette dernière. Dans ce cas, pour empêcher la multiplication intracellulaire des agents infectieux, il est nécessaire d'« activer » les cellules phagocytantes suivant les processus indiqués plus haut.

Lorsque l'on souhaite étudier les propriétés anti-infectieuses de nouveaux produits, les *Klebsiella*, comme souches infectantes, à réplication extracellulaire sont particulièrement représentatives. Ces bactéries présentent en effet une capsule de grande dimension qui ne permet une phagocytose efficace que par les macrophages. Ce mécanisme mettant en œuvre des agents non spécifiques, les résultats obtenus avec les *Klebsiella* peuvent être étendus aux micro-organismes qui se répliquent de la même façon.

Un protocole expérimental a été étudié par Chedid L. et col. dans « Proc. Natl. Acad. Sci. USA, 1977, 74 : 2089 », qui permet de constater l'action stimulante ou non de substances étudiées à l'égard des défenses immunitaires selon qu'elles protègent ou non des souris auxquelles elles sont injectées, les souris étant inoculées par une dose de *Klebsiella Pneumoniae* qui entraîne la mort de la quasi-totalité des témoins.

Lorsque l'on veut étudier les effets de telles substances sur les infections dont les agents se multiplient de façon intracellulaire, les *Listeria* sont parmi les micro-organismes les plus utilisés, et notamment les *Listeria monocytogènes.* Ceux-ci sont à la base de tests biologiques devenus classiques, aussi bien que l'étude des réactions spécifiques que non spécifiques.

A titre d'exemple, on peut citer le protocole expérimental décrit par Medina, Vas and Robson (J. Immunol., 1975, *114,* 1720), qui permet de constater l'action stimulante ou non de substances étudiées à l'égard des défenses immunitaires, selon qu'elles protègent ou non des souris auxquelles elles sont injectées contre une dose de *Listeria monocytogène* qui entraîne la mort de la quasi-totalité des témoins, ou selon qu'elles entraînent ou non la destruction à court terme desdits micro-organismes.

Il est aujourd'hui de plus en plus affirmé que l'immunité à médiation cellulaire constitue un système complexe de défense des organismes intervenant dans de nombreuses situations, non seulement vis-à-vis des micro-organismes intracellulaires mais également vis-à-vis de la croissance néoplasique et la multiplication de nombreux agents fongiques, parasitaires, etc. ; c'est ce système qui également est en cause dans le rejet des greffes et de nombreux processus auto-immuns.

D'une façon générale, on peut faire référence, en ce qui concerne l'ensemble des problèmes évoqués ci-dessus, par exemple aux articles de Priscilla A. CAMPBELL, intitulés « Immunocompetent Cells in Resistance to Bacterial Infections » (Cellules immunocompétentes pour la résistance aux infections bactériennes), parus dans « Bacteriological Reviews », June 1976, p. 284-313, de G.B. MACKANESS, intitulé « Cellular Immunity » (Immunité cellulaire), paru dans les Annales de l'INSTITUT PASTEUR, 1971, 120, 428-437, de G.H. WERNER et coll., intitulé « Toxicological Aspects of Immunopotentiation by Adjuvants and Immunostimulating Substances » (Aspects toxicologiques de l'immunopotentiation par des substances adjuvantes et immunostimulantes), paru dans le Bulletin de l'INSTITUT PASTEUR, volume 75, n° 1 de janvier 1977, et dans un rapport d'un groupe scientifique de l'Organisation Mondiale de la Santé, intitulé « Réponses Immunitaires à Support Cellulaire », paru dans Org. mond. Santé, Sér. Rapp. techn., 1969, n° 423.

Il résulte de ce qui précède que la mise à la disposition des spécialistes, notamment du biologiste et du clinicien, des compositions ou produits capables de stimuler les défenses immunitaires des types sus-indiqués peut revêtir une importance capitale tant pour l'étude d'autres substances au niveau de la recherche, tant fondamentale qu'appliquée, que dans le domaine de la thérapeutique humaine ou vétérinaire.

On connaît déjà certains agents capables de stimuler de façon non spécifique ces différentes réponses immunitaires, tant à médiation cellulaire qu'à médiation humorale. Il en est par exemple ainsi des lipopolysaccharides bactériens (LPS), connus pour leur activité protectrice à l'égard des infections humorales ou cellulaires. Les LPS possèdent en outre des propriétés d'adjuvants immunologiques non spécifiques, en ce qu'ils favorisent une augmentation du taux de synthèse d'anticorps spécifiques par un organisme soumis à l'agression d'antigènes, de quelque nature qu'ils soient.

Il est connu de même que des mycobactéries, et plus particulièrement le « bacille de Calmette-Guérin » (BCG), possèdent des propriétés immunostimulantes non spécifiques puissantes.

Cependant, il ne peut être question d'envisager l'utilisation des LPS en thérapeutique, compte tenu de leur extrême toxicité qui est bien connue. Le BCG lui-même n'est pas exempt de nombreux inconvénients, lesquels peuvent être mis en évidence par exemple dans l'animal, notamment au niveau de l'augmentation de la sensibilité de l'hôte aux endotoxines, de la production d'une hypersensibilité à la tuberculine, de l'induction de granulomes, d'une hyperplasie du tissu lymphoïde et, notamment chez le rat, de polyarthrites.

On sait que de nombreux chercheurs se sont attachés à l'étude d'extraits susceptibles d'être obtenus à partir des mycobactéries, dans le but d'obtenir des agents purifiés ou détoxifiés retenant les propriétés biologiques de valeur du BCG ou des LPS, tout en étant débarrassés des inconvénients susmentionnés. Le développement de ces recherches a conduit à des petites molécules, représentant en elles-mêmes un apport considérable à l'arsenal des substances dont disposent le chercheur et le clinicien, qui sont maintenant accessibles à la synthèse chimique, qui sont pratiquement dépourvues de toxicité et dont l'activité d'adjuvant immunologique, très puissante, se manifeste même lorsqu'elles sont administrées à un hôte, en l'absence de tout support huileux, comme il était nécessaire en ce qui concerne plus particulièrement les fractions obtenues par extraction, notamment à partir des mycobactéries.

Les petites molécules susdites sont, comme il est maintenant bien connu, constituées par des N-acyl-muramyl-peptides ou certains de leurs dérivés de substitution, caractérisés par un groupe N-acyl-muramique auquel est rattachée une chaîne peptidique comportant un premier résidu aminoacyle directement lié à l'acide N-acyl-muramique, constitué par un résidu glycyle ou dérivé d'un autre acide aminé lévogyre, de préférence un résidu L-alanyle ou L-séryle, et un second groupe aminoacyle, lié au premier, dérivé de l'acide D-glutamique (DE 2 450 355).

Des dérivés caractérisés par une certaine analogie de structure, plus particulièrement des N-acyl-Normuramyl-dipeptides, dans lesquels le second résidu aminoacyle de la chaîne dipeptidique est constitué par la D-isoasparagine ou la D-isoglutamine, et présentés comme ayant les propriétés adjuvantes, sont décrits dans la demande de brevet français publiée FR-A 2 349 600.

De même, la demande de brevet français FR-A 2 361 902 décrit des composés semblables, dans lesquels le second résidu aminoacyle de la chaîne dipeptidique est constitué par l'acide glutamique, dont les fonctions carboxyliques peuvent être substituées par diverses fonctions, parmi lesquelles des fonctions ester, plus particulièrement celles résultant de l'estérification des carboxyles du groupe glutamyle par des alcanols inférieurs. Les dérivés résultant de la substitution de diverses fonctions, y compris les esters des susdits N-acyl-muramyl-peptides, dans lesquels le groupe acyle est un acétyle, n'entrent pas dans le champ de la formule générale qui figure dans la demande FR-A 2 361 902.

Les produits les plus actifs en tant qu'adjuvants immunologiques sont constitués par les N-acétyl-muramyl-peptides, dont le premier résidu aminoacyle est un L-alanyle ou L-séryle et le second un résidu D-glutamyle dont la fonction carboxyle en $\alpha$ peut être soit libre, soit estérifiée ou amidée (le groupe amide pouvant lui-même porter des groupes de substitution) et dont la fonction carboxyle en $\gamma$ peut aussi être

soit libre, soit amidée ou estérifiée, soit encore engagée dans une chaîne peptidique plus longue. Le produit le plus représentatif de la série des N-acyl-muramyl-peptides est constitué par la N-acétyl-muramyl-L-alanyl-D-isoglutamine (MDP). Des recherches diverses ont cependant montré que l'activité d'adjuvant immunologique pouvait être maintenue, dans une mesure plus ou moins importante, par l'introduction de substitutions diverses dans le groupe muramyle ou par le remplacement du premier résidu aminoacyle par d'autres dérivés d'acides aminés de série L différents.

Il a déjà été constaté pour certains des muramyl-peptides du genre en question, et plus particulièrement en ce qui concerne la N-acétyl-muramyl-L-alanyl-D-isoglutamine déjà citée (MDP) ou le dérivé correspondant non amidé, à savoir l'acide N-acétyl-muramyl-L-alanyl-D-glutamique (MDPA) ou encore certains des α-esters ou diesters du MDPA, dont les fonctions ester comportent au plus 3 atomes de carbone, qu'ils possédaient en outre des propriétés anti-infectieuses déjà très importantes, qui se manifestent plus particulièrement sur les micro-organismes à multiplication humorale *(Klebsiella)* (DE-A 2 710 454).

On sait cependant que beaucoup de dérivés possibles du type muramyl-peptide, ne portant au plus que des substituants de faible masse moléculaire (comprenant par exemple au plus trois atomes de carbone) sont en fait totalement dépourvues de propriétés anti-infectieuses. C'est ce dont témoigne d'ailleurs l'article de L. CHEDID et Coll. publié dans « Proc. Natl. Acad. Sci. USA », vol. 74, No. 5, pages 2089-2093 de mai 1977, intitulé « Enhancement of nonspecific immunity to *Klebsiella pneumoniae* infection by a synthetic immuno-adjuvant (N-acetylmuramyl-L-alanyl-D-isoglutamine) and several analogs ».

L'invention découle de la découverte que l'introduction d'une chaîne lipophile (c'est-à-dire, une chaîne hydrocarbonnée essentiellement non polaire) sur la fonction γ-carboxylique du résidu glutamyle conduisait à l'obtention d'une nouvelle catégorie de dérivés du type muramyl-peptide caractérisés notamment par une puissante action anti-infectieuse. On remarquera que la fixation de la chaîne lipophile sur la chaîne peptidique, qui caractérise les nouveaux produits selon l'invention, va tout à fait à l'encontre de ce que l'on peut observer dans les produits naturels adjuvants hydrosolubles qui peuvent être obtenus à partir des parois cellulaires de micro-organismes, tels que les mycobactéries, et qui comportent dans leur structure au moins un fragment de peptidoglycane. En effet, certains de ces produits naturels peuvent comporter des chaînes lipophiles fixées indirectement sur certains des motifs saccharidiques du peptidoglycane, mais n'ont jamais été trouvés sur les chaînes peptidiques de ce dernier.

Les différentes propriétés des produits selon l'invention, et plus particulièrement leurs propriétés anti-infectieuses, sont en outre d'autant plus inattendues que les γ-monoesters à courte chaîne du MDPA ne possèdent pas des propriétés anti-infectieuses se manifestant au niveau du mécanisme de défense immunitaire humoral.

Des produits selon l'invention témoignent en outre d'un progrès nouveau vis-à-vis des produits déjà connus, en ce qu'ils se révèlent capables non seulement d'exercer une action de stimulation non spécifique au niveau des défenses immunitaires humorales, mais aussi de l'immunité à support cellulaire.

Les nouveaux composés selon l'invention répondent à la formule générale

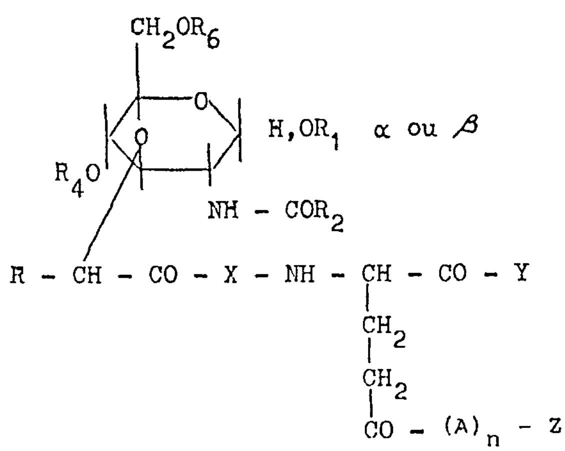

(I)

dans laquelle les substituants R, $R_1$, $R_2$, $R_4$, $R_6$, X, Y et Z ont l'une des significations suivantes :

— R est de préférence soit un atome d'hydrogène, soit un groupe méthyle,

— $R_1$ est de préférence un atome d'hydrogène, un groupe alcoyle ayant au plus 4 atomes de carbone, un groupe aryle ou alcoyl-aryle simple ou substitué comprenant au plus 10 atomes de carbone,

— $R_2$ est un groupe méthyle,

— $R_4$ est un atome d'hydrogène, un radical acyle comprenant au plus 4 atomes de carbone,

— $R_6$ est un atome d'hydrogène, ou un radical acyle contenant de 1 à 90 atomes de carbone, et pouvant en outre porter des groupes fonctionnels : hydroxyle, carboxyle, carbonyle, amino, cyclopropane, alcoxyle, de préférence méthoxyle,

— X est un résidu aminoacyle du groupe comprenant : L-alanyle, L-arginyle, L-asparagyle, L-aspartyle, L-cystéinyle, L-glutaminyle, L-glutamyle, glycyle, L-histidyle, L-hydroxyprolyle, L-isoleucyle, L-leucyle, L-lysyle, L-méthionyle, L-ornithyle, L-phénylalanyle, L-prolyle, L-séryle, L-thréonyle, L-tryptophanyle, L-tyrosyle et L-valyle,

— Y est soit —OH, soit un radical alcoxy comprenant de 1 à 10 atomes de carbone, soit $—NH_2$, les hydrogènes du groupe amino pouvant être substitués par des restes alcoyle de 1 à 10 atomes de carbone, soit un résidu aminoacyle,

— A est un résidu aminoacyle du groupe indiqué ci-dessus pour X, ou, pour le dernier de la chaîne peptidique, un résidu d'aminoalcool ($—NH—\underset{|}{C}H—CH_2—O—$) correspondant à ces aminoacyles ($—NH—\underset{|}{C}H—CO—$), étant entendu que les groupes A présents dans un même composé peuvent être identiques ou différents, n ne représentant que le nombre total de groupes A dans ce composé,

— n est nul ou 1, 2 ou 3,

— Z est un groupe —OR', —NHR', $—OCH_2—CH_2O—COR'$ ou $—OCH_2—CHOH—CH_2O—COR'$ lorsque le dernier résidu A de la chaîne peptidique est un aminoacyle ou un groupe COR' lorsque ce dernier résidu A est un aminoalcool, dans lequel R' est un alcoyle linéaire ou ramifié, saturé ou non, et peut contenir des groupes fonctionnels hydroxyle, carbonyle, carboxyle, cyclopropane, aryle, ce dernier éventuellement substitué, ce groupe comprenant au moins 4 carbones et pouvant renfermer jusqu'à 90 atomes de carbone, sous réserve que, lorsque R est de l'hydrogène, le groupement $(A)_n—Z$ soit un groupe $—NH—CH(CH_3)—COO—CH_2—CHOH—CH_2—O—R°$, avec R° correspondant à un acide mycolique comprenant de 80 à 90 atomes de carbone.

Dans cette formule, le deuxième groupe aminoacyle de la chaîne peptidique liée au groupe de type muramyle est le résidu D-glutamyle. Le premier groupe aminoacyle (désigné par X) peut, par contre, être choisi parmi les différents groupes aminoacyles mentionnés ci-dessus. Parmi les composés de formule I, on préfère ceux dans lesquels le premier groupe aminoacyle est L-alanyle. Un deuxième type de composés préférés est celui dans lequel cet aminoacyle est L-séryle. Un autre type de composés préférés est celui dans lequel cet aminoacyle est le groupe glycyle.

Sont également avantageux les composés dans lesquels le premier groupe aminoacyle est L-prolyle, L-thréonyle ou L-valyle.

Les substituants en position $\gamma$ du résidu glutamyle ont tous en commun un groupe hydrocarboné R' dont une particularité est que dans l'ensemble, quelle que soit la structure exacte de ce groupe, il a tendance soit à conférer un certain caractère lipophile au composé considéré, soit à accroître ce caractère lorsque, indépendamment du groupe R', il se manifeste déjà.

Le caractère lipophile des groupes hydrocarbonés répondant à la définition de R' s'accroît bien évidemment avec le nombre d'atomes de carbone du groupe lorsque l'on part des substituants les plus courts, c'est-à-dire, dans le cas présent, les groupes R' ayant 4 atomes de carbone.

En théorie, le nombre d'atomes de carbone compris dans R' n'est pas limité du côté des grands nombres. Néanmoins, en pratique, l'accroissement du nombre d'atomes de carbone, passé un certain seuil, n'apporte pas d'avantages et nécessite la mise en œuvre de réactifs difficiles à se procurer dans le commerce. En pratique, le groupe R' ne contient pas plus d'une centaine d'atomes de carbone, et de préférence pas plus de 90. Le caractère lipophile atteint un niveau avantageux dès que R' contient environ une dizaine de carbones.

Entre le résidu D-glutamyle et le groupe R' peuvent s'intercaler un ou plusieurs résidus aminoacyles supplémentaires désignés dans la formule générale par A.

De préférence, ces aminoacyles sont choisis parmi le groupe comprenant : alanyle, leucyle, lysyle, glycyle, valyle, glutamyle et isoleucyle.

De façon particulièrement préférée, le premier aminoacyle fixé en $\gamma$ du D-glutamyle est L-alanyle. De façon préférée également, cet aminoacyle est L-lysyle ou L-glutamyle.

Le nombre d'aminoacyles entre le D-glutamyle et le groupe R' peut varier de 0 à 3, de préférence cependant il est soit 0, soit 1, soit 2.

Eventuellement, le dernier aminoacyle peut être remplacé par le résidu d'aminoalcool de même structure carbonée que l'aminoacyle, la liaison avec le groupe R' prenant alors la forme d'un ester de cet aminoalcool avec un acyle —CO—R'.

En position $\alpha$ du résidu D-glutamyle, les variantes ou substitutions possibles sont plus limitées qu'en position $\gamma$ de ce même groupe. Le substituant Y peut tout d'abord représenter le radical —OH, c'est-à-dire que l'on retrouve la fonction carboxylique de l'acide glutamique. Il peut s'agir aussi de la forme amide de cet acide, c'est-à-dire de la forme isoglutaminyle, Y étant alors $—NH_2$, l'un au moins des atomes d'hydrogène du groupe amino pouvant être substitué par des restes alcoyle courts comprenant de 1 à 10 atomes de carbone. Il peut encore s'agir des formes estérifiées de l'acide, Y étant alors un alcoxy, comprenant de 1 à 10 atomes de carbone.

Dans une forme préférée, Y est un hydroxyle.

Dans une autre forme préférée, Y est $—NH_2$.

Une autre forme préférée est constituée par le cas où Y est soit $—OCH_3$, soit $—OC_2H_5$.

Dans la forme préférée la plus usuelle, c'est-à-dire celle pour laquelle on retrouve la structure de l'acide muramique, R est $—CH_3$. Dans une autre forme préférée, le groupe R est un hydrogène ; on retrouve alors la structure de l'homologue inférieur désigné sous le nom d'acide nor-muramique.

La liaison glycosidique de la partie saccharidique dans les produits selon l'invention peut se présenter sous forme α ou β. Le résidu osidique peut recevoir également différents substituants dont la littérature antérieure, relative aux agents adjuvants du type muramyl-peptide, a donné un certain nombre d'exemples. En particulier, la littérature décrit des produits dont les groupes fonctionnels du résidu osidique sont « bloqués » par formation de groupements esters ou éthers sur les hydroxyles, ou de groupements amides sur le radical amino en position 2.

Dans la formule générale des produits selon l'invention, les substituants du cycle glucopyranoside ont été désignés par $R_1$, $R_2$, $R_4$ et $R_6$. Les différentes positions ne présentent pas les mêmes possibilités de substitution, la position 6 étant celle pour laquelle la plus grande latitude est offerte.

Des composés préférés sont ceux dans lesquels un ou plusieurs des substituants $R_1$, $R_4$ et $R_6$, indépendamment les uns des autres ou simultanément, sont l'hydrogène.

Des composés avantageux sont également ceux pour lesquels $R_4$ est le groupe succinyle $—CO—(CH_2)_2—CO_2H$ ou le groupe acétyle.

Des composés préférés sont aussi ceux pour lesquels $R_6$ est un radical acyle contenant de 1 à 4 atomes de carbone, et notamment les radicaux acétyle ($—COCH_3$), succinyle ($—CO(CH_2)_2—CO_2H$), ou encore ceux pour lesquels $R_6$ est le groupe mycoloyle (environ $C_{80}$ à $C_{90}$) ou corynomycoloyle ($C_{32}$).

Parmi les composés selon l'invention sont particulièrement préférés ceux pour lesquels $R_1$, $R_4$, $R_6$ sont simultanément un atome d'hydrogène, R est $—CH_3$, X est L-alanyle, Y est $—NH_2$ et $(A)_n—Z$ est

— un résidu L-alanyle estérifié ou amidé,
— un résidu L-lysyle estérifié ou amidé,
— un résidu ester.

Dans ces groupements $(A)_n—Z$, les résidus esters ou amidés sont de préférence ceux des groupes hydrocarbonés butyle, décyle, pentadécyle, éicosyle, benzyle, ou un reste dérivé d'un acide de type mycolique tel que le glycéryl-mycolate.

Des groupements $(A)_n—Z$ particulièrement préférés sont ceux de formule

$—NH—CH(CH_3)—COO—CH_2—(CH_2)_8—CH_3$,
$—NH—CH(CH_3)—COO—CH_2—(CH_2)_2—CH_3$,
$—NH—CH(CH_3)—CONH—CH_2—(CH_2)_8—CH_3$,
$—NH—CH(CH_3)—COO—CH_2—(CH_2)_{18}—CH_3$,
$—NH—CH(CH_3)—COO—(CH_2)_{14}—CH_3$,
$—NH—CH(CH_3)—COO—CH_2C_6H_5$,
$—NH—CH(CH_3)—COO—CH_2—CHOH—CH_2—O—R°$, avec R° correspondant à un acide mycolique comprenant de 80 à 90 carbones,
$—NH—CH[(CH_2)_4—NH_2]—COO—(CH_2)_9—CH_3$
$—O—(CH_2)_3—CH_3$
$—O—(CH_2)_9—CH_3$

Des composés préférés selon l'invention sont notamment les suivants

— le N-acétyl-muramyl-L-alanyl-D-isoglutaminyl-L-alanyl-décylamide de formule

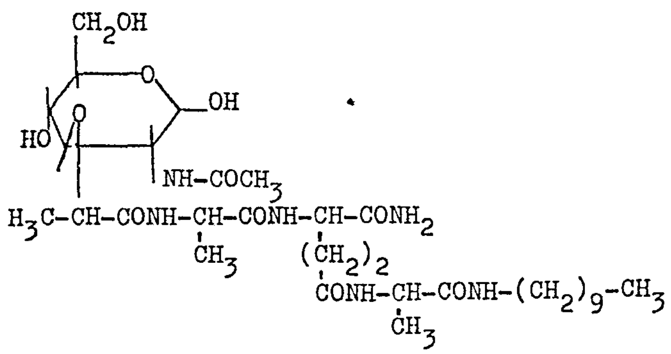

— le N-acétyl-muramyl-L-alanyl-D-isoglutaminyl-décyl-ester de formule

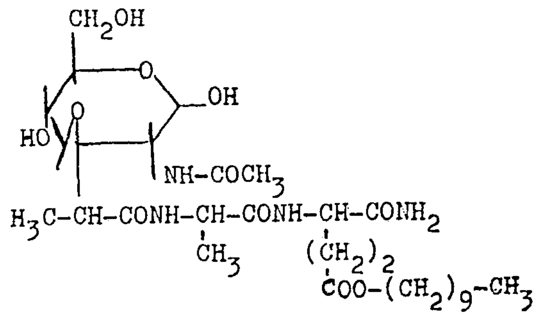

— le β-D-p.aminophényl-N-acétyl-muramyl-L-alanyl-D-isoglutaminyl-L-alanyl-décyl-ester de formule

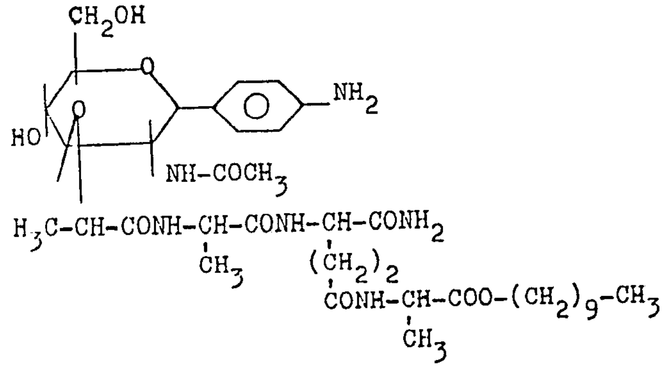

— le N-acétyl-muramyl-L-alanyl-D-isoglutaminyl-L-alanyl-pentadécyl-ester de formule

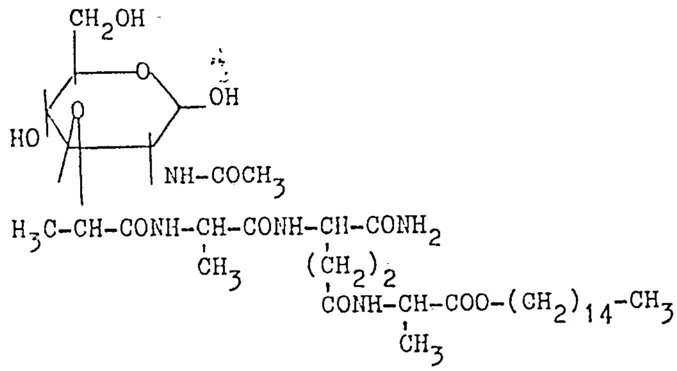

— le N-acétyl-muramyl-L-alanyl-D-isoglutaminyl-L-alanyl-eicosyl-ester de formule

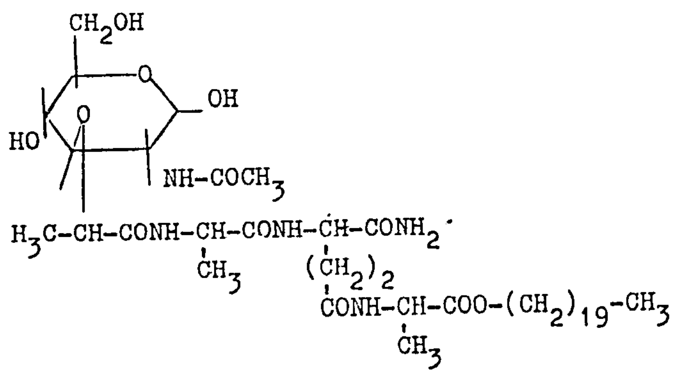

— le N-acétyl-muramyl-L-alanyl-D-isoglutaminyl-L-alanyl-benzyl-ester de formule

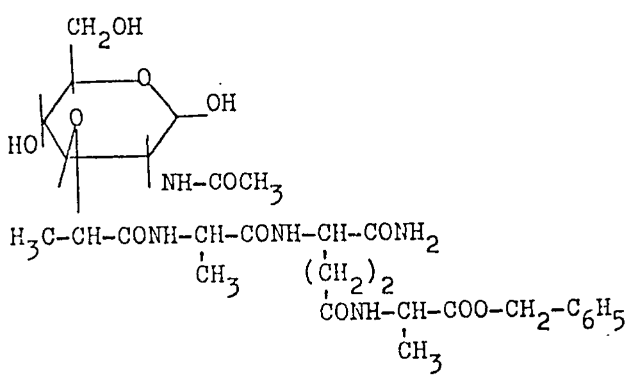

— le N-acétyl-muramyl-L-alanyl-D-isoglutaminyl-L-alanyl-D-glycéryl-mycolate de formule

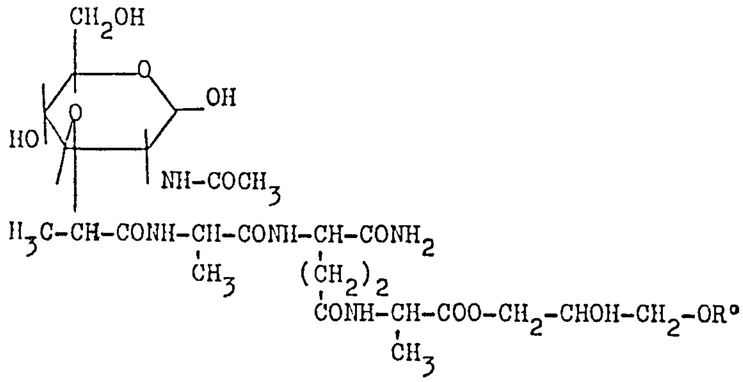

R° étant un radical d'acide mycolique contenant de 80 à 90 atomes de carbone ;
— le N-acétyl-muramyl-L-alanyl-D-isoglutaminyl-L-lysine-décyl-ester de formule

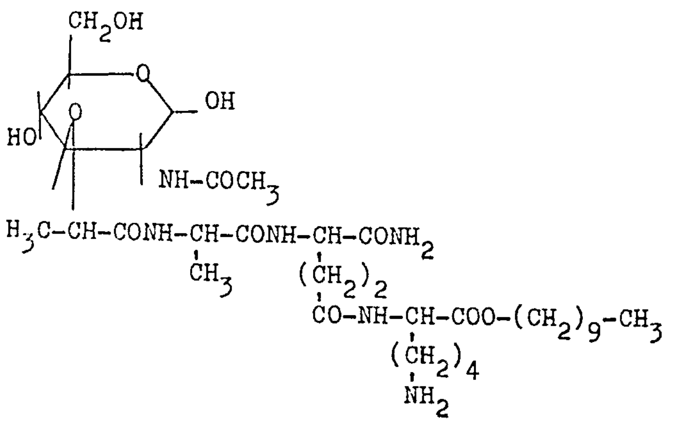

— le N-acétyl-muramyl-L-alanyl-D-isoglutaminyl-butyl-ester de formule

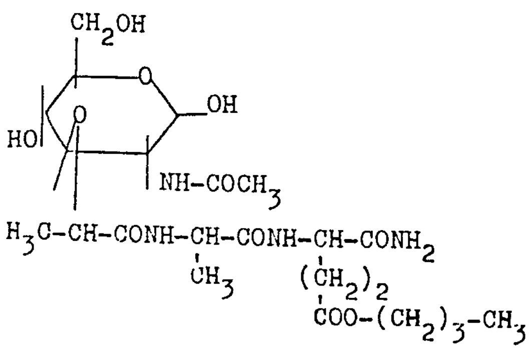

— le N-acétyl-muramyl-L-alanyl-D-glutamyl-α-méthyl-ester-γ-butyl-ester de formule

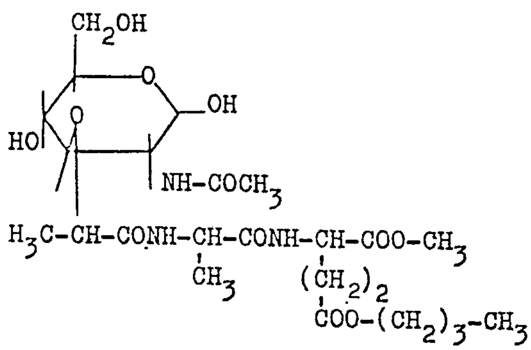

— le N-acétyl-muramyl-L-alanyl-D-glutamyl-α-méthyl-ester-γ-décyl-ester de formule

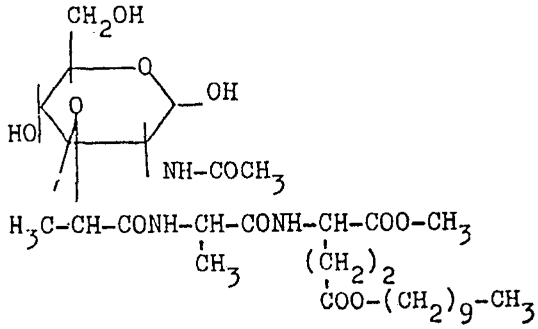

Les produits selon l'invention sont préparés par synthèse. Le cas échéant, certains des « fragments » utilisés pour ces synthèses peuvent provenir de produits naturels.

Pour aboutir à un même composé, diverses voies sont possibles. Dans tous les cas, la synthèse comporte une série d'étapes au cours desquelles les différents « fragments » constituant la structure d'ensemble des composés selon l'invention sont progressivement assemblés. Les principales différences entre les voies possibles se situent dans la séquence choisie pour l'assemblage des fragments. Les modes de réaction conduisant à la fixation d'un fragment au(x) fragment(s) contigu(s) sont dans l'ensemble peu modifiés par l'ordre dans lequel cette intégration est conduite, à ceci près bien entendu que de cet ordre dépend, d'une part, le choix des groupes fonctionnels qui réagissent et qui, par conséquent, doivent être libres pour l'étape considérée, et, d'autre part, le choix des groupes qui doivent être bloqués pour ne pas intervenir au cours de cette même étape.

La préparation des produits selon l'invention peut se faire à partir des composés correspondants du type muramyl-peptide. L'obtention de ces derniers a été décrite dans de nombreuses publications. Eventuellement, pour ceux dont la préparation n'apparaitrait pas expressément dans la littérature et notamment pour les diverses variantes correspondant aux substitutions du groupe muramyle ou des groupes analogues, ils peuvent être obtenus en suivant les modes traditionnels de préparation des dérivés correspondants de la chimie des oligosaccharides.

De la même façon, la constitution de la chaîne peptidique liée à l'acide muramique s'effectue selon les modes traditionnels en synthèse des peptides.

On a donné ci-après de façon succincte les principales indications relatives à différentes opérations qui peuvent être mises en œuvre pour synthétiser les produits selon l'invention, d'abord en envisageant séparément chaque étape, puis en indiquant quelques séquences types préférées.

a) Formation de l'acide muramique ou des analogues

Pour obtenir les analogues de l'acide N-acétyl-muramique de formule

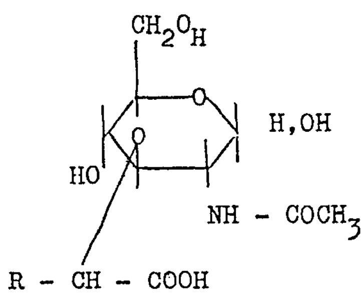

dans laquelle R a la signification précédemment indiquée, il est possible de partir d'un dérivé de la N-acétyl-2-glucosamine dont les hydroxyles en position 1, 4 et 6 sont bloqués de façon traditionnelle. Le mode de préparation d'un tel dérivé, le benzyl-2-acétamido-4,6-O-benzylidène-2-déoxy-D-glucopyranoside, est décrit notamment par P.H. GROSS et R.W. JEANLOZ (J. Org. Chem. 1967, *32*, 2761).

La formation de l'acide N-acétyl-muramique (R = $CH_3$) ou d'un de ses analogues peut être réalisée de la manière décrite dans les demandes de brevets français n° 74 22909 ou 76 19236 (respectivement, pour ces demandes, R = $CH_3$ et R = H) reprenant la méthode décrite par OZAWA et JEANLOZ (J. Org. Chem., 1965, *30*, 448).

Cette formation comprend par exemple la préparation d'un sel de sodium de l'hydroxyle en position 3 et la condensation ultérieure du dérivé sodé avec le sel ou l'ester d'un acide α halogéné tel que les acides chloro-2-propionique ou chloroacétique pour reprendre le cas des deux demandes de brevets indiquées précédemment. Le composé halogéné utilisé de forme L peut être préparé suivant la méthode décrite par

SINAY et al (J. Biol. Chem., 1972, *247*, 391). En utilisant les acides halogénés appropriés, il est possible de préparer tous les dérivés correspondant aux différentes significations de R. Ainsi, pour introduire un groupe R à 4 carbones, on peut utiliser les sels ou esters de l'acide chloro-2-butyrique.

Lorsqu'on utilise un ester d'acide halogéné, afin de pouvoir procéder à la condensation peptidique ultérieure, la fonction carboxylique peut être libérée par une hydrolyse appropriée.

b) Substitution sur le résidu saccharidique

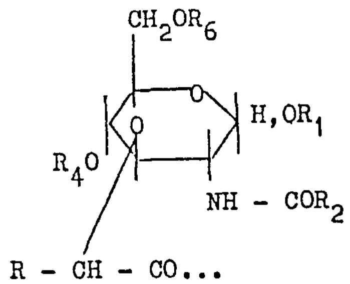

Les substitutions en position 1, 4 et 6 peuvent être réalisées par des méthodes qui ont été décrites antérieurement et qui sont traditionnelles dans la chimie des sucres. Lorsque les substituants envisagés sont différents les uns des autres, on procédera à autant de réactions de substitution successives qu'il y a de substituants distincts. Au cours de ces réactions, les positions ne devant pas être substituées ou celles qui doivent faire ultérieurement l'objet d'une autre substitution sont protégées temporairement par des groupes de blocage selon les modes habituels.

Les groupes de blocage initialement présents, dans le cas où l'on part, comme indiqué précédemment, de benzyl-2-acétamido-4,6-O-benzylidène-2-déoxy-D-glucopyranoside, sont éliminés par exemple par action de l'acide acétique (à 60 % 1 heure à reflux) et hydrogénation catalytique, comme décrit par exemple par MERSER et al. (Biochem. Biophys. Res. Commun., 1975, *66*, 1316), ou par hydrogénation catalytique suivant la méthode de LEFRANCIER et al. (Int. J. Peptide Protein Res., 1977, *9*, 249).

Les modes de substitution sont ceux traditionnellement utilisés. Pour obtenir les dérivés acylés, on opère à l'aide d'un agent acylant correspondant au substituant que l'on souhaite introduire (anhydride, chlorure d'acyle, etc.).

Les positions 1, 4, 6 ne sont pas équivalentes pour ce qui concerne leur réactivité. La position en $C^6$ est la plus facile à substituer, aussi, lorsque seule cette position doit être substituée, il est possible d'opérer sans bloquer les autres positions, avec une quantité d'agent de substitution équivalente à celle nécessaire pour la substitution d'une seule position.

Un exemple particulier de mode de préparation des dérivés substitués en position 6 est donné dans l'article de KUSUMOTO et al. (Tetrahedron Letters, 1976, *47*, 4237).

Les substitutions sur le résidu osidique peuvent être effectuées avant ou après la fixation de la chaîne peptidique ou des fragments de celle-ci.

c) Chaîne peptidique

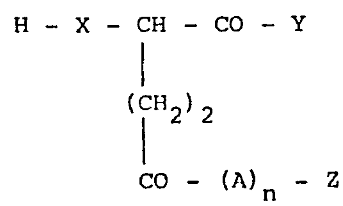

La fixation d'une chaîne peptidique sur l'acide N-acétyl-muramique, ou sur un analogue de celui-ci tels qu'ils ont été indiqués ci-dessus, est obtenue par des méthodes traditionnelles dans le domaine de la synthèse des peptides. De telles méthodes ont été amplement décrites dans la littérature antérieure et en particulier dans les demandes de brevets français indiquées précédemment.

De façon générale, les synthèses glycopeptidiques peuvent être faites soit en fixant un premier aminoacide au groupe muramyle, puis en fixant au composé ainsi obtenu le second aminoacide, et ainsi de suite de proche en proche. Il est aussi possible de préparer séparément la chaîne peptidique entière aminoacide par aminoacide et de fixer celle-ci sur le groupe muramyle. Il est enfin possible de choisir des procédés intermédiaires dans lesquels on prépare des fragments de la chaîne, puis soit d'assembler ces fragments entre eux jusqu'à former la chaîne complète qui est ensuite fixée au groupe muramyle, soit de fixer un premier fragment au groupe muramyle, puis un second au produit ainsi obtenu, etc. Le choix de la séquence est guidé principalement par des raisons de commodité ou de rendement.

Les substitutions Y et Z sont avantageusement réalisées sur le groupe glutamyle avant la synthèse de

la chaîne. De la même façon, lorsque n est différent de 0, c'est-à-dire lorsque un ou plusieurs groupes aminoacyle complètent la chaîne peptidique, le groupe Z est d'abord fixé à l'aminoacyle terminal avant que celui-ci ne soit intégré dans la chaîne peptidique.

Les synthèses peptidiques sont réalisées suivant des méthodes traditionnelles. A titre d'exemple, on peut utiliser les méthodes d'activation des carboxyles, comme la méthode dite des anhydrides mixtes. De façon avantageuse, la synthèse peptidique est réalisée à l'aide d'un composé du type carbodiimide tel que le N,N'-dicyclo-hexylcarbodiimide ou des carbodiimides équivalents. On trouvera une revue des modes de synthèse peptidique traditionnels dans J.H. JONES, Chemistry and Industry, 723 (1974). On peut aussi se reporter aux demandes de brevets français déjà citées, ou encore aux demandes de brevets français déjà citées, ou encore aux demandes suivantes : 75 29624, 76 06819, 76 06820, 76 06821, 76 21889, 77 02646, et aux articles de LEFRANCIER et al. (Int. J. Peptide Protein Res., 1977, *9*, 249 et 1978, 11, 289).

La formation des dérivés estérifiés ou amidés correspondant au groupe Y est obtenue de façon connue. On peut en particulier se reporter aux demandes de brevets français indiquées ci-dessus, et notamment aux demandes 76 06820, 76 06821, 76 21889 et 77 02646.

Pour fixer le reste Z à l'aminoacide situé à l'extrémité de la chaîne peptidique, on procède à une activation du groupe carboxylique de l'aminoacide de façon connue en soi, et on le soumet à une alcoolyse ou aminolyse par un alcool R'OH ou une amine $R'NH_2$.

d) Séquence de synthèse des composés glycopeptidiques correspondant à la variante de la formule générale dans laquelle n est 1 ou 2

Le schéma (I) représente une séquence type de réactions aboutissant à l'obtention de dérivés peptidiques correspondant à la partie $A_nZ$ de la formule générale (I).

Un ester activé BOC-$A_1$-OR" (ester succinimidique, ester p-nitrophénylique, etc.) d'un acide aminé N-protégé (comme par exemple par le ter-butyloxycarbonyle désigné par BOC, ou bien tout autre groupement convenable de protection temporaire de la fonction amine, utilisé en synthèse peptidique) est soumis soit à une alcoolyse par un alcool à chaîne de plus de 4 carbones en présence d'imidazole, comme catalyseur (comme indiqué par BODANZKY et al., J. Org. Chem., 1977, *42*, 149), cependant, dans le cas où $R'_1$ est un groupe alcoyle à chaîne de 4 à 20 carbones, les méthodes classiques connues d'estérification des acides aminés sont avantageusement utilisées, soit à une aminolyse par une alcoylamine à chaîne de plus de 4 carbones. Une fois l'acyl-aminoacyl-alcoyle ester ou l'acyl-aminoacyl-alcoyle amide respectivement obtenus, libérés du groupement acyle N-protecteur (par exemple, pour le ter-butyloxycarbonyl, par une solution N d'acide chlorhydrique dans l'acide acétique glacial), on obtient un composé de formule ($A_1$—$Z_1$) ou ($A_1$—$Z_2$). Le produit obtenu peut être couplé suivant les méthodes connues de la synthèse peptidique avec un deuxième acyl-aminoacide pour donner, après élimination du groupement acyle N-protecteur employé, un composé dipeptide de formule $A_2$—$A_1$Z.

Pour préparer les composés selon l'invention dans lesquels le dernier résidu de $(A)_n$ correspond à un aminoalcool, on peut opérer selon une variante de la séquence précédente représentée par le schéma (I').

Pour cette variante, un dérivé N-protégé d'un acide aminé tel que BOC-A'-COOH est réduit sélectivement en son dérivé aminoalcool BOC-A'-$CH_2OH$ suivant des méthodes bien connues. L'acide aminé peut aussi de la même façon être réduit en son aminoalcool correspondant, puis acylé sélectivement sur sa fonction amine suivant des méthodes couramment utilisées en synthèse peptidique. La fonction alcool ainsi créée peut alors être estérifiée par un ester activé (ester succinimidique, ester p-nitrophénylique, etc.) d'un acide gras à chaîne de plus de 4 carbones ($R'_1$—COOR"), en présence d'imidazole utilisé comme catalyseur (ainsi qu'indiqué par BODANZKY et al., J. Org. Chem., 1977, *42*, 149). Cet ester peut également se faire à partir du chlorure, de l'anhydride ou de l'imidazolide de l'acide gras. La suite des réactions est essentiellement celle décrite ci-dessus pour la variante du schéma (I).

Le schéma (II) représente des séquences réactionnelles conduisant à l'obtention des dérivés glycopeptidiques. On part d'un dérivé (1) avec $R_1$ radical benzyle, décrit par GROSS et JEANLOZ (J. Org. Chem., 1967, *32*, 2759). Pour obtenir le composé dans lequel $R_1$ est un groupement alcoyle ou aryl-alcoyle, on peut utiliser la méthode de préparation des α- ou β-glycosides correspondants également décrite dans ce même article, ou toute méthode connue pour de telles préparations en chimie des oligosaccharides.

Les dérivés de formule (1) sont couplés avec un dérivé dipeptidique de formule générale H-X-D-Glu (OBzl)-OY, chlorhydrate. Formule dans laquelle X correspond à un acide aminé, et Y par exemple à un radical amino-, hydroxy-, méthylamino-, méthoxy- ou glycyl-amide. Ces divers dérivés peptidiques sont préparés suivant les méthodes décrites par LEFRANCIER et al. (Int. J. Peptide Protein Res., 1977, *9*, 249, et Int. J. Peptide Protein Res., 1978, sous presse). Les méthodes de couplage utilisées pour obtenir les dérivés glycopeptidiques de formule (2) sont également décrites dans les articles précédemment cités. Toutefois, aussi bien dans la synthèse des dérivés dipeptidiques que dans celle des dérivés de la formule (2), toute méthode de couplage utilisée en synthèse peptidique peut être utilisée.

L'hydrogénation catalytique des composés de formule (5) est effectuée de façon traditionnelle (LEFRANCIER et al., Int. J. Peptide Protein Res., 1977, *9*, 249) pour aboutir aux composés de formule (3).

Les dérivés de formule (6) sont couplés, par exemple suivant la méthode décrite plus loin en détail pour la variante (point d), page 71) de préparation de l'ester décylique du MDP-L-alanine, au moyen d'un carbodiimide et de l'hydroxy-benzotriazole, avec un des dérivés de formule générale H—$(A)_n$—Z chlorhydrate dont le schéma (I) donne la séquence de synthèse. On obtient les composés de formule (4).

Dans une variante, les dérivés de formule (2) subissent une débenzylidénation sélective telle que décrite par MERSER et al. (Biochem. Biophys. Res. Commun., 1975, *66*, 1316) pour donner les dérivés de formule (5). L'acylation sélective de l'hydroxyle primaire en position 6 du résidu saccharidique peut alors se faire directement par action d'un faible excès d'anhydride d'acides carboxyliques ou d'acyl-imidazole. On obtient des dérivés de formule (6).

Les dérivés de la formule (6) peuvent être synthétisés suivant une séquence totalement différente (schéma IV, formule 4) semblable à celle mise au point par KUSUMOTO et al. (Tetrahedron Letters, 1976, *47*, 4237), à partir de la tosylation spécifique de l'alcool primaire du résidu saccharidique.

Après une hydrogénation catalytique des composés (6), effectuée comme usuellement en présence de palladium 5 % sur charbon, on obtient les composés de formule (7) auxquels, comme précédemment, peut être couplé un reste pour donner le composé (11) selon l'invention.

Dans une autre variante, les dérivés de formule (5) sont diacylés sur les deux hydroxyles en positions 4 et 6 du résidu saccharidique par action d'un excès d'anhydride d'acide carboxylique, puis soumis à une hydrogénation catalytique effectuée comme usuellement en présence de palladium 5 % sur charbon, pour obtenir les composés de formule (10). Après couplage avec le reste $(A)_n$Z, comme précédemment, on obtient les composés (11) selon l'invention.

e) Séquences de synthèse des composés glycopeptidiques correspondant à la variante de la formule générale dans laquelle n est nul

Lorsque n = 0, la fonction γ-carboxyle du résidu D-glutamyle est engagée dans une liaison ester avec un alcool à chaîne de plus de 4 carbones.

Une méthode particulière de préparation de ces dérivés consiste à faire l'ester γ-p-nitrophénylique du fragment peptidique bloqué BOC-X-D-Glu-O-Y, puis à procéder à l'alcoolyse par un alcool à chaîne de plus de 4 carbones en présence d'imidazole comme catalyseur (comme indiqué par BODANZKY et al., J. Org. Chem., 1977, *42*, 149). Après l'acidolyse usuelle du groupement ter-butyloxycarbonyle, le dérivé obtenu est couplé avec un dérivé saccharidique convenablement protégé correspondant à la formule (1) (Schéma III) pour aboutir aux dérivés de formule (2) suivant la méthode décrite plus loin en détail aux points a), b) et c) pour la préparation de l'ester décyclique du MDP-L-alanine ou suivant les procédés décrits dans MERSER et al. (Biochem. Biophys. Res. Commun., 1974, *466*, 1316), LEFRANCIER et al. (Int. J. Peptide Protein Res., 1977, *9*, 249, et Int. J. Peptide Protein Res., 1978, *11*, 289). Après l'hydrogénation catalytique et la purification effectuée comme décrit dans les deux derniers articles cités, les composés de formule (3) sont obtenus. Dans une variante, les dérivés de formule (2) subissent une débenzylidénation sélective telle que décrite par MERSER et al. (Biochem. Biophys. Res. Commun., 1975, *66*, 1316) pour donner les dérivés de formule (4).

L'acylation sélective de l'hydroxyle primaire en position 6 du résidu saccharidique peut se faire directement par action d'un faible excès d'anhydride, ou d'acyl-imidazole d'acide carboxylique. Les dérivés de formule (5) sont ainsi préparés (si $R_1$ est un radical benzyle, il peut être éliminé par une hydrogénation catalytique effectuée comme usuellement en présence de palladium 5 % sur charbon). Ces dérivés peuvent être synthétisés suivant une séquence totalement différente (schéma IV, formule (5)) semblable à celle mise au point par KUSUMOTO et al. (Tetrahedron Letters, 1976, *47*, 4237).

Dans une autre variante, les dérivés de formule (4) sont diacylés sur les hydroxyles en position 4 et 6 du résidu saccharidique par action d'un excès d'anhydride d'acide carboxylique pour obtenir finalement les composés de formule (7) (soumis éventuellement à une hydrogénation catalytique effectuée comme usuellement en présence de palladium 5 % sur charbon, si $R_1$ est initialement un radical de protection benzyle).

(Voir schémas pages suivantes)

Schéma (I)

Séquence réactionnelle de la préparation de dérivés peptidiques de formule $(A)_n—Z$ avec n = 1 ou 2

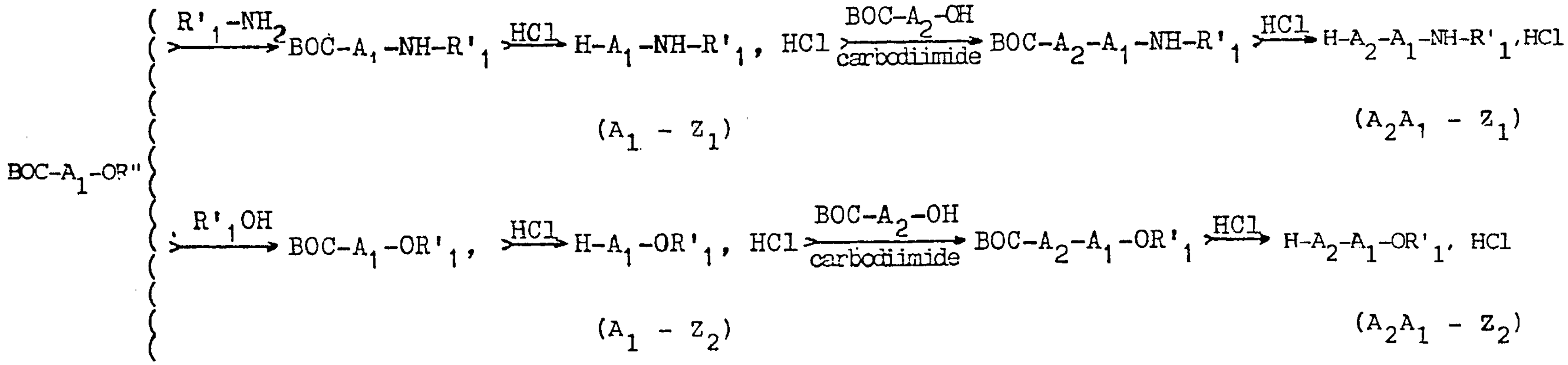


Schéma (I')

Séquence réactionnelle de la préparation de dérivés peptidiques

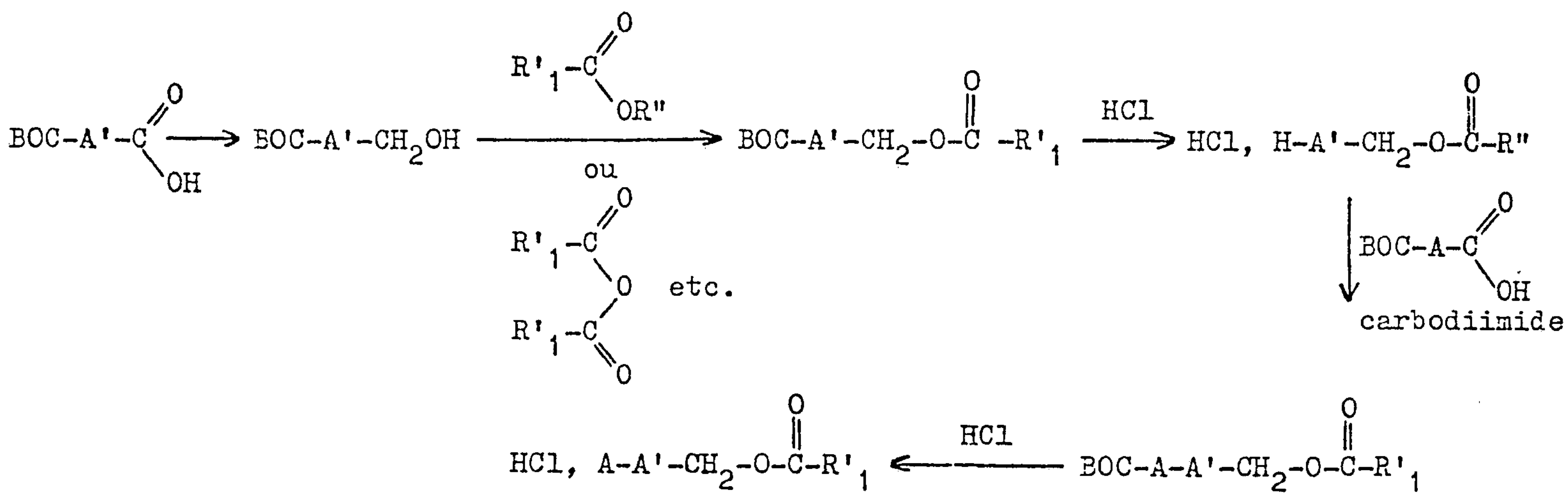

Schéma (II)

Séquences de synthèse des composés glycopeptidiques correspondant à la variante de la formule générale dans laquelle n est 1 ou 2

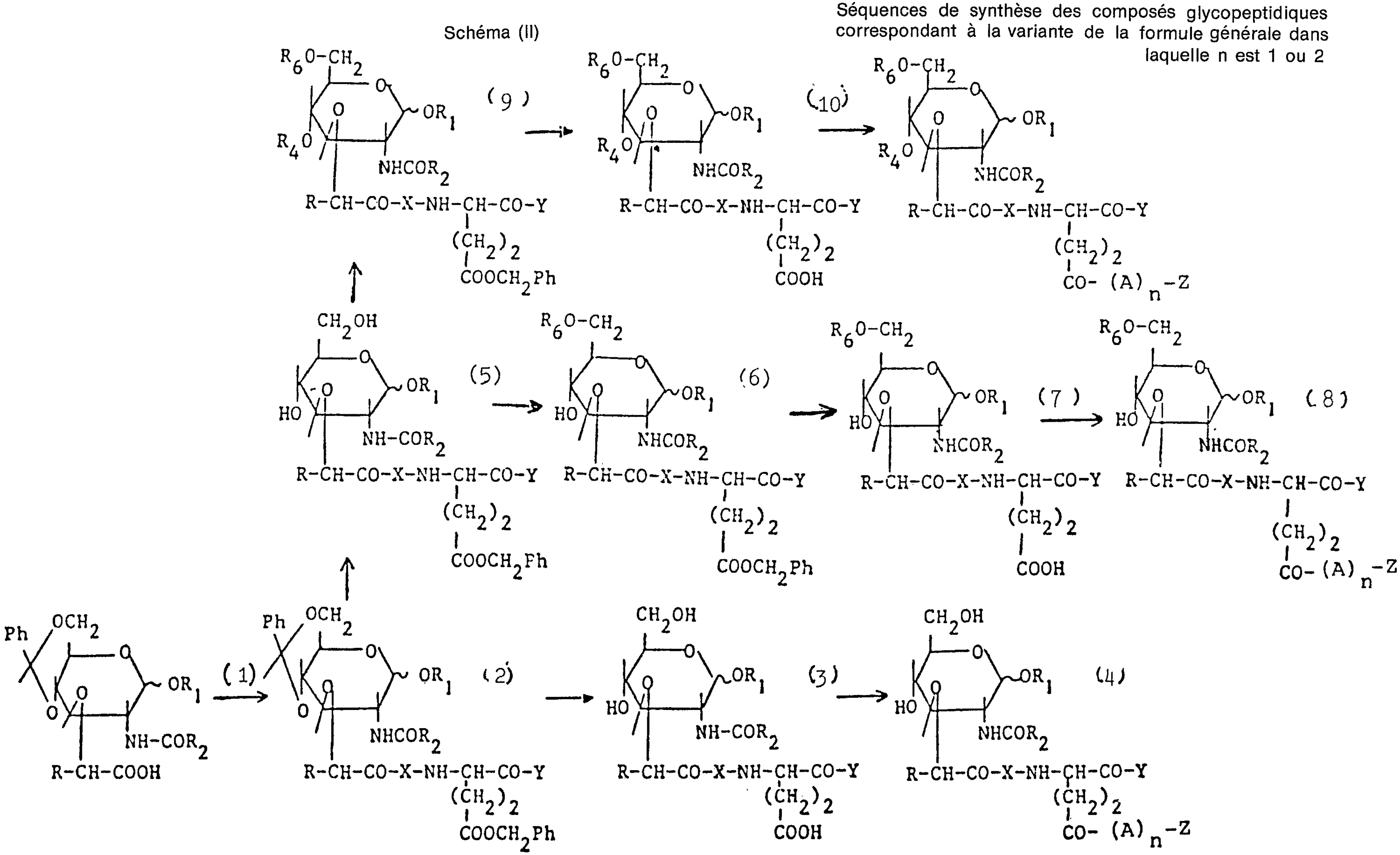

Séquences de synthèse des composés glycopeptidiques correspondant à la variante de la formule générale dans laquelle n est nul

Schéma (III)

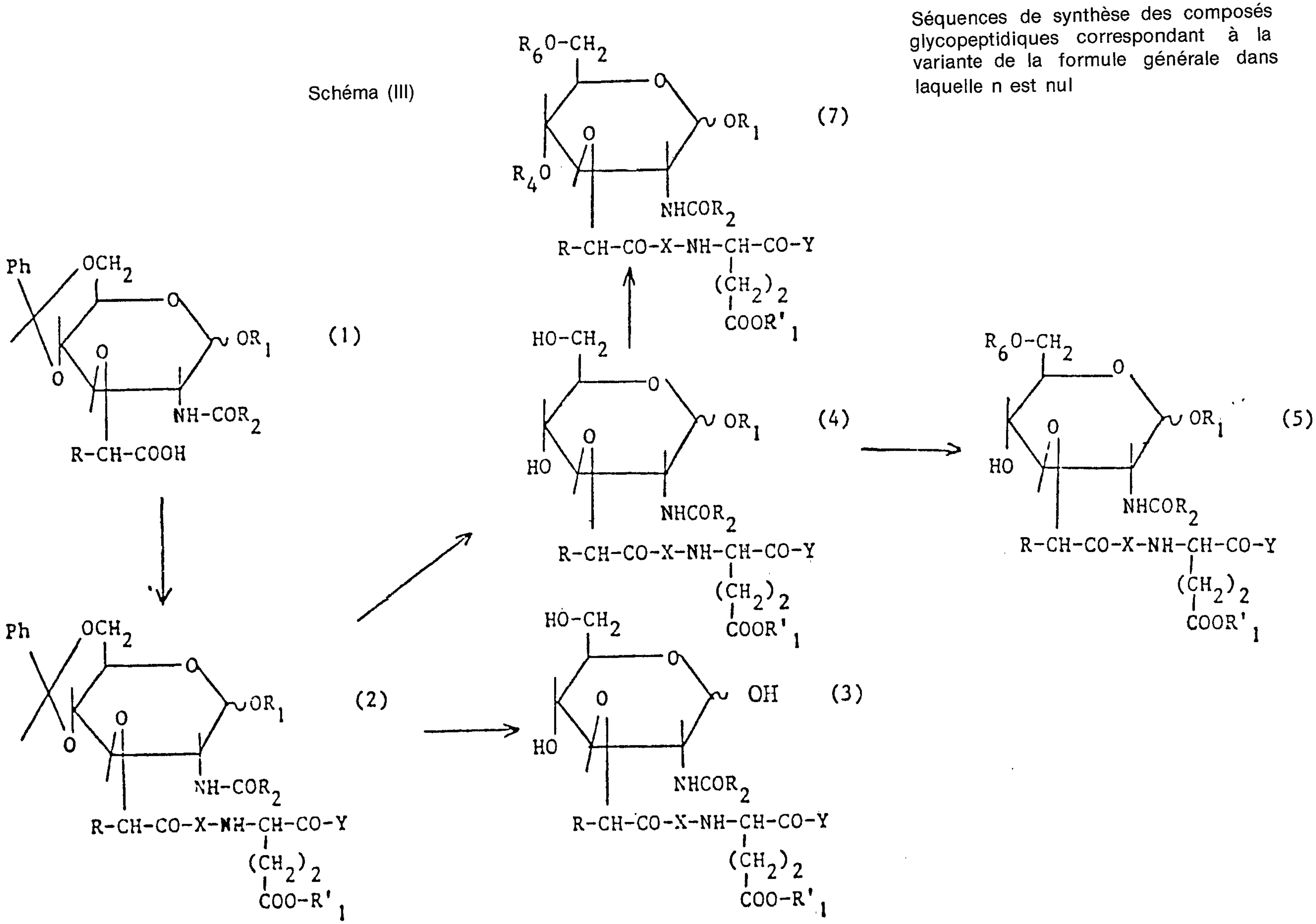

(1) (2) (3) (4) (5) (7)

Séquence de synthèse pour les dérivés glycopeptidiques correspondant à la formule générale et dont l'hydroxyle en C6 du résidu saccharidique est acylé par un acide gras à longue chaîne

Schéma (IV)

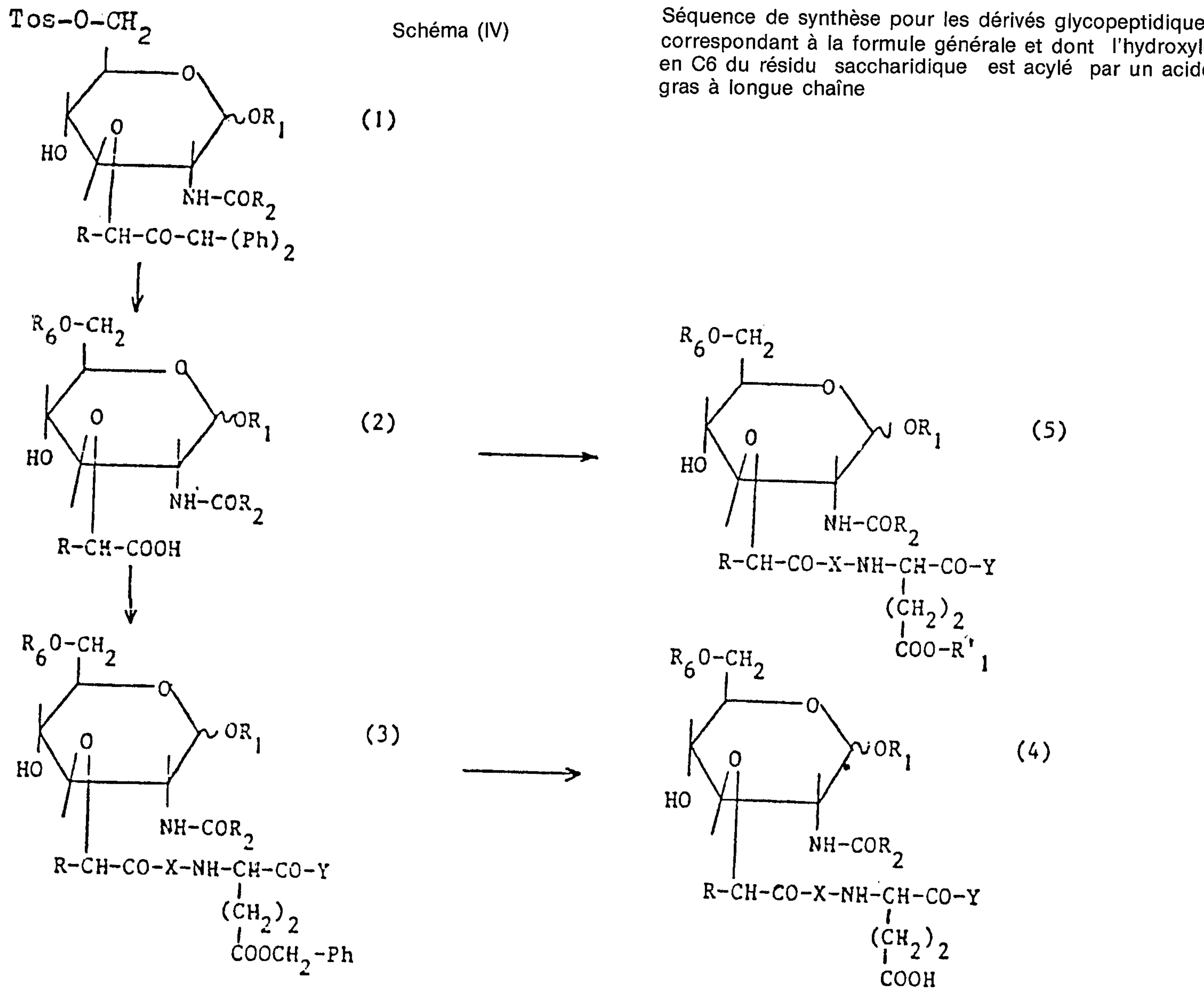

(1)

(2)

(3)

(4)

(5)

L'invention est aussi relative à des modes d'utilisation des composés répondant aux définitions précédentes, notamment en tant que réactif ou en tant que substance active dans des compositions pharmaceutiques.

L'invention concerne des réactifs biologiques, par exemple des adjuvants immunologiques standards, qui peuvent être constitués à l'aide des composés selon l'invention, notamment en vue d'étudier les éventuelles propriétés adjuvantes de substances à l'étude, par comparaison à de tels adjuvants standards ou, au contraire, comme agent susceptible de s'opposer à certains effets liés à l'administration de substances immunosuppressives.

Plus particulièrement, l'invention est relative à des médicaments renfermant à titre de principe actif au moins l'un des composés selon l'invention, ce médicament étant applicable en tant que régulateur de la réponse immunitaire du sujet auquel il est administré.

Ces médicaments sont notamment applicables lorsque l'on recherche un renforcement de la réponse immunitaire à tout agent immunogène. De tels agents immunogènes peuvent être naturels ou synthétiques, et nécessitent l'utilisation d'un agent stimulant le système immunitaire, soit que l'agent immunogène soit faible par nature, soit qu'il soit fort et puisse être utilisé à très faible dose, ou encore que le caractère immunogène ait été amoindri, par exemple au cours de modifications ou purifications antérieures. De façon générale, l'utilisation des composés immunorégulateurs selon l'invention est utile chaque fois que l'agent immunogène ne permet pas d'induire une réponse suffisante.

L'invention concerne plus particulièrement encore l'application des composés en question à l'amplification de l'effet immunogène de principes actifs de vaccins administrés à un hôte, animal ou humain, notamment dans le cas où ces principes vaccinants appartiennent aux catégories d'agents immunogènes rappelées ci-dessus. En conséquence, l'invention concerne également encore des compositions pharmaceutiques dont le principe actif est constitué par un au moins des composés selon l'invention, en association avec le véhicule pharmaceutique approprié au mode d'administration requis ou utilisable en égard à la nature du principe vaccinant utilisé.

L'invention s'applique en particulier à ceux des agents vaccinants dont le caractère immunogène est fort mais qui sont d'une utilisation difficile en temps normal en raison d'une trop grande toxicité ou d'effets secondaires indésirables. Il a été confirmé que les agents adjuvants selon l'invention étaient capables de compenser efficacement la perte de l'effet immunogène qui résulterait normalement d'une dilution ou d'une diminution des doses utilisées, notamment dans le but de réduire la toxicité ou les effets secondaires susdits dans une proportion correspondante, et ce sans influencer défavorablement ces derniers phénomènes.

On constate les mêmes effets dans le cas d'agents vaccinants forts dont on a réduit le caractère immunogénique, notamment par des purifications poussées, dans la mesure où cela s'avère nécessaire à la diminution correspondante de leurs effets toxiques ou secondaires néfastes. Tel est en particulier le cas des principes vaccinants constitués par des anatoxines bactériennes ou virales ou, d'une façon générale, des principes vaccinants constitués par une partie seulement des constituants initialement contenus dans les bactéries ou virus contre lesquels une protection est recherchée.

D'une façon générale, l'invention s'applique à tout antigène ayant subi des transformations chimiques ou physiques visant à éliminer ou modifier les parties de l'antigène qui sont responsables de ses effets secondaires nuisibles tout en conservant les parties qui sont à l'origine de ses propriétés immunogènes. C'est à ce type d'immunogènes faibles que se rattachent par exemple les principes constitués par les « sous-unités » dérivées de virus de la grippe, et qui ne retiennent de ceux-ci que les hémagglutinines et des neuraminidases, à l'exclusion des nucléoprotéines et autres constituants nucléotidiques des virus dont elles sont dérivées. Ceci s'applique également à certaines anatoxines, telles que celles par exemple de la diphtérie ou du tétanos, lesquelles, on le sait, peuvent être constituées par des substances solubles, telles qu'obtenues par l'action simultanée du formol et de la chaleur sur des toxines bactériennes dérivées des bactéries correspondantes.

L'invention concerne encore l'application des composés selon l'invention pour le traitement des maladies infectieuses. Dans cette application, il faut remarquer que les produits selon l'invention se distinguent clairement des antibiotiques habituellement utilisés. Les produits selon l'invention, contrairement aux antibiotiques, n'ont pas d'effet bactéricide ou bactériostatique *in vitro*. Par contre, ils peuvent activer les macrophages isolés *in vitro* et leur action *in vivo* est manifeste comme on le verra dans les exemples d'essais pharmacologiques. Contrairement aux antibiotiques encore, l'action n'est pas limitée à certaines variétés de micro-organismes. Ceci s'explique, nous l'avons vu, par le fait que leur activité n'est pas directe mais se développe par l'intermédiaire des mécanismes de défense immunitaire non spécifique de l'hôte, mécanismes que leur administration stimule et amplifie. Cette différence d'action par rapport aux antibiotiques rend ces produits d'autant plus intéressants qu'ils peuvent être utilisés contre des germes pathogènes devenus résistants aux antibiotiques.

Comme on l'a vu, le mode d'action des produits selon l'invention les rapproche des composés anti-infectieux connus tels que le BCG ou les lipopolysaccharides et comme tels peuvent être employés avec succès pour le traitement des infections sans présenter les inconvénients, notamment de toxicité, qui limitent ou interdisent l'utilisation des LPS ou du BCG.

L'application des produits selon l'invention comprend aussi bien le traitement des maladies causées par des micro-organismes à croissance extracellulaire comme les *Klebsiella* (ou encore notamment les

Pseudomonas, les staphylocoques, les streptocoques) que celui des micro-organismes à croissance intracellulaire (*Listeria*, mycobactéries, corynobactéries...).

Les applications indiquées précédemment à titre d'exemples ne sont pas exclusives des autres applications mettant en jeu les propriétés immunorégulatrices des composés selon l'invention. On peut encore citer à titre d'exemple leur action de renforcement au niveau de l'immunisation spécifique de l'hôte à l'égard d'antigènes parasitaires, la restauration de l'immunocompétence de l'hôte, lorsque celle-ci a un niveau inférieur à la normale, notamment lorsque celle-ci a été endommagée par les antigènes ou parasites eux-mêmes, ou sous l'effet d'une chimiothérapie, d'une radiothérapie, ou de tout autre traitement ayant une action immunosuppressive.

Les compositions pharmaceutiques selon l'invention, de façon générale, sont utiles pour le traitement ou la prévention de maladies infectieuses d'origine bactérienne ou parasitaire, ou pour l'inhibition d'affections tumorales.

Les adjuvants selon l'invention peuvent être administrés à l'hôte — animal ou être humain — de toute façon appropriée à l'obtention de l'effet désiré. Les administrations du principe immunorégulateur, notamment adjuvant, et de l'agent immunogène, notamment de l'antigène vaccinant, peuvent être envisagées simultanément ou séparément, dans ce dernier cas éventuellement décalées dans le temps, éventuellement encore par des voies d'administration semblables ou différentes (par exemple voies parentérale et orale respectivement ou vice versa).

L'invention concerne naturellement aussi les diverses compositions pharmaceutiques auxquelles les composés selon l'invention peuvent être incorporés, le cas échéant en association avec d'autres substances actives. En particulier, les composés I sont avantageusement associés à des agents immunogènes, qu'il s'agisse par exemple d'agents immunogènes utilisés à de très faibles doses, ou d'agents immunogènes faibles.

Des compositions pharmaceutiques avantageuses sont constituées par des solutions ou suspensions injectables contenant une dose efficace d'au moins un produit selon l'invention. De préférence, ces solutions ou suspensions sont réalisées dans une phase aqueuse stérilisée isotonique, de préférence saline ou glucosée.

L'invention concerne plus particulièrement de telles suspensions ou solutions qui sont aptes à être administrées par injections intradermiques, intramusculaires ou sous-cutanées, ou encore par scarifications, et notamment les compositions pharmaceutiques sous forme de liposomes dont la constitution sera explicitée plus loin.

Elle concerne également des compositions pharmaceutiques administrables par d'autres voies, notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des compositions pharmaceutiques dans lesquelles l'un au moins des composés selon l'invention se trouve associé à des excipients pharmaceutiquement acceptables, solides ou liquides, adaptés à la constitution de formes orale, oculaire ou nasale, ou avec des excipients adaptés à la constitution de formes d'administration rectale, ou encore avec des excipients gélatineux pour l'administration vaginale. Elle concerne aussi des compositions liquides isotoniques contenant l'un au moins des produits selon l'invention, adaptés à l'administration sur les muqueuses, notamment oculaire ou nasale. Elle concerne enfin des compositions formées de gaz liquéfiés pharmaceutiquement acceptables, du type « propellent », dans lesquelles les produits selon l'invention sont dissous ou maintenus en suspension, et dont la détente provoque la dispersion en un aérosol.

L'invention consiste également en un procédé visant à renforcer les défenses immunitaires de l'hôte, consistant à lui administrer une dose efficace de l'un au moins des produits selon l'invention, sous l'une des formes d'administration qui a été évoquée ci-dessus. A titre d'exemple de doses susceptibles d'induire une action, on mentionnera des doses de 10 à 1 000 μg par kg de corps, par exemple de 50 μg, lorsque l'administration est effectuée par voie parentérale, ou encore d'une dose de 200 à 20 000 μg par kg de corps, par exemple de 1 000 μg, pour d'autres modes d'administration, tels que par exemple la voie orale.

En dehors des composés eux-mêmes, on sait déjà toute l'importance de la forme sous laquelle ces composés sont utilisés pour la manifestation de leurs propriétés immunorégulatrices. Les émulsions eau dans l'huile ont été les premiers véhicules proposés pour l'administration de ce type de produits. Cependant, la préparation et surtout l'utilisation de ces émulsions soulèvent des difficultés. Ainsi, lorsque la phase huileuse n'est pas métabolisable, l'injection de la composition peut conduire à des réactions locales indésirables.

La découverte de l'activité des composés en l'absence d'une phase huileuse a constitué un progrès remarquable par la commodité d'administration qui en résulte et l'absence d'effets secondaires gênants. Cependant, le passage des formes en émulsion eau dans l'huile aux formes caractéristiques des composés actifs en l'absence de phase huileuse, et, notamment aux solutions aqueuses, peut modifier certains aspects de l'activité des produits considérés.

Dans cet ordre d'idées, les composés « actifs » en l'absence de phase huileuse présentent notamment des propriétés adjuvantes très intéressantes en ce qui concerne la protection immunitaire à médiation humorale.

Il semble à l'heure actuelle que l'administration de ces produits en l'absence de phase huileuse ne

permette pas d'obtenir les mêmes résultats que lors de l'administration en émulsion pour ce qui concerne les réponses immunitaires du type de celles que l'on met en évidence, par exemple, par des réactions d'hypersensibilité retardée.

Des études ont montré qu'un moyen commode pour modifier le spectre d'activité des produits du type muramyl-peptide est de les utiliser sous forme de liposomes (technique qui a été décrite notamment pour l'administration de préparations enzymatiques).

Les liposomes, on le sait, sont en général produits à partir de phospholipides ou d'autres substances lipidiques et sont formés par des cristaux liquides hydratés mono ou multilamellaires. Ils sont habituellement utilisés en dispersion dans un milieux aqueux.

On a constaté ainsi que les formes liposomiques pouvaient conduire à une augmentation de l'activité anti-infectieuse dans les essais effectués suivant le protocole décrit ci-après pour déceler l'activité vis-à-vis d'infection par *Klebsiella*. On a pu aussi, dans certains cas, obtenir un accroissement des réponses immunitaires de type à médiation humorale et/ou cellulaire.

Les variations entraînées par l'utilisation de la forme liposome ne sont pas nécessairement orientées dans le sens d'un accroissement général des activités des produits considérés. Le point essentiel est, à partir d'un produit donné, de pouvoir obtenir toute une gamme de propriétés en fonction du résultat recherché, en choisissant la forme la plus appropriée.

Comme on l'a indiqué ci-dessus, des compositions pharmaceutiques selon l'invention particulièrement avantageuses sont présentées sous la forme de liposomes.

Pour former les liposomes, on opère de façon traditionnelle. Tous les lipides non toxiques physiologiquement acceptables et métabolisables, capables de former des liposomes, peuvent être utilisés.

Les lipides les plus usuels sont des phospholipides, et notamment les phosphatidyl-cholines (lécithines) naturelles ou synthétiques. Peuvent aussi être utilisés des phospholipides, et parmi ceux-ci notamment les phosphatidyl-sérines, les phosphatidyl-inositides ou les sphingomyélines. D'autres lipides peuvent encore être utilisés, qui ont été décrits notamment par W. R. Hargreaves et D. W. Deamer (Conference on liposomes and their Uses in Biology and Medecine, Sept. 14-16, 1977, New York Acad. Sci.) et dans l'article de Biochem. 1978, 18, p. 3759.

Les techniques et appareillages traditionnels peuvent être employés pour former les liposomes selon l'invention. Ces techniques ont été décrites notamment au chapitre IV de l'ouvrage intitulé « Methods in cell biology », édité par David M. Prescott, volume XIV, 1976, Academic Press, New York, pages 33 et suivantes.

Avantageusement, la dispersion initiale est obtenue à partir d'une mince pellicule de lipide formée sur la paroi d'un récipient à partir d'une solution de ces lipides qui avait auparavant été introduite dans ce récipient et après évaporation du solvant. La phase aqueuse est alors introduite dans le récipient et la dispersion des lipides au sein de la solution aqueuse est formée par agitation du milieu, et généralement en ayant recours à des ultrasons.

On obtient, dans une première étape, une suspension de liposomes ayant un aspect laiteux. Il semble que, à ce stade, les liposomes sont formés d'une série de doubles couches lipidiques concentriques alternant avec des compartiments aqueux.

Les liposomes ainsi obtenus peuvent être séparés du milieu aqueux, par exemple par centrifugation. Les liposomes contenus dans le culot de centrifugation peuvent alors être lavés, de façon à éliminer toutes substances actives non incorporées aux liposomes. Ceux-ci peuvent alors être remis en suspension dans la solution tampon, dans laquelle ils peuvent être conservés, notamment au froid, de préférence à une température de l'ordre de + 4 °C. De tels liposomes sont stables pendant des périodes prolongées.

De préférence, la composition lipidique initiale contient également un agent de stabilisation, par exemple du cholestérol. Si besoin, on peut également avoir recours à un agent amphiphile, lequel peut être ajouté dans la dispersion lipidique initiale afin de permettre l'obtention finale de liposomes porteurs de charges électriques. De tels agents comprennent par exemple le dicétyl-phosphate ou la phosphatidyl-sérine, dans la mesure où l'on désire obtenir des liposomes chargés négativement, ou la stéarylamine, si l'on veut obtenir des liposomes chargés positivement.

On opère avantageusement à partir de lécithine et de cholestérol. De façon générale, après avoir formé un film de lécithine-cholestérol dans un récipient, on ajoute une solution aqueuse tampon et l'on agite pour obtenir une dispersion du film lipidique dans la phase aqueuse conduisant à la formation des liposomes. Le composé de type muramyl-peptide, en fonction de ses caractéristiques de solubilité, est introduit soit avec la phase lipidique (lécithine-cholestérol), soit dans la solution aqueuse tampon.

Les proportions molaires des constituants de la phase lipidique sont avantageusement comprises entre 8 : 1 et 1 : 1 (lécithine : cholestérol).

Avantageusement, la phase aqueuse est tamponnée de sorte que son pH soit voisin de la neutralité. On utilise une solution de tampon par exemple phosphate-NaCl 0,9 %.

Les compositions sous forme de liposomes selon l'invention peuvent renfermer d'autres substances compatibles avec cette forme particulière. Elles peuvent notamment, pour constituer des compositions vaccinantes, contenir des agents immunogènes.

De façon générale, les compositions sous forme de liposomes peuvent contenir, en plus du composé

du type muramyl-peptide, tous les constituants : stabilisants, conservateurs, excipients ou autres substances actives susceptibles d'être utilisés dans les solutions ou émulsions injectables présentées antérieurement pour l'administration des composés muramyl-peptide, sous la réserve qu'ils soient compatibles avec cette forme liposome.

Des teneurs de l'ordre de 1 mg de muramyl-peptide pour 35 mg de lipides sont avantageusement utilisées.

Les raisons pour lesquelles les formes liposomes conduisent à des résultats différents de ceux que l'on obtient à partir des préparations sous forme émulsions eau dans l'huile ou sous forme de solutions ne sont pas connues avec précision. On peut émettre quelques hypothèses sur des caractères bien précis. C'est ainsi que l'absence des réactions gênantes, qui apparaissent au point d'injection lorsqu'on utilise des émulsions eau dans l'huile (minérale) et ceci malgré la présence de constituants lipidiques, peut raisonnablement s'expliquer par le fait que ceux-ci sont par nature métabolisables.

L'invention est décrite de façon plus détaillée dans les exemples qui suivent relatifs à la préparation de produits selon l'invention, d'un mode de préparation de la forme pharmaceutique « liposome », et de divers essais concernant les propriétés pharmacologiques de ces produits et de cette forme particulière.

Préparation de produits selon l'invention

1. Ester décylique de N-acétyl-muramyl-L-alanyl-D-isoglutaminyl-L-alanine

a) Ester décylique de la L-alanine, paratoluène sulfonate

3 g de L-alanine, 30 ml de n-décanol et 6,54 g d'acide paratoluène sulfonique (monohydrate) sont mélangés dans un ballon de 250 ml. Ce ballon est chauffé avec précaution à la flamme nue d'un bec bunsen jusqu'à ébullition du n-décanol et obtention d'une solution homogène. On ajoute alors à cette solution encore chaude, avec précaution, 100 ml de benzène bouillant. La solution résultante est mise à reflux pendant 55 heures dans un appareil de Soxhlet dont la cartouche contient de la magnésie. On concentre alors la solution pour éliminer le benzène, puis on évapore l'excès de décanol sous vide poussé à 100 °C. Le mélange réactionnel est alors refroidi à 0 °C, et la pâte cristalline obtenue est triturée avec de l'éther froid. Le précipité cristallin est rapidement essoré et rincé à l'éther froid. On obtient 1,71 g du paratoluène sulfonate de l'ester décylique de la L-alanine. Ce produit présente les caractéristiques suivantes : P. F. 65-66 °C.

L'analyse élémentaire est pour $C_{20}H_{35}NO_5S$ (401,567)
calculé : C 59,82 H 8,79 N 3,49
trouvé : C 59,97 H 9,02 N 3,36

L'évaporation des eaux mères à sec sous vide fournit 6,66 g du même produit contenant probablement encore du n-décanol.

b) Ester décylique de la L-alanyl-D-isoglutaminyl-L-alanine, chlorhydrate

A 350 mg du produit préparé en a), dissous dans 5 ml de diméthylformamide à − 10 °C, on ajoute 100 µl de N-méthylmorpholine. A cette solution, on ajoute alors successivement 317 mg de t-butyloxycarbonyl-L-alanyl-D-isoglutamine, obtenus selon la méthode décrite par Lefrancier P. et Bricas E., (Bull. Soc. Chim. Biol., 1967, *49*, 1257), 135 mg d'hydroxybenzotriazole et 178 mg de dicyclohexylcarbodiimide. Le mélange réactionnel est agité 24 heures à température ambiante. Celui-ci est alors évaporé à sec, puis la dicyclohexylurée est précipitée au dichlorométhane. La solution est alors filtrée, le filtrat est lavé successivement avec une solution d'acide citrique (10 %), à l'eau, avec une solution N de bicarbonate de sodium et à l'eau. Après évaporation à sec de la phase organique, on obtient 307 mg de produit brut. Ce produit est dissous dans un volume minimum de diméthylformamide, puis passé sur une colonne (2 × 10 cm) d'Amberlyst 15 (H+) préalablement équilibrée avec du diméthylformamide. Après évaporation à sec de l'éluat, on obtient 275 mg de l'ester décylique de BOC-L-alanyl-D-isoglutaminyl-L-alanine.

La totalité de ce produit est alors traitée par 2 ml d'acide chlorhydrique (solution 1 N dans l'acide acétique) pendant 30 minutes. La solution est évaporée à sec sous vide, le résidu est rincé plusieurs fois à l'acétone et l'acétone est évaporée jusqu'à disparition de l'odeur acétique. Le produit résultant est repris à l'eau, ultrafiltré, puis lyophilisé. On obtient 206 mg de chlorhydrate de l'ester décylique de la L-alanyl-D-isoglutaminyl-L-alanine. Ce produit a un pouvoir rotatoire de $[\alpha]_D = -14,7°$ (eau).

Son analyse élémentaire est pour $C_{21}H_{41}N_4O_5Cl$ (465,04)
calculé : C 54,23 H 8,89 N 12,05
trouvé : C 54,15 H 8,93 N 11,31

c) Ester décylique de N-acétyl-muramyl-L-alanyl-D-isoglutaminyl-L-alanine (Mur-NAc-L-Ala-D-isoGln-γ-L-Ala-décyl-ester)

100 mg du produit obtenu en b) sont dissous dans 2 ml de diméthylformamide. Cette solution est refroidie à − 15 °C, et 23 µl de N-méthylmorpholine y sont ajoutés. Par ailleurs, dans une cellule

thermostatée à − 15 °C, on prépare une solution de 81 mg d'acide benzyl-O-benzylidène-4,6-N-acétyl muramique, préparé selon la méthode décrite par Ozawa T. et Jeanloz R. W. (J. Org. Chem., 1965, *30*, 448), dans 2 ml de diméthylformamide, puis on ajoute à cette solution 20 µl de N-méthylmorpholine, 23 µl d'isobutyle chloroformate et, 5 minutes après, la solution obtenue précédemment. Après 4 heures, on laisse la température remonter à 0 °C. On ajoute alors 0,18 ml d'une solution de $KHCO_3$ 2,5 M et on agite encore 30 minutes. Environ 40 ml d'eau sont alors ajoutés à cette solution, et le précipité résultant est essoré, lavé avec $KHCO_3$ 2,5 M, puis à l'eau. On obtient 162 mg de l'ester décylique du (benzyl-O-benzylidène-4,6-N-acétyl muramyl)-L-alanyl-D-isoglutaminyl-L-alanine. Ce précipité est dissous dans environ 10 ml d'acide acétique glacial. 150 mg de charbon palladié à 5 % sont ajoutés et le mélange est hydrogéné pendant 40 heures. Le charbon est alors filtré, le résidu est évaporé, puis repris dans un mélange eau/acide acétique et lyophilisé. On obtient 90 mg de l'ester décylique de la N-acétyl-muramyl-L-alanyl-D-isoglutaminyl-L-alanine. Son pouvoir rotatoire est $|\alpha|_D = + 13,1$ (acide acétique glacial).

L'analyse élémentaire est pour $C_{32}H_{57}N_5O_{12}$, 2 $CH_3COOH$ (823,92)
calculé : C 52,48 H 7,88 N 8,5
trouvé : C 52,30 H 7,77 N 8,97

d) Dans la méthode qui vient d'être présentée, la chaîne peptidique complète est d'abord synthétisée, puis fixée au résidu muramyle. Une autre méthode consiste à partir de la N-acétyl-muramyl-L-alanyl-D-isoglutamine, à fixer le dérivé décyl-ester de la L-alanine préparé comme il a été indiqué en a).

A un mélange de N-acétyl-muramyl-L-alanyl-D-isoglutamine (0,5 mmole, 246,25 mg), d'hydroxybenzotriazole (0,5 mmole, 67,5 mg) et de N-cyclohexyl-N'-[β(N-méthylmorpholino)éthyl]-carbodiimide, p-toluène sulfonate (0,5 mmole, 211,8 mg) dans 5 ml de diméthylformamide, on ajoute, après une heure, le p-toluène sulfonate de l'ester décylique de la L-alanine (0,6 mmole, 241 mg) et de la N-méthylmorpholine (0,6 mmole, 0,066 ml), en solution dans 5 ml de diméthylmorpholine. Après 48 heures d'agitation à température ambiante, le mélange réactionnel est dilué d'un égal volume d'acide acétique 0,1 N et la solution obtenue est passée sur une colonne de AG50 WX2, préalablement équilibrée dans le mélange acide acétique 0,1 N-diméthylformamide (50/50).

Après concentration des fractions contenant le produit, le résidu sirupeux obtenu est repris dans un volume de 5 à 10 ml de diméthylformamide auquel est ajouté un égal volume d'acide acétique $2 \cdot 10^{-3}$ M. La solution est alors passée sur une colonne de AGI X2, préalablement équilibrée dans le mélange acide acétique $2 \cdot 10^{-3}$ M-diméthylformamide (50/50).

Après concentration des fractions contenant le produit, celui-ci est obtenu par lyophilisation de sa solution acétique.

Une purification est finalement effectuée sur une colonne de gel de silice dans le solvant méthanol-chloroforme (1-3 v/v). On obtient 113 mg du produit, soit un rendement de 32 %. Son pouvoir rotatoire est $|\alpha|_D^{25} = + 20,9°$ (acide acétique glacial).

L'analyse élémentaire est pour $C_{32}H_{57}N_5O_{12}$, 1 $H_2O$ (721,85)
calculé : C 53,19 H 8,23 N 9,66
trouvé : C 53,11 H 7,9 N 9,45

De la même façon, on prépare en ayant recours à la même technique les produits suivants :

— l'ester eicosylique de la N-acétyl-muramyl-L-alanyl-D-isoglutamine-L-alanine dont le pouvoir rotatoire et l'analyse élémentaire sont respectivement $|\alpha|_D^{25} = + 20°$ (acide acétique glacial) pour $C_{42}H_{77}N_5O_{12}$, 0,75 $CH_3OH$, 0,25 $CHCl_3$
calculé : C 57,52 H 9,00 N 7,80
trouvé : C 57,53 H 8,74 N 7,79

— l'ester butylique de la N-acétyl-muramyl-L-alanyl-D-isoglutamine-L-alanine dont le pouvoir rotatoire et l'analyse élémentaire sont respectivement
$|\alpha|_D^{25} = + 6,4°$ (méthanol-eau 1/3) pour $C_{26}H_{45}N_5O_{12}$, 0,15 $CHCl_3$, 0,34 $H_2O$
calculé : C 48,79 H 7,17 N 10,88
trouvé : C 48,77 H 7,11 N 10,88

2. Décylamide de la N-acétyl-muramyl-L-alanyl-D-isoglutamine-L-alanine

a) Décylamide de la BOC-L-alanine

333 mg (1,16 mmoles) de BOC-Ala-OSu et 189 mg (1,2 mmoles) de décylamine sont dissous dans 10 ml de diméthylformamide. Le mélange réactionnel est laissé 1 heure à température ordinaire, puis concentré à sec. Le produit cristallise en aiguilles quand l'éther ajouté au sirop obtenu est évaporé. Il est séché sous vide en présence de $P_2O_5$. On obtient 367 mg de produit, soit un rendement de 96 %. Ses constantes physiques sont : P.F. 60-62 °C. $|\alpha|_D^{25} = - 24,1°$ (chloroforme)

L'analyse élémentaire est pour $C_{18}H_{36}N_2O_3$
calculé : C 65,81 H 11,04 N 8,52
trouvé : C 65,67 H 10,8 N 8,28

b) Chlorhydrate du décylamide de l'alanine

330 mg (1 mmole) de BOC-L-Ala-décylamide sont traités par 4 ml d'une solution normale d'HCl dans l'acide acétique glacial. Après 30 minutes, le mélange réactionnel est concentré à sec et le produit mis en suspension dans l'éther. On obtient 231 mg de produit, soit un rendement de 87,5 %. La détermination du point de fusion fait apparaître un changement d'aspect à 95 °C. P.F. 157-158 °C. $|\alpha|_D^{25} = +4{,}8°$ (méthanol)

L'analyse élémentaire est pour $C_{13}H_{28}N_2OCl$, 0,25 $H_2O$
calculé : C 58,18 H 10,70 N 10,44
trouvé : C 58,24 H 10,53 N 10,26

c) Décylamide de la N-acétyl-muramyl-L-alanyl-D-isoglutaminyle-L-alanyle

Le mode expérimental suivi pour la synthèse de ce produit est essentiellement le même que celui utilisé pour la synthèse du décyl-ester de la N-acétyl-muramyl-L-alanyl-D-isoglutaminyle-L-alanyle (deuxième mode de préparation d). Le pouvoir rotatoire et l'analyse élémentaire du produit sont : $|\alpha|_D^{25} = +18{,}7°$ (acide acétique glacial) pour $C_{32}H_{57}N_6O_{11}$, 0,5 $H_2O$
calculé : C 53,99 H 8,35 N 11,80
trouvé : C 53,94 H 8,10 N 11,83

3. Ester n-butylique du N-acétyl-muramyl-L-alanyl-D-isoglutaminyl-L-alanine

a) p-toluène sulfonate de l'ester n-butylique de la L-alanine

891 mg (10 mmoles) de L-alanine et 2 g (10,5 mmoles) d'acide p-toluène sulfonique sont dissous dans 10 ml de n-butanol et 2 ml de benzène. Le mélange réactionnel est chauffé à reflux (110-120 °C) pendant 3 heures dans un appareil soxhlet, du benzène étant ajouté régulièrement. Le n-butanol et le benzène ayant été évaporés, le produit est précipité à l'éther, puis cristallisé dans le mélange méthanol-éther. On obtient 2,65 g de produit, soit un rendement de 83 %. Les caractéristiques du produit sont : P.F. 98-99 °C. $|\alpha|_D^{20} = 0°$ (méthanol absolu) pour $C_{14}H_{23}NO_5S$
calculé : C 52,97 H 7,30 N 4,41
trouvé : C 52,58 H 7,05 N 4,23

b) ester n-butylique du N-acétyl-muramyl-L-alanyl-D-isoglutaminyl-L-alanine

En solution dans 8 ml de diméthylformamide, 493 mg (1 mmole) de N-acétyl-muramyl-L-alanyl-D-isoglutamine, 170 mg (1 mmole) de N-hydroxybenzotriazole, et 424 mg (1 mmole) de N-cyclohexyl-N'-[β(N-méthyl-morpholino)éthyl]-carbodiimide, p-toluène sulfonate, sont laissés pendant 1 heure à la température ambiante, puis ajoutés à une solution dans 7 ml de diméthylformamide de 224 mg (0,7 mmole) du p-toluène sulfonate de l'ester n-butylique de la L-alanine et de 0,077 ml (0,7 mmole) de N-méthyl morpholine. Après 4 jours, le mélange réactionnel est concentré à sec, repris dans une solution 0,1 M d'acide acétique et passé sur une colonne de résine échangeuse d'ions commercialisée par la Société BIOARD sous le nom AG-50-W-X2 (8 à 10 ml). Les fractions intéressantes sont réunies, lyophilisées, reprises dans une solution $2 \cdot 10^{-3}$ M d'acide acétique et passées sur une colonne de résine échangeuse d'ions commercialisée par la Société BIORAD sous le nom AG-1-X2. Les fractions intéressantes sont réunies et lyophilisées. Le produit est alors chromatographié sur une colonne de gel de silice (8 g) dans le mélange méthanol-chloroforme (1 : 4). Après lyophilisation, on obtient 158 mg de produit, soit un rendement de 36,4 %. Les caractéristiques du produit sont : $|\alpha|_D^{20} = +6{,}4°$ (méthanol-eau, 1/3) pour $C_{26}H_{45}N_5O_{12}$, 0,15 $CHCl_3$, 0,34 $H_2O$
calculé : C 48,79 H 7,17 N 10,88
trouvé : C 48,77 H 7,11 N 10,88

4. Ester décylique du N-acétyl-muramyl-L-alanyl-D-isoglutaminyl-L-lysine

a) p-toluène sulfonate de l'ester décylique de la $N^{\varepsilon}$-benzyloxycarbonyl-L-lysine 561 mg (2 mmoles) de $N^{\varepsilon}$-benzyloxycarbonyl-L-lysine et 418 mg (2,2 mmoles) d'acide p-toluène sulfonique sont dissous dans 2 ml (10 mmoles) de décanol. Le mélange réactionnel est chauffé à reflux (120 °C) pendant 5 heures dans un appareil soxhlet, du benzène étant ajouté régulièrement. Le produit est précipité à partir de ce mélange réactionnel, puis cristallisé dans le mélange méthanol-éther. On obtient 941 mg de produit, soit un rendement de 77,5 %. Les caractéristiques du produit sont : P.F. 99-100 °C. $|\alpha|_D^{20} = +1{,}8°$ (chloroforme) pour $C_{31}H_{48}N_2O_7S$
calculé : C 62,80 H 8,16 N 4,72
trouvé : C 62,88 H 8,25 N 4,79

b) Ester décylique du N-acétyl-muramyl-L-alanyl-D-isoglutaminyl-$N^{\varepsilon}$-benzyloxycarbonyl-L-lysine

En solution dans 5 ml de diméthylformamide, 492,5 mg (1 mmole) de N-acétyl-muramyl-L-alanyl-D-isoglutamine, 170 mg (1 mmole) de N-hydroxybenzotriazole et 424 mg (1 mmole) de N-cyclohexyl-N'-[β-(N-méthylmorpholino)éthyl]-carbodiimide, p-toluène sulfonate, sont laissés pendant 1 heure à température ambiante, puis ajoutés à une solution dans 5 ml de diméthylformamide de 415 mg (0,7 mmole) du p-toluène sulfonate de l'ester décylique de la $N^{\varepsilon}$-benzyloxycarbonyl-L-lysine et de 0,077 ml (0,7 mmole) de N-méthylmorpholine. Après 48 heures à température ambiante, le produit est précipité à partir du mélange réactionnel par addition de 250 ml d'eau glacée. Il est alors chromatographié sur colonne de gel de silice (20 g) dans le mélange méthanol-chloroforme (1/5). On obtient 393 mg de produit, soit un rendement de 62,7 %. Les caractéristiques du produit sont : P.F. 174-185 °C. $|\alpha|_D^{20} = +21°$ (acide acétique glacial) pour $C_{43}H_{70}N_6O_{14}$

calculé : C 57,69 H 7,88 N 9,39
trouvé : C 56,8 H 7,79 N 9,14

c) Ester décylique du N-acétyl-muramyl-L-alanyl-D-isoglutaminyl-L-lysine

279 mg (0,31 mmole) de l'ester décylique du N-acétyl-muramyl-L-alanyl-D-isoglutaminyl-$N^{\varepsilon}$-benzyloxycarbonyl-L-lysine, sont hydrogénés, en solution dans 25 ml d'acide acétique glacial, en présence de 200 mg de palladium 5 % sur charbon. Après 2 heures, le catalyseur est filtré et le produit est obtenu par lyophilisation. On obtient 255 mg de produit, soit un rendement de 100 %. Les caractéristiques du produit sont : $|\alpha|_D^{20} = +19°$ (acide acétique glacial) pour $C_{37}H_{66}N_6O_{14}$, 1 $H_2O$

calculé : C 52,91 H 8,41 H 10,02
trouvé : C 52,78 H 7,99 H 10,02

5. Ester pentadécylique du N-acétyl-muramyl-L-alanyl-D-isoglutaminyl-L-alanine

a) p-toluène sulfonate de l'ester pentadécylique de la L-alanine

400 mg (4,48 mmoles) de L-alanine, 937 mg (4,92 mmoles) d'acide p-toluène sulfonique, et 4,4 g (19 mmoles) de pentadécanol sont chauffés à reflux (120 °C) pendant 2 heures dans un appareil soxhlet, en présence de benzène. Le produit est précipité à l'éther à partir du mélange réactionnel, puis recristallisé dans l'éther. On obtient 2,076 g de produit, soit un rendement de 98 %. Les caractéristiques du produit sont : P.F. 73 °C. $|\alpha|_D^{20} = 0°$ (chloroforme) pour $C_{25}H_{45}NO_5S$

calculé : C 63,65 H 9,61 N 2,96
trouvé : C 63,27 H 9,26 N 3,25

b) Ester pentadécylique du N-acétyl-muramyl-L-alanyl-D-isoglutaminyl-L-alanine

En solution dans 5 ml de diméthylformamide, 492,5 mg (1 mmole) de N-acétyl-muramyl-L-alanyl-D-isoglutamine, 170 mg (1 mmole) de N-hydroxybenzotriazole et 424 mg (1 mmole) de N-cyclohexyl-N'-[β-(N-méthylmorpholino)éthyl]-carbodiimide, p-toluène sulfonate, sont laissés pendant 1 heure à la température ambiante, puis ajoutés à une solution dans 5 ml de diméthylformamide de 330,2 mg (0,7 mmole) du p-toluène sulfonate de l'ester pentadécylique de la L-alanine et 0,077 ml (0,7 mmole) de N-méthylmorpholine. Après 48 heures, le mélange réactionnel est évaporé à sec et chromatographié sur une colonne de gel de silice (40 g) dans méthanol-chloroforme (1-4). Les fractions intéressantes sont réunies, concentrées et le produit est précipité dans méthanol-eau. On obtient 125 mg de produit, soit un rendement de 23 %. Après passage sur une nouvelle colonne de gel de silice (MERCK - type A) dans le mélange n-butanol-acide acétique-eau (4 : 1 : 5 phase supérieure), le produit est récupéré par lyophilisation. On obtient 68 mg de produit dont les caractéristiques sont : $|\alpha|_D^{20} = +19,5°$ (acide acétique glacial) pour $C_{37}H_{67}N_5O_{12}$, 0,75 $CH_3COOH$, 0,5 $H_2O$

calculé : C 55,84 H 8,64 N 8,45
trouvé : C 55,60 H 8,10 N 8,47

6. Ester benzylique du N-acétyl-muramyl-L-alanyl-D-isoglutaminyl-L-alanine

En solution dans 8 ml de diméthylformamide, 493 mg (1 mmole) de N-acétyl-muramyl-L-alanyl-D-isoglutamine, 170 mg (1 mmole) de N-hydroxybenzotriazole et 424 mg (1 mmole) de N-cyclohexyl-N'-[β-(N-méthylmorpholino)éthyl]-carbodiimide, p-toluène sulfonate, sont laissés pendant 1 heure à la température ordinaire, puis ajoutés à une solution dans 7 ml de diméthylformamide de 248 mg (0,7 mmole) du p-toluène sulfonate de l'ester benzylique de la L-alanine et de 0,77 ml (0,7 mmole) de N-méthylmorpholine. Après 5 jours, le mélange réactionnel est concentré à sec, repris dans une solution 0,1 M d'acide acétique et passé sur une colonne de résine AG-50-W-X2. Les fractions intéressantes sont réunies, lyophilisées, reprises dans une solution $2 \cdot 10^{-3}$ M d'acide acétique et passées sur une colonne de résine AG-1-X2. Les fractions contenant le produit sont réunies, lyophilisées, puis chromatographiées sur une colonne de gel de silice (MERCK - type A) dans le mélange n-butanol-acide acétique-eau (150 : 5 : 25). Le produit est récupéré par lyophilisation. On obtient 151 mg de produit, soit un rendement de 33 %. Les caractéristiques du produit sont : $|\alpha|_D^{20} = +5,2°$ ($H_2O$) pour $C_{29}H_{43}N_5O_{12}$

calculé : C 53,28 H 6,63 N 10,71
trouvé : C 52,29 H 6,68 N 10,21

7. Ester décylique du N-acétyl-muramyl-L-alanyl-D-isoglutamine

a) Chlorhydrate de l'ester décylique de D-isoglutamine

Une solution de 490 mg (2 mmoles) de BOC-D-isoglutamine (BOC = butyloxycarbonyl) dissous dans 10 ml de méthanol et 1 ml d'eau est ajustée à pH 7 avec une solution aqueuse de 20 % $Cs_2CO_3$.

Après évaporation à sec et dessiccation du résidu, 5 ml de diméthylformamide et 0,5 ml (2,2 mmoles) de 1-bromodécane sont ajoutés. Après 24 heures à la température ordinaire, le mélange réactionnel est concentré à sec, repris dans l'acétate d'éthyle aqueux et lavé à l'eau. La phase acétate d'éthyle est séchée sur $Na_2SO_4$, puis évaporée. Le produit est cristallisé dans le mélange acétate d'éthyle-éther de pétrole. On obtient 719 mg de produit, soit un rendement de 93 %. Les caractéristiques du produit sont : P.F. 106-107 °C. $|\alpha|_D^{20}$ = + 4,5° (chloroforme) pour $C_{20}H_{38}N_2O_5$

calculé : C 62,09 H 9,90 N 7,24
trouvé : C 62,35 H 9,96 N 7,26

La débutyloxycarbonylation, effectuée par action d'une solution normale d'HCl dans l'acide acétique glacial, permet d'obtenir le produit donné en titre.

b) Ester décylique du chlorhydrate de L-alanyl-D-isoglutamine

284 mg (1,5 mmole) de BOC-L-alanine sont dissous dans 7 ml de diméthylformamide. A cette solution refroidie à – 15 °C sont successivement ajoutés 0,165 ml (1,5 mmole) de N-méthylmorpholine et 0,195 ml (1,5 mmole) de chloroformate d'isobutyle. Après 3 minutes, une solution, refroidie à – 15 °C, de 485 mg (1,5 mmole) du chlorhydrate de l'ester décylique de la D-isoglutamine et 0,165 ml (1,5 mmole) de N-méthylformamide, dans 5 ml de diméthylformamide, est ajoutée. Après 4 heures, le mélange réactionnel est amené à 0 °C, et 2,5 ml d'une solution 2,5 M $KHCO_3$ y sont ajoutés, puis 50 ml d'eau. Le produit est extrait à l'acétate d'éthyle, et l'extrait est successivement lavé à l'acide citrique 10 %, à l'eau, avec une solution $NaHCO_3$ 1 M, puis à l'eau.

La phase acétate d'éthyle est séchée, puis concentrée. Le produit est précipité par addition d'éther de pétrole. On obtient 632 mg de produit, soit un rendement de 92 %. Les caractéristiques du produit sont : P.F. 92-93 °C. $|\alpha|_D^{20}$ = + 2,5° (chloroforme) pour $C_{23}H_{43}N_3O_6$

calculé : C 60,36 H 9,47 N 9,18
trouvé : C 60,25 H 9,0 N 9,0

La débutyloxycarbonylation, effectuée par action d'une solution normale d'HCl dans l'acide acétique glacial, permet d'obtenir le produit donné en titre.

c) Ester décylique du N-acétyl-muramyl-(1-α-benzyl-4,6-O-benzylidène)-L-alanyl-D-isoglutamine

471 mg (1 mmole) de l'acide (1-α-benzyl-4,6-O-benzylidène)-N-acétyl-muramique sont dissous dans 5 ml de diméthylformamide. A cette solution, refroidie à – 15 °C, sont successivement ajoutés 0,11 ml (1 mmole) de N-méthylmorpholine et 0,13 ml (1 mmole) de chloroformate d'isobutyle. Après 3 minutes, une solution, refroidie à – 15 °C, de 394 mg (1 mmole) du chlorhydrate de l'ester décylique de la L-alanyl-D-isoglutamine et 0,11 ml (1 mmole) de N-méthylmorpholine, dans 5 ml de diméthylformamide, sont ajoutés.

Au bout de 4 heures, le mélange réactionnel est amené à 0 °C, et sont ajoutés 1,65 ml d'une solution 2,5 M $KHCO_3$ et, après 30 minutes, 100 ml d'eau. Le produit précipité est cristallisé dans le méthanol. On obtient 768 mg de produit, soit un rendement de 94,7 %. Les caractéristiques du produit sont : P.F. 235-241 °C. $|\alpha|_D^{20}$ = + 83,7° (diméthylformamide) pour $C_{43}H_{62}N_4O_{11}$

calculé : C 63,68 H 7,70 N 6,90
trouvé : C 63,56 H 7,55 N 6,89

d) Ester décylique du N-acétyl-muramyl-L-alanyl-D-isoglutamine

400 mg (0,5 mmole) de l'ester décylique de N-acétyl-muramyl-(1-α-benzyl-4,6-O-benzylidène)-L-alanyl-D-isoglutamine sont hydrogénés dans l'acide acétique glacial, en présence de Pd 5 % sur charbon (400 mg).

Après 48 heures, le catalyseur est filtré, l'acide acétique évaporé, et le produit précipité dans méthanol-acétate d'éthyle (2 fois). On obtient 200 mg de produit. Les caractéristiques du produit sont : P.F. 185-190 °C. $|\alpha|_D^{20}$ = + 38,7° (acide acétique glacial) pour $C_{29}H_{52}N_4O_{11}$

calculé : C 55,04 H 8,28 N 8,692
trouvé : C 54,52 H 8,12 N 8,7

8. Diester α-méthylique, γ-décylique du N-acétyl-muramyl-L-alanyl-D-glutamique

a) Chlorhydrate du diester α-méthylique, γ-décylique de l'acide D-glutamique

884,6 mg (2 mmoles) du sel de dicyclohexylamine de l'ester α-méthylique de l'acide BOC-D-glutamique sont dissous dans 5 ml d'eau. Cette solution est additionnée de 3,26 ml d'une solution aqueuse de 20 % de $Cs_2CO_3$ (soit 2 mmoles), puis concentrée à sec et desséchée. Le résidu est repris dans 20 ml de diméthylformamide et 0,46 ml (2,2 mmoles) de bromodécane sont ajoutés. Après une nuit, le mélange réactionnel est concentré et repris dans de l'acétate d'éthyle aqueux. La phase organique est lavée avec de l'acide citrique 10 %, à l'eau, avec une solution molaire de $NaHCO_3$, puis à l'eau. Elle est séchée sur $MgSO_4$, filtrée et concentrée. On obtient 791 mg de produit, soit un rendement de 98,5 %.

La débutyloxycarbonylation effectuée par action d'une solution normale d'HCl dans l'acide acétique glacial permet d'obtenir le produit donné en titre. On obtient 987 mg de produit, soit un rendement de 100 %. Les caractéristiques du produit sont : P.F. 95-97 °C. $|\alpha|_D^{20} = -13,3°$ (méthanol absolu) pour $C_{16}H_{32}NO_4Cl$

calculé : C 56,98 H 9,55 N 4,15
trouvé : C 56,49 H 9,34 N 4,25

b) Chlorhydrate de l'α-ester méthylique, γ-ester décylique de L-alanyl-D-glutamique

593 mg (3,13 mmoles) de BOC-L-alanine sont dissous dans 5 ml de diméthylformamide. A cette solution, refroidie à − 15 °C, sont successivement ajoutée 0,35 ml (3,13 mmoles) de N-méthylmorpholine et 0,4 ml (3,13 mmoles) de chloroformate d'isobutyle. Après 3 minutes, une solution de 962 mg (2, 85 mmoles) de chlorhydrate de l'α-ester méthylique, γ-ester décylique de l'acide D-glutamique et 0,3 ml (2,85 mmoles) de N-méthylmorpholine dans 5 ml de diméthylformamide, refroidie à − 15 °C, est ajoutée.

Au bout d'une nuit à − 15 °C, le mélange réactionnel est amené à 0 °C, puis additionné de 3 ml d'une solution 2,5 M $KHCO_3$. Après 1 heure, 100 ml d'eau sont ajoutés et le produit est extrait à l'acétate d'éthyle, la phase organique est lavée à l'acide citrique 10 %, à l'eau, avec une solution molaire de $KHCO_3$, puis à l'eau. Elle est séchée sur $MgSO_4$, filtrée, concentrée pour donner un résidu non cristallisé. On obtient 1,22 g de produit, soit un rendement de 90,6 %.

La débutyloxycarbonylation effectuée par action d'une solution normale d'acide chlorhydrique dans l'acide acétique glacial permet d'obtenir le dérivé donné en titre.

c) Diester α-méthylique, γ-décylique du N-acétyl-muramyl-(1-α-benzyl-2,6-O-benzylidène)-L-alanyl-D-glutamique

1,216 g (2,8 mmoles) de l'acide 1-α-benzyl-4,6-O-benzylidène-N-acétyl-muramique sont dissous dans 5 ml de diméthylformamide. A cette solution, refroidie à − 15 °C, sont successivement ajoutés 0,31 ml (2,8 mmoles) de N-méthylmorpholine et 0,37 ml (2,8 mmoles) de chloroformate d'isobutyle. Après 3 minutes, une solution de 1,15 g (2,58 mmoles) du chlorhydrate de l'α-ester méthylique, γ-ester décylique de L-alanyl-D-glutamique et 0,28 ml (2,58 mmoles) de N-méthylformamide, dans 5 ml de diméthylformamide, refroidie à − 15 °C, est ajoutée.

Au bout de 4 heures, le mélange réactionnel est amené à 0 °C et additionné de 2,8 ml d'une solution 2,5 M $KHCO_3$. Après 30 minutes, le produit est précipité par addition d'eau. On obtient 1,87 g de produit, soit un rendement de 87,7 %. Les caractéristiques du produit sont : P.F. 207-211 °C. $|\alpha|_D^{20} = +79°$ (diméthylformamide) pour $C_{44}H_{63}N_3O_{12}$

calculé : C 63,98 H 7,69 N 5,09
trouvé : C 63,81 H 7,73 N 5,07

d) Diester α-méthylique, γ-décylique du N-acétyl-muramyl-L-alanyl-D-glutamique

1,6 g (2,3 mmoles) du diester α-méthylique, γ-décylique du N-acétyl-muramyl-(1-α-benzyl-4,6-O-benzylidène)-L-alanyl-D-glutamique sont hydrogénés dans 100 ml d'acide acétique glacial, pendant 41 heures, en présence de Pd 5 % sur charbon (1,9 g). Après filtration du catalyseur, l'acide acétique est évaporé et le résidu repris dans 4 ml de chloroforme, chromatographié sur une colonne de gel de silice (MERCK — type C) dans le mélange chloroforme-méthanol-acide acétique (7 : 1 : 0,2). Les fractions contenant le produit sont réunies, concentrées, reprises à l'eau et lyophilisées.

Le produit obtenu est à nouveau purifié sur une colonne de gel de silice (silice 60-80 g) dans le même mélange de solvants que précédemment, et finalement lyophilisé. On obtient 929 mg de produit dont les caractéristiques sont : $|\alpha|_D^{20} = +26,3°$ (acide acétique glacial) pour $C_{30}H_{53}N_3O_{12}$ ; 0,5 $CH_3COOH$, 1 $H_2O$

calculé : C 53,51 H 8,26 N 6,04
trouvé : C 54,14 H 7,96 N 6,14

9. Diester α-méthylique, γ-n-butylique du N-acétyl-muramyl-L-alanyl-D-glutamique

a) Chlorhydrate du diester α-méthylique, γ-n-butylique de l'acide D-glutamique

1,33 g (3 mmoles) du sel de dicyclohexylamine de l'ester α-méthylique de l'acide BOC-D-glutamique sont dissous dans 5 ml d'eau. Cette solution est additionnée de 4,9 ml d'une solution aqueuse de 20 % de $Cs_2CO_3$ (soit 3 mmoles), puis concentrée à sec et desséchée. Le résidu est repris dans 50 ml de diméthylformamide, et 0,36 ml (3,3 mmoles) de bromobutane sont ajoutés. Après 20 heures, le mélange réactionnel est concentré à sec et repris dans de l'acétate d'éthyle aqueux. La phase organique est lavée avec de l'acide citrique 10 % à l'eau, avec une solution molaire de $KHCO_3$ et à l'eau. Elle est séchée sur $MgSO_4$, filtrée et concentrée. On obtient 884 mg de produit, soit un rendement de 93 %.

La débutyloxycarbonylation, effectuée par action d'une solution normale d'HCl dans l'acide acétique glacial, conduit au produit donné en titre. On obtient 587 mg de produit, soit un rendement de 83 %. Les caractéristiques du produit sont : P.F. 84-88 °C. $|\alpha|_D^{20} = -20°$ (méthanol absolu) pour $C_{10}H_{20}NO_4Cl$

calculé : C 47,34 H 7,95 N 5,52
trouvé : C 47,39 H 7,49 N 5,15

b) Chlorhydrate du diester α-méthylique, γ-n-butylique de L-alanyl-D-glutamique

473 mg (2,5 mmoles) de BOC-L-alanine sont dissous dans 5 ml de diméthylformamide. A cette solution, refroidie à − 15 °C, sont successivement ajoutés 0,28 ml (2,5 mmoles) de N-méthylmorpholine et 0,33 ml (2,5 mmoles) de chloroformate d'isobutyle. Après 3 minutes, une solution de 558 mg (2,2 mmoles) de chlorhydrate du diester α-méthylique, γ-n-butylique de l'acide D-glutamique et 0,24 ml (2,2 mmoles) de N-méthylmorpholine, dans 5 ml de diméthylformamide, refroidie à − 15 °C, est ajoutée.

Au bout d'une nuit à − 15 °C, le mélange réactionnel est amené à 0 °C, puis additionné de 3 ml d'une solution 2,5 M $KHCO_3$. Après 1 heure, 100 ml d'eau sont ajoutés et le produit est extrait à l'acétate d'éthyle. La phase organique est lavée à l'acide citrique 10 %, à l'eau, avec une solution molaire de $KHCO_3$, puis à l'eau. Elle est séchée sur $MgSO_4$, filtrée, concentrée pour donner un résidu non cristallisé. On obtient 804 mg de produit, soit un rendement de 94 %.

La débutyloxycarbonylation, effectuée par action d'une solution normale d'acide chlorhydrique dans l'acide acétique glacial permet d'obtenir le dérivé donné en titre.

c) Diester α-méthylique, γ-n-butylique du N-acétyl-muramyl-(1-α-benzyl-4,6-O-benzylidène)-L-alanyl-D-glutamique

1,037 g (2,2 mmoles) de l'acide 1-α-benzyl-4,6-O-benzylidène-N-acétyl-muramique sont dissous dans 5 ml de diméthylformamide. A cette solution, refroidie à − 15 °C, sont successivement ajoutés 0,24 ml (2, 2 mmoles) de N-méthylmorpholine et 0,29 ml (2,2 mmoles) de chloroformate d'isobutyle. Après 3 minutes, une solution de 700 mg (2,1 mmoles) du chlorhydrate du diester α-méthylique, γ-n-butylique de L-alanyl-D-glutamique et 0,24 ml (2,1 mmoles) de N-méthylmorpholine dans 5 ml de diméthylformamide, refroidie à − 15 °C, est ajoutée.

Au bout de 4 heures, le mélange réactionnel est amené à 0 °C et additionné de 2,5 ml d'une solution 2,5 M $KHCO_3$. Après 30 minutes, le produit est précipité par addition d'eau. On obtient 1,365 g de produit, soit un rendement de 89,3 %. Les caractéristiques du produit sont : P.F. 195-203 °C. $|\alpha|_D^{20} = +92,3°$ (diméthylformamide) pour $C_{38}H_{51}N_3O_{12}$

calculé : C 61,52 H 6,93 N 5,66
trouvé : C 61,47 H 7,02 H 5,41

d) Diester α-méthylique, γ-n-butylique du N-acétyl-muramyl-L-alanyl-D-glutamique

1,34 g (1,8 mmoles) du diester α-méthylique, γ-n-butylique du N-acétyl-muramyl-(1-α-benzyl-4,6-O-benzylidène)-L-alanyl-D-glutamique sont hydrogénés dans 50 ml d'acide acétique glacial, pendant 40 heures, en lrésence de Pd 5 % sur charbon (1,35 g). Après filtration du catalyseur et évaporation de l'acide acétique, le produit est chromatographié sur colonne de gel de silice (silice 60-80 g) dans le mélange chloroforme-méthanol-acide acétique (6 : 1 : 0,2). Les fractions contenant le produit sont réunies, concentrées, reprises à l'eau, puis lyophilisées. On obtient 658,4 mg de produit, soit un rendement de 66 %. Les caractéristiques du produit sont : $|\alpha|_D^{20} = +30,8°$ (acide acétique glacial) pour $C_{24}H_{41}N_3O_{12}$ ; 0,3 $CH_3COOH$, 1 $H_2O$

calculé : C 49,44 H 7,12 N 7,03
trouvé : C 49,01 H 7,03 N 7,12

10. N-acétyl-muramyl-L-alanyl-D isoglutaminyl-L-alanyl-glycéryl-mycolate

a) Tosyl-glycéryl-mycolate

A 3,5 g de monomycolate de glycérol, en solution dans 25 ml de pyridine sèche, sont ajoutées 6 portions de 126 mg de chlorure de tosyle. Au bout de 72 heures, le mélange réactionnel est concentré et le résidu est trituré dans le toluène. Ce dernier est recueilli et concentré après filtration. Ce dernier est recueilli et concentré après filtration. Le produit est purifié par chromatographie sur colonne de gel de

silice (silice 60) par lots de 1,8 g chacun, dans le mélange chloroforme-éther (95-5). On obtient 1,719 g de produit, soit un rendement de 46 %.

b) Chlorhydrate de la L-alanyl-glycéryl-mycolate

1,45 g (1 mmole) de tosyl-glycéryl-mycolate dissous dans 20 ml de benzène sec sont ajoutés à 250 mg du sel de potassium de la BOC-L-alanine et à 140 mg de 18-crown-6, en solution dans 15 ml de benzène sec. Le mélange réactionnel est chauffé à reflux pendant 6 heures dans des conditions rigoureusement anhydres, puis, après refroidissement, il est filtré et concentré à sec. Le produit est chromatographié sur colonne de gel de silice (silice 60) dans le mélange benzène-éther (65-35). On obtient 958 mg de produit, soit un rendement de 65 %.

La débutyloxycarbonylation par action d'une solution normale d'HCl dans l'acide acétique glacial permet d'obtenir 970 mg du produit donné en titre.

c) N-acétyl-muramyl-L-alanyl-D-isoglutaminyl-L-alanyl-glycéryl-mycolate

En solution dans 15 ml de diméthylformamide, 352 mg de N-acétyl-muramyl-L-alanyl-D-isoglutamine, 121,5 mg de N-hydroxybenzotriazole et 303 mg de N-cyclohexyl-N'-[β-(N-méthylmorpholino-éthyl]-carbodiimide, p-toluène sulfonate, sont laissés pendant 1 heure à la température ambiante, puis ajoutés à une solution dans 10 ml de benzène sec de 970 mg du chlorhydrate de la L-alanyl-glycéryl-mycolate et de 0,075 ml de N-méthylmorpholine.

Après 28 heures à température ambiante, le mélange réactionnel est amené à siccité. Le résidu est repris dans le mélange benzène-méthanol (50 : 1) et filtré sur silice pour éliminer toute trace de diméthylformamide. L'éluat concentré est purifié par passage sur une colonne de gel de silice dans le mélange benzène-méthanol (5 : 1). Les fractions intéressantes sont réunies, concentrées, reprises dans l'acide acétique à chaud (60 °C) et lyophilisées. On obtient 684,6 mg de produit, soit un rendement de 57 %. Les caractéristiques du produit sont : $|\alpha|_D^{20} = +12°$ (benzène) pour $C_{109}H_{205}O_{16}N_5$ ; 1,5 $CH_3COOH$

calculé : C 69,63 H 11,01 N 3,62
trouvé : C 69,90 H 10,91 N 3,34

Mode de préparation des liposomes

10 µmoles (7,3 mg) de DL-dipalmitoyl-α-phosphatidylcholine (Pm 734, grade I, approximativement 99 %, commercialisé par SIGMA) et 10 µmoles (3,9 mg) de cholestérol (PM 386,6, 99 %, SIGMA) sont dissous dans environ 5 ml de chloroforme (qualité purissime), dans un ballon à fond rond de 10 ml.

Les adjuvants lipophiles, à raison de 1,0 mg, sont introduits avec la phase chloroformique.

La solution est évaporée à l'évaporateur rotatif sous vide à une température inférieure à 30 °C de manière à obtenir un film fin de lécithine + cholestérol sur la paroi intérieure du ballon.

5 ml d'une solution tampon phosphate de sodium 0,013 M, pH 7,0, contenant 9 ‰ de NaCl sont portés à 55 °C. Les adjuvants hydrosolubles sont introduits dans cette solution. Par ailleurs, le ballon contenant le film lipidique ci-dessus est également chauffé à 55 °C.

La solution tampon est versée lentement dans le ballon en maintenant la température à 55 °C. Le contenu est alors soumis à une agitation douce à 55 °C de manière à obtenir une suspension de liposomes contenant l'adjuvant envisagé. On laisse reposer environ 1 heure.

La préparation est soumise ensuite à une sonication à 0 °C sous atmosphère d'azote ou d'argon durant 30 secondes (0,3 kW).

Lorsque les adjuvants sont hydrosolubles, la suspension est en outre centrifugée à 100 000 g, le culot est repris par le tampon phosphate-NaCl. L'opération est renouvelée six fois. Le culot finalement obtenu est repris par du tampon phosphate-NaCl, au volume choisi.

Propriétés pharmacologiques

Les essais dont les résultats sont donnés ci-après ont porté sur divers produits répondant à la formule générale (I). Par commodité pour ces produits, le groupe N-acétyl-muramyl-L-alanyl-D-isoglutaminyle est désigné par MDP, de même que le groupe N-acétyl-muramyl-L-alanyl-D-glutamyle est désigné par MDPA.

1. Toxicité

On a étudié la toxicité des produits selon l'invention par administration parentérale sur des souris. On a constaté que les doses toxiques sont d'un ordre de grandeur très supérieur à celui des doses auxquelles ces produits manifestent leur activité. Ainsi, la dose létale 50 des produits selon l'invention testés est supérieure à 5 mg/kg d'animal chez la souris surrénalectomisée, dont la sensibilité aux endotoxines est bien connue.

Les produits essayés étaient notamment :

— le MDP décyl-ester
— le MDP-L-Ala-décyl-amide
— le MDP-L-Ala-eicosyl-ester
— le MDP-L-Ala-glycéryl-mycolate
— le MDP-L-Ala-benzyl-ester
— le MDP-L-Lys-décyl-ester

On peut mettre en évidence les propriétés très favorables des produits selon l'invention, notamment en ayant recours aux tests décrits ci-après.

2. Test du Limulus

Pour montrer l'absence d'activité de type endotoxique, on a soumis des produits selon l'invention au test du Limulus. A cet effet, on mélange 0,1 ml de la préparation de lysat de Limulus amoebocyte (commercialisé par la Société MALLINCKRODT, à SAINT-LOUIS, E.U.A.) avec un volume égal du produit testé à diverses concentrations en solution dans de l'eau distillée apyrogène. Les ustensiles utilisés sont également rendus apyrogènes par chauffage à sec à l'étuve, à 180 °C, pendant 2 heures.

Après incubation du mélange pendant 20 minutes à 37 °C dans des tubes, on évalue la formation de gel caractéristique de la présence d'endotoxine.

Un test est positif lorsqu'on constate la présence d'un gel ferme qui reste adhérent au fond du tube lorsque celui-ci est renversé (technique décrite par ELIN R.J. et WOLFF S.M., J. Infect. Dis., 1973, *128* : 349). Les résultats de ces essais sont les suivants :

| | |
|---|---|
| — MDP-L-Ala-butyl-ester | 100 μg/ml |
| — MDP-L-Ala-décyl-ester | > 100 μg/ml |
| — MDP-L-Ala-décyl-amide | > 100 μg/ml |
| — MDP-L-Ala-eicosyl-ester | 10 μg/ml |
| — MDP-Ala-glycosyl-mycolate | > 100 μg/ml |

Dans les mêmes conditions, le LPS extrait d'*Escherichia coli* à titre de comparaison est positif à 0,01 μg/ml.

Ces résultats montrent, compte tenu des doses anti-infectieuses efficaces, que les propriétés mises en évidence dans les tests suivants ne peuvent pas provenir d'une contamination endotoxique.

3. Caractère adjuvant en phase aqueuse et en émulsion

a) En phase aqueuse

Des groupes de 8 souris Swiss âgées de deux mois reçoivent, par injection sous-cutanée (SC), 0,5 mg d'antigène constitué par de l'albumine de sérum bovin (BSA) avec 0,1 mg ou sans la substance essayée dans une solution saline isotonique. Cette dose élevée d'antigène, parce qu'elle se situe à la limite de la dose paralysante vis-à-vis de la réponse immunitaire, entraîne, de ce fait, une réponse faible ou nulle à l'antigène seul chez les témoins ; elle constitue donc un critère sévère pour mettre en évidence l'activité d'une substance adjuvante. Trente jours plus tard, les souris reçoivent, par la même voie d'administration, un rappel contenant 0,1 mg du même antigène.

Le taux d'anticorps est déterminé, six jours après le rappel, par hémagglutination passive en utilisant des hématies de mouton traitées à la formaline et recouvertes de l'antigène étudié selon la méthode décrite A.A. HIRATA et M.W. BRANDISS (J. Immunol., *100*, 641-648, 1968).

Le titre en anticorps, représenté par la dilution sérique maximale agglutinant une quantité donnée d'hématies de moutons, atteint un maximum au 36e jour et s'établit de la façon suivante :

| | $Log_2$ du titre hémagglutinant |
|---|---|
| — Témoins | 3,15 ± 1,72 |
| — MDP | 8,24 ± 1,27 |
| — MDP-décyl-ester | 8,64 ± 1,31 |
| — MDP-L-Ala-butyl-ester | 10,31 ± 0,49 |
| — MDP-L-Ala-décyl-ester | 6,23 ± 1,23 |
| — MDP-L-Ala-décyl-amide | 8,64 ± 0,95 |
| — MDP-L-Ala-penta-décyl-ester | 8,39 ± 0,89 |
| — MDP-L-Ala-eicosyl-ester | 7,11 ± 2,03 |
| — MDP-L-Ala-glycéryl-mycolate | 7,47 ± 1,17 |
| — MDP-L-Ala-benzyl-ester | 7,47 ± 0,89 |
| — MDP-L-Lys-décyl-ester | 9,64 ± 0,70 |

Les titres sont exprimés en logarithme de base 2. On voit que tous les composés ont permis d'augmenter considérablement le niveau de la réponse humorale (au moins 8 fois et jusqu'à plus de 100 fois).

b) En émulsion

Les essais sont effectués sur des lots de 6 cobayes Hartley mâles de 350 g. L'administration est faite par injection intradermique dans le coussinet plantaire de chacune des pattes postérieures. L'ovalbumine (constituant l'antigène) à raison de 1 mg est préparée dans 0,1 ml d'une émulsion de solution isotonique saline, dans une phase huileuse constituée soit par l'adjuvant incomplet de Freund (FIA), soit par l'adjuvant complet (FCA) formé par le FIA auquel est ajouté 0,1 mg de cellules entières de *Mycobacterium smegmatis.* Le composé selon l'invention est administré à raison de 0,05 mg additionné dans l'émulsion contenant le FIA.

Dix-huit jours après cette immunisation, on recherche d'éventuelles réactions d'hypersensibilité retardée à l'antigène en injectant par voie intradermique 0,01 mg d'ovalbumine sur le flanc des animaux, et l'on observe, 48 heures après, la réaction au point d'injection. On mesure le diamètre en millimètres de la réaction ainsi provoquée.

Vingt et un jours après l'injection, les animaux sont saignés. Sur le sérum recueilli, on mesure la teneur en anticorps spécifiques de l'ovalbumine par précipitation du complexe anticorps-antigène dans la zone d'équivalence. La quantité d'azote protéique contenue dans ce précipité est évaluée suivant la méthode de Folin. Les valeurs moyennes des teneurs en anticorps sont indiquées dans le tableau de résultats. Ces valeurs expriment la quantité, en microgrammes d'azote précipitable par l'antigène, par millilitre de sérum.

Les résultats de ces essais sont les suivants :

| | HSR | $Log_2$ du titre hémagglutinant |
|---|---|---|
| Témoin (AIF) | 0 | 10,29 ± 0,75 |
| AIF + 50 μg MDP | 14 ± 4 | 12,58 ± 1,02 |
| AIF + 50 μg MDP-L-Ala-butyl-ester | 18 ± 2 | 13,31 ± 0,52 |
| AIF + 50 μg MDP-L-Ala-décyl-ester | 17 ± 2,5 | 10,81 ± 1,47° |
| AIF + 50 μg MDP-L-Ala-décyl-amide | < 5 | 12,31 ± 0,82 |
| AIF + 50 μg MDP-L-Ala-pentadécyl-ester | < 5 | 10,97 ± 1,04 |
| AIF + 50 μg MDP-L-Ala-eicosyl-ester | 6 ± 4 | 11,25 ± 1,14⁺ |
| AIF + 50 μg MDP-L-Ala-glycéryl-mycolate | 21 ± 3 | 12,77 ± 0,83 |
| AIF + 50 μg MDP-L-Ala-benzyl-ester | 12,5 ± 3,5 | 12,31 ± 0,82 |
| AIF + 50 μg MDP-L-Ala-décyl-ester | 13 ± 2 | 13,4 ± 0,55 |

Ces résultats montrent que les produits essayés provoquent, à des degrés divers, un accroissement du taux des anticorps formés.

L'administration du produit engendre aussi une hypersensibilisation de type retardé chez le sujet traité vis-à-vis de l'antigène, hypersensibilisation qui est révélée par le test cutané.

4. Activité anti-infectieuse vis-à-vis de *Klebsiella*

Le protocole des essais est décrit dans l'article CHEDID L. et col., Proc. Natl. Acad. Sci. USA, 1977, *74* : 2089.

On a ainsi établi de façon préalable un mode expérimental permettant de mettre en évidence le caractère anti-infectieux des produits. On a montré qu'une dose d'environ $10^4$ *Klebsiella pneumoniae,* injectée par voie intramusculaire à des souris, entraîne le décès progressif d'une part importante, sinon de la totalité, des animaux dans la semaine suivant l'inoculation. Après huit jours, la survie des animaux est définitivement acquise.

On a suivi la survie de groupes de souris inoculées dans les conditions indiquées ci-dessus et traitées à l'aide des produits selon l'invention.

Pour ces essais, on a utilisé des souris hybrides (C57Bl/6 × AKR)Fl élevées de l'INSTITUT PASTEUR, à partir de souches provenant de l'élevage du C.N.R.S. à ORLEANS.

L'infection par *Klebsiella pneumoniae,* souche de type capsulaire 2, biotype d, est faite à partir d'une culture de 16 heures en milieu pour pneumocoques (n° 53515, INSTITUT PASTEUR). La dose infectante est $2 \cdot 10^4$ *Klebsiella* ; elle est administrée par voie intramusculaire.

L'administration du produit testé est réalisée par voie intraveineuse dans 0,2 ml de soluté physiologique apyrogène, les témoins recevant le soluté seul. Elle est réalisée 24 heures avant l'inoculation.

Les résultats de ces essais sont rapportés dans le tableau suivant. Le pourcentage de protection indiqué est la différence des pourcentages de survivants du groupe traité par rapport au groupe témoin.

| Traitement i. v. 24 h avant $2 \times 10^4$ bactéries i. m. | Dose par souris | Nombre de souris traitées | Nombre de survivants au jour 3 | 5 | 8 | % de protection |
|---|---|---|---|---|---|---|
| Témoins | — | 24 | 8 | 3 | 3 | |
| MDP-décyl-ester | 10 | 8 | 1 | 0 | 0 | |
| | 100 | 24 | 24 | 21 | 21 | 75 |
| Témoins | — | 24 | 13 | 5 | 4 | |
| MDP-L-Ala-butyl-ester | 100 | 24 | 23 | 18 | 17 | 54 |
| Témoins | — | 32 | 13 | 9 | 8 | |
| MDP-L-Ala-décyl-ester | 100 | 32 | 30 | 22 | 22 | 44 |
| Témoins | — | 24 | 13 | 5 | 4 | |
| MDP-L-Ala-décyl-amide | 100 | 24 | 21 | 17 | 16 | 50 |
| Témoins | — | 24 | 8 | 3 | 3 | |
| MDP-L-Ala-penta-décyl-ester | 100 | 24 | 24 | 16 | 15 | 50 |
| Témoins | — | 24 | 12 | 4 | 3 | |
| MDP-L-Ala-eicosyl-ester | 10 | 24 | 18 | 14 | 14 | 46 |
| | 100 | 24 | 23 | 23 | 20 | 70 |
| Témoins | — | 24 | 7 | 4 | 4 | |
| MDP-L-Ala-glycéryl-mycolate | 10 | 8 | 5 | 4 | 4 | 33 |
| | 100 | 24 | 23 | 23 | 23 | 79 |
| Témoins | — | 24 | 8 | 3 | 3 | |
| MDP-L-Ala-benzyl-ester | 100 | 24 | 24 | 14 | 14 | 46 |
| Témoins | — | 24 | 8 | 3 | 3 | |
| MDP-L-Lys-décyl-ester | 100 | 24 | 22 | 20 | 20 | 71 |
| Témoins | — | 16 | 7 | 3 | 3 | |
| MDPA (butyl-ester) $OCH_3$ | 100 | 16 | 12 | 11 | 9 | 38 |
| Témoins | — | 16 | 7 | 3 | 3 | |
| MDPA (décyl-ester) $OCH_3$ | 100 | 16 | 13 | 11 | 10 | 44 |

Les résultats font apparaître une protection accrue de façon significative en appliquant le test t de Student, pour les animaux ayant reçu les produits selon l'invention par rapport aux animaux témoins.

5. Activité anti-infectieuse vis-à-vis des *Listeria*

Dans des conditions analogues à celles des essais du 4., on a déterminé l'influence de l'administration du Mur-NAc-L-Ala-D-isoGln-γ-L-Ala-décyl-ester sur la mortalité de souris auxquelles on inocule une dose de *Listeria monocytogènes,* connues pour engendrer de façon typique une infection de type cellulaire.

Comme précédemment, on a comparé l'action du produit selon l'invention à celle du BCG dont les propriétés anti-infectieuses sont bien connues dans ce domaine. On a également déterminé, toujours à titre de comparaison, l'action du LPS, le *Corynebacterium granulosum,* et du Mur-NAc-L-Ala-D-isoGln (MDP).

Le traitement des souris et l'inoculation sont effectués par voie intraveineuse. Les produits injectés sont en solution ou suspension dans 0,2 ml de soluté physiologique apyrogène. Les témoins ne reçoivent que le soluté.

La dose de *Listeria* administrée est, dans tous les essais, de $1 \cdot 10^3$ unités.

Les essais dont les résultats sont donnés ci-après ont été effectués en faisant varier les doses de produits essayés et l'intervalle de temps séparant le traitement de l'inoculation des *Listeria monocytogè-*

*nes* (soit 1, soit 7 jours). On suit le nombre de souris survivantes au 5e et au 10e jour suivant l'inoculation, et on détermine le pourcentage de protection comme pour les essais précédents.

| Traitement intra-veineux | Dose par souris µg | Nombre de jours avant infection | Nombre de souris | Nombre d'animaux survivants au jour 5 | 10 | % de protection |
|---|---|---|---|---|---|---|
| Témoins | | | 40 | 1 | 1 | |
| LPS | 0,3 | 1 | 32 | 18 | 9 | 26 |
| Témoins | | | 8 | 0 | | |
| BCG | 100 | 1 | 8 | 1 | 0 | |
| BCG | 100 | 7 | 8 | 6 | 6 | 75 |
| Témoins | | | 24 | 3 | 2 | |
| *C. granulosum* | 300 | 1 | 8 | 0 | | |
| *C. granulosum* | 300 | 7 | 24 | 12 | 11 | 38 |
| Témoins | | | 40 | 9 | 4 | |
| MDP | 100 | 1 | 32 | 6 | 2 | |
| MDP | 1 000 | 1 | 8 | 2 | 1 | |
| MDP | 1 000 | 4 | 8 | 2 | 2 | |
| MDP | 100 | 7 | 16 | 3 | 1 | |
| Témoins | — | — | 24 | 11 | 2 | |
| Mur-NAc-L-Ala-D-isoGln-γ-L-Ala-décyl-ester | 10 | 1 | 8 | 7 | 5 | 50 |
| Mur-NAc-L-Ala-D-isoGln-γ-L-Ala-décyl-ester | 100 | 1 | 24 | 19 | 11 | 38 |
| Mur-NAc-L-Ala-D-isoGln-γ-L-Ala-décyl-ester | 300 | 1 | 16 | 11 | 5 | 23 |

Ces résultats montrent, d'une part, pratiquement l'absence de protection au moyen de MDP dans les conditions de l'expérience et, au contraire, une protection très significative dans le cas des produits selon l'invention, et ceci même pour une dose très faible de produit (10 µg).

6) Essais des formes liposomes

Des essais pharmacologiques analogues aux précédents ont été reproduits avec le MDP, le MDP-L-Ala-eicosyl-ester et le MDP-L-Ala-glycéryl-mycolate en comparant les effets de l'administration sous forme de liposomes à ceux obtenus pour des produits en solution et avec les liposomes sans muramyl-peptide.

Les liposomes étaient préparés de la façon décrite plus haut.

a) Toxicité sur la souris surrénalectomisée

Les produits, les doses administrées et les effets sur la mortalité de groupes de six souris sont indiqués ci-dessous.

| | Nombre de souris mortes |
|---|---|
| Liposomes vides 1/20 ml | 0/6 |
| MDP 100 µg | 0/6 |
| Liposomes MDP 1,3µg (= 1/20 ml) | 0/6 |
| MDP-L-Ala-eicosyl-ester 100 µg | 0/6 |
| Liposomes MDP-L-Ala-eicosyl-ester 100 µg (= 1/20 ml) | 0/6 |
| MDP-L-Ala-glycéryl-mycolate 100 µg | 0/6 |
| Liposomes MDP-L-Ala-glycéryl-mycolate 100 µg (= 1/20 ml) | 0/6 |

On constate, comme précédemment, que la dose létale 50 se situe bien au-delà de 5 mg/kg, que le produit soit administré en solution ou sous forme de liposome.

b) Caractère adjuvant

La série d'essais est réalisée sur des souris dans les mêmes conditions qu'indiqué en 3a) en utilisant le BSA comme antigène.

Les résultats de ces essais sont les suivants :

| | $Log_2$ du titre hémagglutinant |
|---|---|
| Témoins | < 1,64 |
| Liposomes vides | < 1,64 |
| MDP 100 µg | 8,21 ± 0,53 |
| 10 µg | 6,89 ± 2,05 |
| MDP-L-Ala-eicosyl-ester 10 µg | 3,64 ± 1,15 |
| MDP-L-Ala-glycéryl-mycolate 10 µg | 2,07 ± 1,13 |
| Liposomes MDP-L-Ala-eicosyl-ester (10 µg de dérivé MDP) | 2,78 ± 1,35 |
| Liposomes MDP-L-Ala-glycéryl-mycolate (10 µg de dérivé MDP) | 2,07 ± 1,13 |

Une deuxième série d'essais est effectuée sur des rats Wistar, dans les conditions suivantes.

L'antigène est l'ovalbumine injectée par voie sous-cutanée à des rats Wistar mâles sous un volume de 0,5 ml à la dose de 0,5 mg. Les préparations adjuvantes sont injectées, simultanément, à la dose indiquée. Au jour 20, les animaux reçoivent un rappel de 0,5 mg d'antigènes seul par la même voie. Les sérums sont prélevés et testés dans les mêmes conditions que chez la souris avec l'antigènes approprié.

Les résultats obtenus sont :

| | $Log_2$ du titre hémagglutinant |
|---|---|
| Témoins | 5,14 ± 1,38 |
| Liposomes vides | 5,31 ± 2,8 |
| MDP 200 µg | 4,97 ± 0,82 |
| Liposomes MDP 2,5 µg | 7,64 ± 1,24 |
| MDP-L-Ala-glycéryl-mycolate (200 µg) | 7,64 ± 1,90 |
| Liposomes MDP-L-Ala-glycéryl-mycolate | 6,47 ± 2,32 |
| Liposomes eicosyl-ester (200 µg) | 8,64 ± 1,26 |

Une troisième série d'essais est conduite sur des cobayes. Les conditions de ces essais sont les suivantes.

L'antigène est l'ovalbumine injectée en solution aqueuse par voie plantaire à des cobayes Hartley mâles sous un volume de 0,1 ml dans chaque patte postérieure, à la dose de 1 mg par cobaye. Les préparations adjuvantes sont injectées simultanément aux doses indiquées. Après trois semaines, les animaux reçoivent par voie dermique, sous un volume de 0,1 ml, 0,025 mg d'antigène. Le diamètre des réactions cutanées est mesuré après 48 heures. Les animaux sont saignés et les titres mesurés.

Les résultats obtenus sont les suivants :

| | HSR | $Log_2$ du titre hémagglutinant |
|---|---|---|
| Témoins | 0 | 6,64 ± 0,82 |
| MDP 200 µg | 0 | 9,19 ± 1,37 |
| Liposomes MDP 2,5 µg | 4 (5)*0 (6) | 7,37 ± 1,19 |
| MDP-L-Ala-eicosyl-ester 200 µg | 0 | 7,33 ± 1,51 |
| Liposomes MDP-L-Ala-eicosyl-ester 200 µg | 8 (3) 0 (3) | 9,64 ± 0,63 |
| MDP-L-Ala-glycéryl-mycolate | 0 | 8,64 ± 1,83 |
| Liposomes MDP-L-Ala-glycéryl-mycolate 200 µg | 9 ± 2 | 9,64 ± 1,55 |

* Entre parenthèses, nombre d'animaux présentant la réaction indiquée.

L'ensemble de ces résultats montre que l'administration sous forme de liposomes de façon générale conserve l'activité adjuvante des produits et peut même parfois l'améliorer. L'effet le plus sensible se manifeste au niveau des phénomènes d'hypersensibilité retardée.

c) Activité anti-*Klebsiella*

Dans les conditions décrites précédemment, à l'exception de l'infection réalisée par injection intraveineuse de $1,5 \cdot 10^3$ *Klebsiella,* on renouvelle les essais pour déterminer l'action anti-infectieuse. Les administrations sous forme de solution et de liposome ont été comparées.

Les résultats regroupés dans le tableau suivant font apparaître, à teneur égale en composé testé, une amélioration sensible de l'efficacité pour la forme liposome par rapport à la solution.

| Traitement i.v. à J − 1 | | Nombre de souris | Nombre de survivants à J + 3 | J + 5 | J + 8 | % de protection |
|---|---|---|---|---|---|---|
| Témoins | | 24 | 8 | 6 | 6 | — |
| Liposomes vides | 1/20 000 ml | 16 | 3 | 3 | 2 | 0 |
| | 1/2 000 ml | 16 | 5 | 4 | 4 | 0 |
| | 1/2 00 ml | 24 | 10 | 7 | 6 | 0 |
| MDP | 1 µg | 16 | 5 | 3 | 2 | 0 |
| | 10 µg | 16 | 9 | 5 | 5 | 6 |
| | 100 µg | 24 | 21 | 15 | 15 | 38 |
| Liposomes MDP | 0,001 µg | 16 | 0 | 0 | 0 | 0 |
| | 0,01 µg | 16 | 1 | 1 | 1 | 0 |
| | 0,13 µg = 1/200 ml | 16 | 11 | 10 | 10 | 38 |
| MDP-L-Ala-eicosyl-ester | 1 µg | 8 | 4 | 4 | 4 | 25 |
| | 10 µg | 16 | 13 | 9 | 9 | 31 |
| | 100 µg | 16 | 14 | 14 | 14 | 63 |
| Liposomes MDP-L-Ala-eicosyl-ester | 0,1 µg | 24 | 9 | 8 | 8 | 8 |
| | 1 µg | 24 | 10 | 8 | 8 | 8 |
| | 10 µg = 1/200 ml | 24 | 19 | 19 | 19 | 54 |
| MDP-L-Ala-glycéryl-mycolate | 1 µg | 8 | 2 | 2 | 2 | 0 |
| | 10 µg | 16 | 9 | 8 | 8 | 25 |
| | 100 µg | 8 | 8 | 7 | 7 | 63 |
| Liposomes MDP-L-Ala-glycéryl-mycolate | 0,1 µg | 24 | 5 | 4 | 3 | 0 |
| | 1 µg | 24 | 10 | 10 | 10 | 17 |
| | 10 µg | 24 | 15 | 15 | 14 | 33 |

d) Activité anti-*Listeria*

Les essais sont pratiqués dans les conditions du 5. Une dose de *Listeria monocytogène,* connues pour engendrer de façon typique une infection de type cellulaire.

Le traitement des souris et l'inoculation sont effectués par voie intraveineuse. Les produits sont injectés sous forme de liposomes aux doses indiquées. Les témoins ne reçoivent que du soluté isotonique.

La dose de *Listeria* administrée est, dans tous les essais, de $1 \cdot 10^3$ cellules.

Les essais dont les résultats sont donnés ci-après ont été effectués en faisant varier l'intervalle de temps séparant le traitement de l'inoculation des *Listeria monocytogènes* (soit 1, soit 4, soit 8 jours). On suit le nombre de souris survivantes au $5^e$ et au $10^e$ jour suivant l'inoculation, et on détermine le pourcentage de protection comme pour les essais précédents.

(Voir tableau page 34)

| Traitement i.v. | | | | | | | |
|---|---|---|---|---|---|---|---|
| Temps | Produit | Dose µg | Nombre de souris | Nombre de survivants J + 5 | J + 10 | % de protection |
| | Témoins | — | 16 | 6 | 2 | — |
| | Liposomes vides (1/20 ml) | — | 16 | 6 | 3 | — |
| | Liposomes MDP | 1,3 | 16 | 8 | 7 | 25 |
| J − 1 | MDP-L-Ala-eicosyl-ester | 100 | 16 | 3 | 0 | 0 |
| | Liposomes MDP-L-Ala-eicosyl-ester | 100 | 16 | 2 | 0 | 0 |
| | MDP-L-Ala-glycéryl-mycolate | 100 | 8 | 3 | 2 | 0 |
| | Liposomes MDP-L-Ala-glycéryl-mycolate | 100 | 16 | 8 | 6 | 19 |
| | Témoins | — | 16 | 6 | 2 | — |
| | Liposomes vides (1/20 ml) | — | 16 | 8 | 4 | — |
| | Liposomes MDP | 1,3 | 16 | 14 | 5 | 6 |
| J − 4 | MDP-L-Ala-eicosyl-ester | 100 | 16 | 4 | 4 | 0 |
| | Liposomes MDP-L-Ala-eicosyl-ester | 100 | 16 | 9 | 2 | 0 |
| | MDP-L-Ala-glycéryl-mycolate | 100 | 8 | 1 | 0 | 0 |
| | Liposomes MDP-L-Ala-glycéryl-mycolate | 100 | 16 | 14 | 9 | 31 |
| | Témoins | — | 16 | 5 | 2 | — |
| | Liposomes vides (1/20 ml) | — | 16 | 4 | 3 | — |
| | Liposomes MDP | 1,3 | 16 | 6 | 3 | 0 |
| J − 8 | MDP-L-Ala-eicosyl-ester | 100 | 8 | 2 | 2 | 0 |
| | Liposomes MDP-L-Ala-eicosyl-ester | 100 | 16 | 12 | 7 | 25 |
| | MDP-L-Ala-glycéryl-mycolate | 100 | 8 | 1 | 0 | 0 |
| | Liposomes MDP-L-Ala-glycéryl-mycolate | 100 | 16 | 11 | 10 | 44 |

Ces résultats montrent que la protection par liposome, dans les conditions de l'expérience, des produits selon l'invention est fonction du moment auquel ils sont administrés par rapport à l'infection. Ils montrent aussi que l'administration sous forme de liposome est susceptible d'engendrer une protection non négligeable dans des conditions pour lesquelles le produit administré en solution ne manifeste pas d'activité anti-*Listeria*.

**Revendications**

1. Composé de formule générale

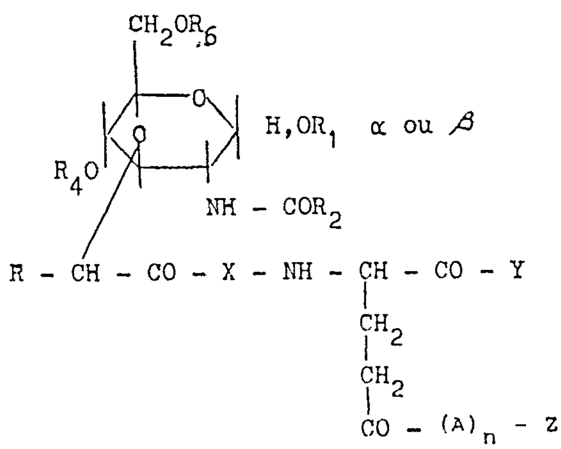

dans laquelle les substituants R, $R_1$, $R_2$, $R_4$, $R_6$, X, Y et Z ont l'une des significations suivantes :

— R est soit un atome d'hydrogène soit un groupe méthyle,

— $R_1$ est un atome d'hydrogène, un groupe alcoyle ayant au plus 4 atomes de carbone, un groupe aryle ou alcoyl-aryle simple ou substitué comprenant au plus 10 atomes de carbone,

— $R_2$ est un groupe méthyle,

— $R_4$ est un atome d'hydrogène, un radical acyle comprenant au plus 4 atomes de carbone,

— $R_6$ est un atome d'hydrogène, ou un radical acyle saturé ou non, éventuellement ramifié, substitué

ou non, contenant de 1 à 90 atomes de carbone, et pouvant en outre porter des groupes fonctionnels : hydroxyle, carboxyle, carbonyle, amino, cyclopropane, méthoxyle,

— X est un résidu aminoacyle du groupe comprenant : L-alanyle, L-arginyle, L-asparagyle, L-aspartyle, L-cystéinyle, L-glutaminyle, L-glutamyle, glycyle, L-histidyle, L-hydroxy-prolyle, L-isoleucyle, L-leucyle, L-lysyle, L-méthionyle, L-ornithyle, L-phénylalanyle, L-prolyle, L-séryle, L-thréonyle, L-tryptophanyle, L-tyrosyle et L-valyle,

— Y est soit —OH, soit un radical alcoxy comprenant de 1 à 10 atomes de carbone, soit $—NH_2$, les hydrogènes du groupe amino pouvant être substitués par des restes alcoyle de 1 à 10 atomes de carbone, soit un résidu aminoacyle,

— A est un résidu aminoacyle du groupe indiqué ci-dessus pour X, ou, pour le dernier de la chaîne peptidique, un résidu d'aminoalcool $(—NH—\underset{|}{C}H—CH_2—O—)$ correspondant à ces aminoacyles $(—NH—\underset{|}{C}H—CO—)$ étant entendu que les groupes A présents dans un même composé peuvent être identiques ou différents, n ne représentant que le nombre total de groupes A dans ce composé,

— n est nul ou 1, 2 ou 3,

— Z est un groupe —OR', —NHR', $—OCH_2—CH_2O—COR'$ ou $—OCH_2—CHOH—CH_2O—COR'$ lorsque le dernier résidu A de la chaîne peptidique est un aminoacyle, ou un groupe COR' lorsque ce dernier résidu A est un aminoalcool, dans lequel R' est un alcoyle linéaire ou ramifié, saturé ou non, et peut contenir des groupes fonctionnels hydroxyle, carbonyle, carboxyle, cyclopropane, aryle, ce dernier éventuellement substitué, ce groupe comprenant au moins 4 carbones et pouvant renfermer jusqu'à 90 atomes de carbone, sous réserve que lorsque R est de l'hydrogène, le groupement $(A)_n—Z$ soit un groupe $—NH—CH(CH_3)—COO—CH_2—CHOH—CH_2—O—R°$, avec R° correspondant à un acide mycolique comprenant de 80 à 90 atomes de carbone.

2. Composé selon la revendication 1, caractérisé en ce que X est un résidu aminoacyle du groupe comprenant L-alanyle, L-séryle, glycyle, L-prolyle, L-thréonyle et L-valyle.

3. Composé selon l'une quelconque des revendications précédentes, caractérisé en ce que le ou les A sont des résidus aminoacyles du groupe comprenant : alanyle, leucyle, lysyle, glycyle, valyle, isoleucyle.

4. Composé selon la revendication 3, caractérisé en ce que le premier aminoacyle A fixé à la fonction γ-carbonyle du résidu D-glutamyle est le résidu L-alanyle, le résidu L-lysyle ou le résidu L-glutamyle.

5. Composé selon l'une quelconque des revendications 3 et 4, caractérisé en ce que n = 1 ou 2.

6. Composé selon l'une quelconque des revendications précédents, caractérisé en ce que Y est un groupe —OH, $—OCH_3$, $—OC_4H_9$ ou $—NH_2$.

7. Composé selon l'une quelconque des revendications précédentes, caractérisé en ce que $R_4$ est un atome d'hydrogène.

8. Composé selon l'une quelconque des revendications 1 à 6, caractérisé en ce que $R_4$ est un groupe $—CO—(CH_2)_2—CO_2H$ ou $—COCH_3$.

9. Composé selon l'une quelconque des revendications précédentes, caractérisé en ce que $R_6$ est un hydrogène.

10. Composé selon l'une quelconque des revendications 1 à 8, caractérisé en ce que $R_6$ est un radical acyle comprenant de 1 à 4 atomes de carbone.

11. Composé selon la revendication 10, caractérisé en ce que $R_6$ est le groupe $—COCH_3$ ou $—CO(CH_2)_2—CO_2H$.

12. Composé selon l'une quelconque des revendications 1 à 8, caractérisé en ce que $R_6$ est un groupe mycoloyle ($C_{80}$ à $C_{90}$) ou corynomycoloyle ($C_{32}$).

13. Composé selon l'une quelconque des revendications précédentes, caractérisé en ce que R est un hydrogène.

14. Composé selon l'une quelconque des revendications 1 à 12, caractérisé en ce que R et $R_2$ sont tous deux des groupes $—CH_3$.

15. Composé selon la revendication 14, caractérisé en ce que R' est un groupe alkyle contenant de 5 à 90 atomes de carbone, pouvant porter des groupes fonctionnels hydroxyle, carboxyle, carbonyle, cyclopropane et méthoxyle.

16. Composé selon la revendication 14, caractérisé en ce que le résidu $(A)_n—Z$ est l'un des suivants :

$—NH—CH(CH_3)—COO—(CH_2)_{19}—CH_3$
$—NH—CH(CH_3)—CONH—(CH_2)_9—CH_3$
$—NH—CH(CH_3)—COO—(CH_2)_9—CH_3$
$—NH—CH(CH_3)—COO—(CH_2)_{14}—CH_3$
$—NH—CH(CH_3)—COO—(CH_2)_3—CH_3$
$—NH—CH(CH_3)—COO—CH_2—C_6H_5$
$—NH—CH(CH_3)—COO—CH_2—CHOH—CH_2—O—R°$, avec R° correspondant à un acide mycolique comprenant de 80 à 90 carbones,
$—NH—CH\ [(CH_2)_4—NH_2]—COO—(CH_2)_9—CH_3$
$—O—(CH_2)_3—CH_3$
$—O—(CH_2)_9—CH_3$

17. Composé selon la revendication 1, constitué par l'un des produits suivants :
— le N-acétyl-muramyl-L-alanyl-D-isoglutaminyl-décyl-ester de formule

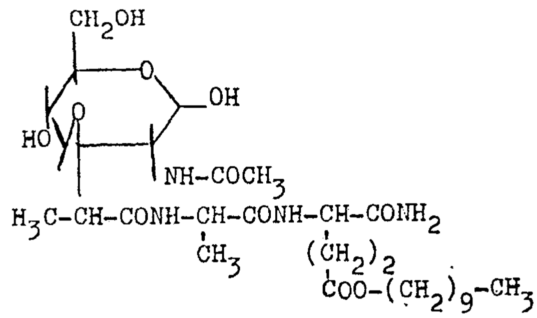

— le β-D-p.aminophényl-N-acétyl-muramyl-L-alanyl-D-isoglutaminyl-L-alanyl-décyl-ester de formule

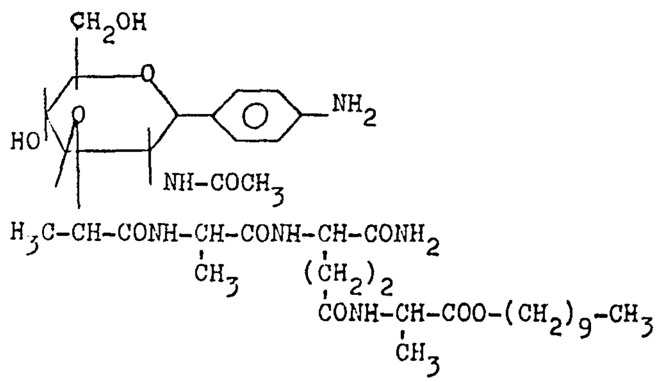

— le N-acétyl-muramyl-L-alanyl-D-isoglutaminyl-L-alanyl-pentadécyl-ester de formule

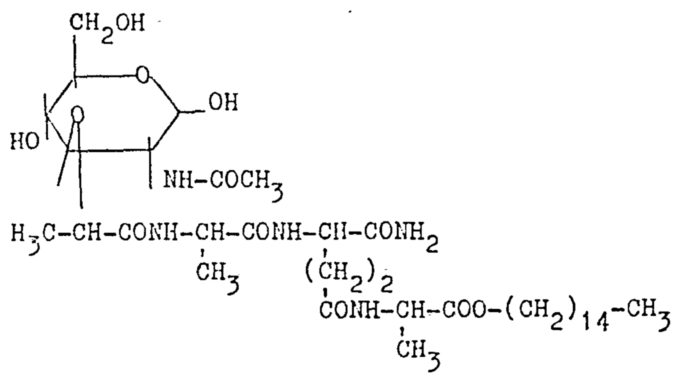

— le N-acétyl-muramyl-L-alanyl-D-isoglutaminyl-L-alanyl-benzyl-ester de formule

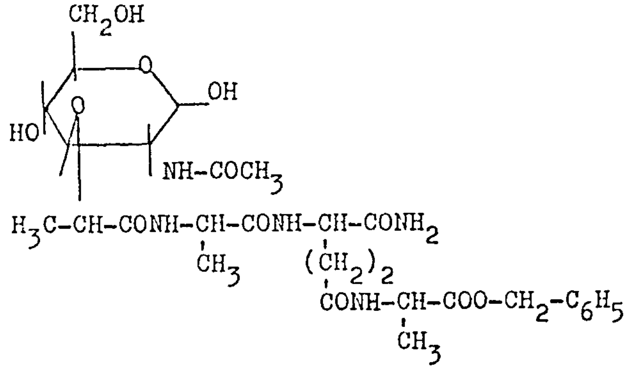

— le N-acétyl-muramyl-L-alanyl-D-isoglutaminyl-L-alanyl-D-glycéryl-mycolate de formule

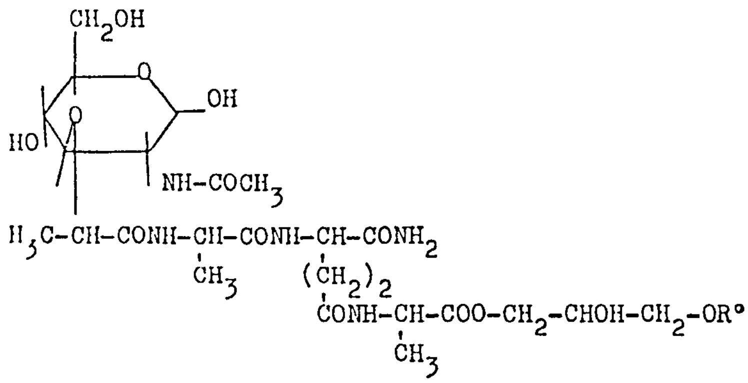

R° étant un radical d'acide mycolique contenant de 80 à 90 atomes de carbone ;
— le N-acétyl-muramyl-L-alanyl-D-isoglutaminyl-L-lysine-décyl-ester de formule

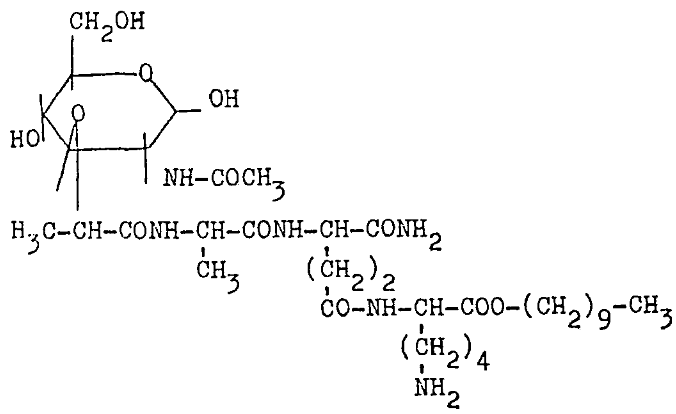

— le N-acétyl-muramyl-L-alanyl-D-isoglutaminyl-butyl-ester de formule

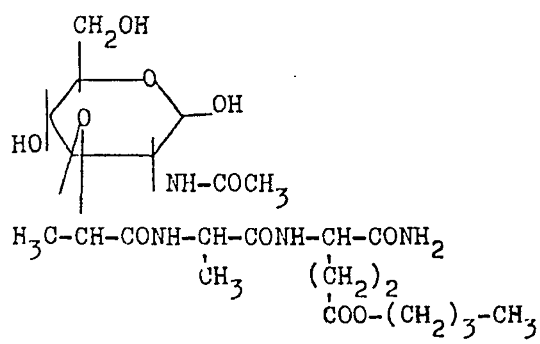

— le N-acétyl-muramyl-L-alanyl-D-glutamyl-α-méthyl-ester-γ-butyl-ester de formule

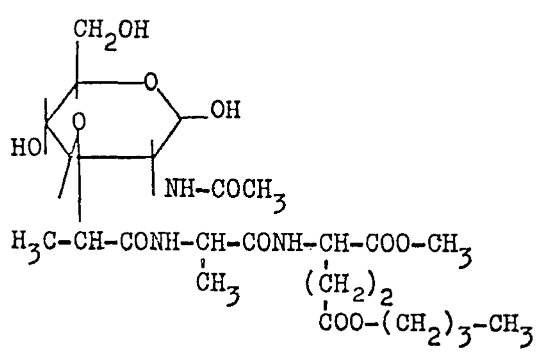

— le N-acétyl-muramyl-L-alanyl-D-glutamyl-α-méthyl-ester-γ-décyl-ester de formule

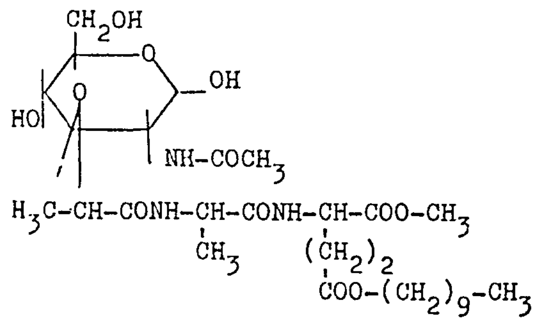

18. Composition pharmaceutique, caractérisée en ce qu'elle contient une dose efficace d'au moins un composé de formule (I) selon l'une quelconque des revendications 1 à 17.

19. Composition pharmaceutique selon la revendication 18, caractérisée en ce que le ou les composés (I) sont associés à des véhicules ou excipients pharmaceutiquement acceptables.

20. Composition pharmaceutique selon la revendication 18 ou la revendication 19, constituée par une solution ou suspension du composé (I) dans un milieu injectable.

21. Composition pharmaceutique selon l'une des revendications 18 à 20, caractérisée en ce que ladite composition est présentée sous forme de liposomes.

22. Composition selon la revendication 21, caractérisée en ce que la partie lipidique des liposomes est constituée par un phospholipide.

23. Composition pharmaceutique selon la revendication 22, caractérisée en ce que la partie lipidique des liposomes est constituée par de la lécithine et du cholestérol.

24. Composition pharmaceutique selon la revendication 23, caractérisée en ce que les constituants de la phase lipidique sont dans des rapports molaires entre 8 : 1 et 1 : 1 lécithine/cholestérol.

25. Composition pharmaceutique selon l'une quelconque des revendications 21 à 24, caractérisée en ce que la phase aqueuse est tamponnée à un pH voisin de la neutralité.

26. Composition pharmaceutique selon la revendication 25, caractérisée en ce que la phase aqueuse est tamponnée au phosphate.

27. Composition pharmaceutique selon la revendication 20, caractérisée en ce que le milieu injectable est constitué par une solution aqueuse pour injection, notamment une solution isotonique saline ou glucosée et stérile.

28. Composition pharmaceutique selon la revendication 18 ou la revendication 19, caractérisée en ce que le composé (I) est associé à des excipients adaptés à l'administration orale.

29. Composition pharmaceutique selon la revendication 18 ou la revendication 19, caractérisée en ce que le composé (I) est associé à des excipients permettant l'administration par application sur les muqueuses oculaires ou les voies respiratoires, l'administration vaginale ou l'administration rectale.

30. Composition pharmaceutique selon la revendication 20 ou la revendication 21, caractérisée en ce que, pour un traitement anti-infectieux et une administration parentérale, les doses unitaires contiennent de 10 à 1 000 μg de composé (I).

31. Composition pharmaceutique selon la revendication 28, caractérisée en ce que, pour un traitement anti-infectieux et une administration orale, les doses unitaires contiennent de 200 à 20 000 μg de composé (I).

32. Réactif de laboratoire comprenant au moins un composé selon l'une quelconque des revendications 1 à 17.

## Claims

1. Compound of the general formula

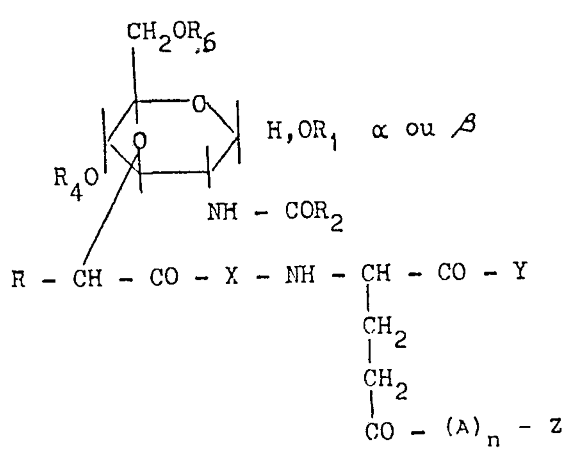

in which the substituents R, $R_1$, $R_2$, $R_4$, $R_6$, X, Y and Z have one of the following meanings :

— R is either a hydrogen atom, or a methyl group,

— $R_1$ is a hydrogen atom, an alkyl group having at most 4 carbon atoms, a simple or substituted aryl or alkyl-aryl group comprising at the most 10 carbon atoms,

— $R_2$ is a methyl group,

— $R_4$ is a hydrogen atom, an acyl radical comprising at most 4 carbon atoms,

— $R_6$ is a hydrogen atom, or an acyl radical, saturated or not, optionally branched, substituted or not, containing from 1 to 90 carbon atoms, and also optionally carrying functional groups : hydroxyl, carboxyl, carbonyl, amino, cyclopropane, methoxyl,

— X is an aminoacyl residue of the group comprising : L-alanyl, L-arginyl, L-asparagyl, L-aspartyl, L-cysteinyl, L-glutaminyl, L-glutamyl, glycyl, L-histidyl, L-hydroxy-prolyl, L-isoleucyl, L-leucyl, L-lysyl, L-methionyl, L-ornithyl, L-phenylalanyl, L-prolyl, L-seryl, L-threonyl, L-tryptophanyl, L-tyrosyl and L-valyl,

— Y is either —OH, or an alkoxy radical comprising from 1 to 10 carbon atoms, or $—NH_2$, the hydrogens of the amino group being optionally substituted by alkyl residues of 1 to 10 carbon atoms, or an aminoacyl residue,

— A is an aminoacyl residue of the group indicated above for X, or for the last of the peptide chain, an aminoalcohol residue ($—NH—CH—CH_2—O$) corresponding to these aminoacyls (—NH—CH—CO—) it being understood that the A groups present in a same compound may be identical or different, whereby n only represents the total number of A groups in this compound,

— n is zero or 1, 2 or 3,

— Z is a group —OR', —NHR', $—OCH_2—CH_2O—COR'$ or $—OCH_2—CHOH—CH_2O—COR'$ when the last A residue of the peptidic chain is an aminoalcohol, in which R' is a linear or branched alkyl saturated or not, and optionally containing functional groups hydroxyl, carbonyl, carboxyl, cyclopropane, aryl, the latter being optionally substituted, this group comprising at least 4 carbons and being likely to include up to 90 carbon atoms, with the proviso that when R is hydrogen, the $(A)_n—Z$ group is a $—NH—CH(CH_3)—COO—CH_2—CHOH—CH_2—O—R°$ group, with R° being a mycolic acid comprising from 80 to 90 carbon atoms.

2. Compound according to claim 1, characterized in that X is an aminoacyl residue from the group comprising L-alanyl, L-seryl, glycyl, L-prolyl, L-threonyl and L-valyl.

3. Compound according to any one of the preceding claims characterized in that the one or several A are aminoacyl residues from the group comprising : alanyl, leucyl, lysyl, glycyl, valyl, isoleucyl.

4. Compound according to claim 3, characterized in that the first aminoacyl residue A linked to the $\gamma$-carboxyl function of the D-glutamyl residue is the L-alanyl residue, the L-lysyl residue or the L-glutamyl residue.

5. Compound according to any one of claims 3 and 4, characterized in that $n = 1$ or 2.

6. Compound according to any one of the preceding claims, characterized in that Y is an —OH, $—OCH_3$, $—OC_4H_9$ or $—NH_2$ group.

7. Compound according to any one of the preceding claims, characterized in that $R_4$ is a hydrogen atom.

8. Compound according to any one of claims 1 to 6, characterized in that $R_4$ is a $—CO—(CH_2)_2—CO_2H$ group or a $—COCH_3$ group.

9. Compound according to any one of the preceding claims, characterized in that $R_6$ is hydrogen.

10. Compound according to any one of claims 1 to 8 characterized in that $R_6$ is an acyl radical comprising from 1 to 4 carbon atoms.

11. Compound according to claim 10 characterized in that $R_6$ is the $—COCH_3$ or $—CO(CH_2)_2$-$CO_2H$ group.

12. Compound according to any one of claims 1 to 8 wherein $R_6$ is a mycoloyl group ($C_{80}$ to $C_{90}$) or corynomycoloyl group ($C_{32}$).

13. Compound according to any one of the preceding claims characterized in that R is hydrogen.

14. Compound according to any one of claims 1 to 12, characterized in that both R and $R_2$ are $—CH_3$ groups.

15. Compound according to claim 14 characterized in that R' is an alkyl group comprising from 5 to 90 carbon atoms, optionally bearing hydroxyl, carboxyl, carbonyl, cyclopropan and methoxyl functional groups.

16. Compound according to claim 14, characterized in that the residue $(A)_n—Z$ is one of the following :

$—NH—CH(CH_3)—COO—(CH_2)_{19}—CH_3$
$—NH—CH(CH_3)—CONH—(CH_2)_9—CH_3$
$—NH—CH(CH_3)—COO—(CH_2)_9—CH_3$
$—NH—CH(CH_3)—COO—(CH_2)_{14}—CH_3$
$—NH—CH(CH_3)—COO—(CH_2)_{14}—CH_3$
$—NH—CH(CH_3)—COO—(CH_2)_3—CH_3$
$—NH—CH(CH_3)—COO—CH_2—C_6—H_5$

$-NH-CH(CH_3)-COO-CH_2-CHOH-CH_2-O-R°$, with R° corresponding to a mycolic acid comprising from 80 to 90 carbon atoms,
$-NH-CH\ [(CH_2)_4-NH_2]-COO-(CH_2)_9-CH_3$
$-O-(CH_2)_3-CH_3$
$-O-(CH_2)_9-CH_3$.

17. Compound according to claim 1, constituted by one of the following products :
— N-acetyl-muramyl-L-alanyl-D-isoglutaminyl-decyl-ester of formula

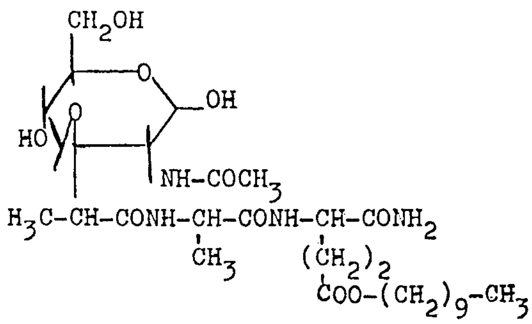

— β-D-p-aminophenyl-N-acetyl-muramyl-L-alanyl-D-isoglutaminyl-L-alanyl-decyl-ester of formula

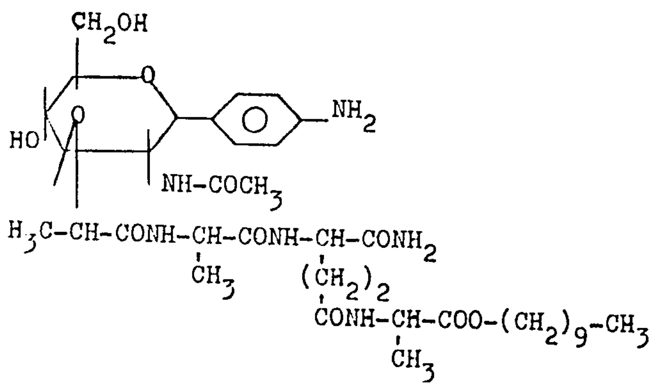

— N-acetyl-muramyl-L-alanyl-D-isoglutaminyl-L-alanyl-pentadecyl-ester of formula

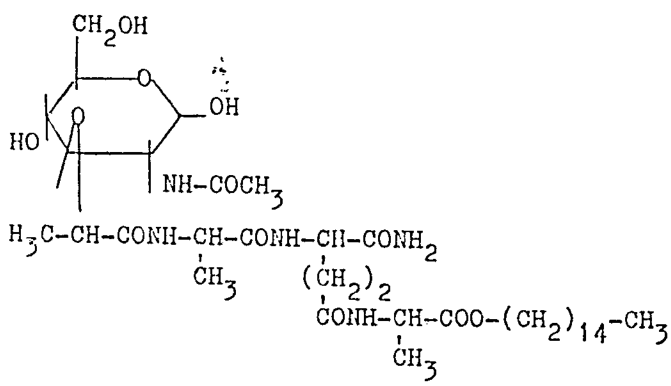

— N-acetyl-muramyl-L-alanyl-D-isoglutaminyl-L-alanyl-benzyl-ester of formula

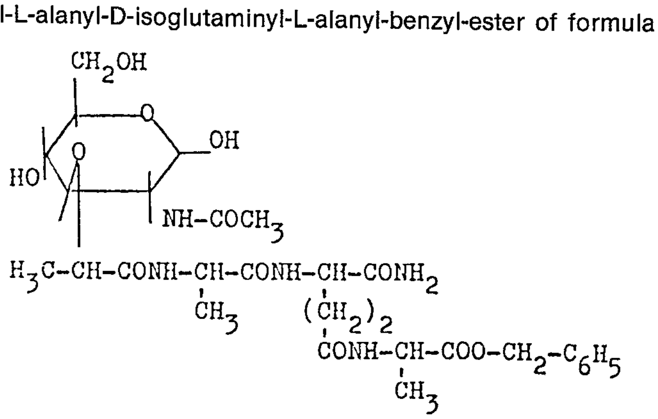

— N-acetyl-muramyl-L-alanyl-D-isoglutaminyl-L-alanyl-D-glyceryl-mycolate of formula

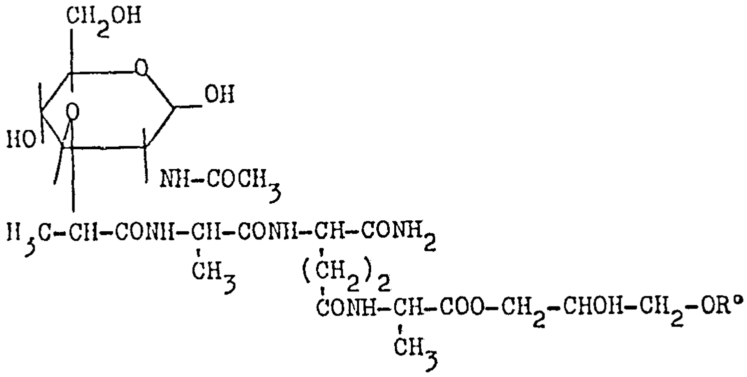

R° being a mycolic acid radical containing from 80 to 90 carbon atoms ;

— N-acetyl-muramyl-L-alanyl-D-isoglutaminyl-L-lysine-decyl-ester of formula

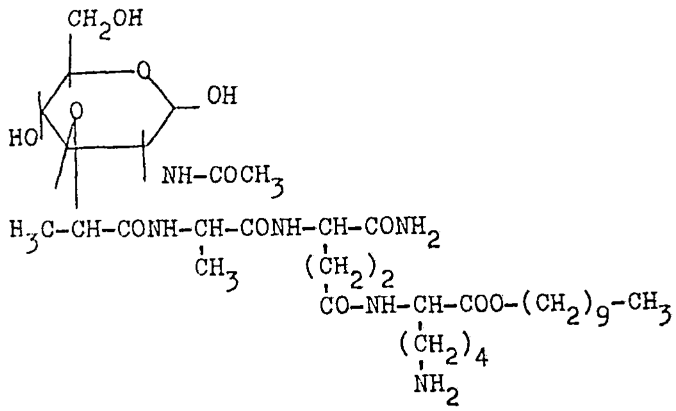

— N-acetyl-muramyl-L-alanyl-D-isoglutaminiyl-butyl-ester of formula

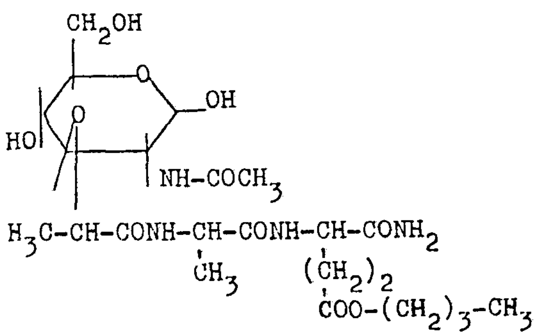

— N-acetyl-muramyl-L-alanyl-D-glutamyl-α-methyl-ester-γ-butyl-ester of formula

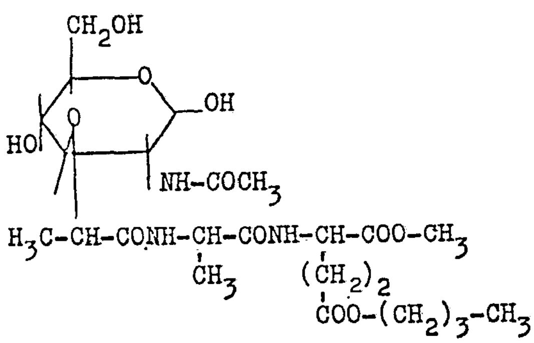

— N-acetyl-muramyl-L-alanyl-D-glutamyl-α-methyl-ester-γ-decyl-ester of formula

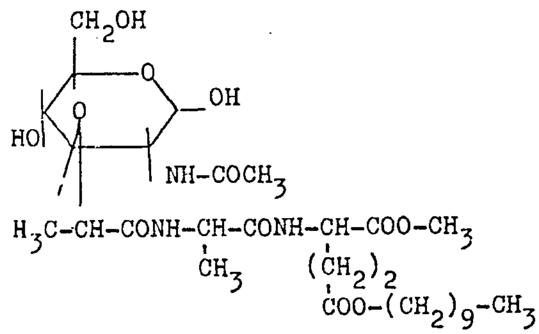

18. Pharmaceutical composition, characterized in that it contains an effective dose of at least one compound of formula (I) according to any one of claims1 to 17.

19. Pharmaceutical composition according to claim 18, characterized in that the one or several compounds (I) are associated with pharmaceutically acceptable vehicles or excipients.

20. Pharmaceutical composition according to claim 18 or claim 19, constituted by a solution or suspension of compound (I) in an injectable medium.

21. Pharmaceutical composition according to one of claim 18 to 20, characterized in that said composition is presented in the form of liposomes.

22. Composition according to claim 21, characterized in that the lipidic part of the liposomes is constituted by a phospholipid.

23. Pharmaceutical composition according to claim 22, characterized in that the lipidic part of the liposomes is constituted by lecithin and cholesterol.

24. Pharmaceutical composition according to claim 23, characterized in that the constituents of the lipidic phase are in molar ratios between 8 : 1 and 1 : 1 lecithin/cholesterol.

25. Pharmaceutical composition according to any one of claims 21 to 24, characterized in that the aqueous phase is buffered to a pH close to neutrality.

26. Pharmaceutical composition according to claim 25, wherein the aqueous phase is buffered with phosphate.

27. Pharmaceutical composition according to claim 20, characterized in that the injectable medium is constituted by an aqueous solution for injection, particularly a saline or glucosed isotonic and sterile solution.

28. Pharmaceutical composition according to claim 18 or claim 19, characterized in that compound (I) is associated with excipients adapted for oral administration.

29. Pharmaceutical composition according to claim 18 or claim 19, characterized in that compound (I) is associated with excipients permitting administration by application to the ocular mucous membranes or to the respiratory tracts, vaginal administration or rectal administration.

30. Pharmaceutical composition according to claim 20 or claim 21, characterized in that, for an anti-infectious treatment and parenteral administration, the unit doses contain from 10 to 1,000 μg of compound (I).

31. Pharmaceutical composition according to claim 28, characterized in that, for an anti-infectious treatment and oral administration, the unit doses contain from 200 to 20,000 μg of compound (I).

32. Laboratory reagent comprising at least one compound according to any one of claims 1 to 17.

**Ansprüche**

1. Verbindung der allgemeinen Formel

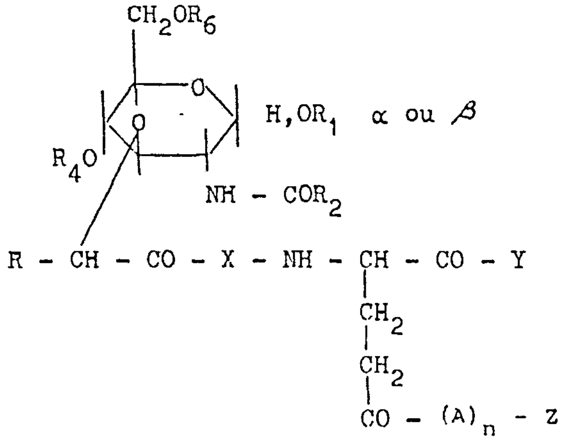

worin die Substituenten R, $R_1$, $R_2$, $R_4$, $R_6$, X, Y und Z die folgenden Bedeutungen besitzen :

— R kann ein Wasserstoffatom oder eine Methylgruppe sein,

— $R_1$ ist ein Wasserstoffatom, eine Alkylgruppe mit bis zu 4 Kohlenstoffatomen, eine Aryl- oder Alkylarylgruppe die unsubstituiert oder substituiert und bis zu 10 Kohlenstoffatome enthalten kann,

— $R_2$ ist eine Methylgruppe,

— $R_4$ ist ein Wasserstoffatom, ein Acylradikal mit bis zu 4 Kohlenstoffatomen,

— $R_6$ ist ein Wasserstoffatom, ein gesättigtes oder ungesättigtes, gegebenenfalls verzweigtes, substituiertes oder nicht-substituiertes Acylradical mit 1 bis 90 Kohlenstoffatomen, das weiters die folgenden funktionellen Gruppen tragen kann : Hydroxyl, Carboxyl, Carbonyl, Amino, Cyclopropan, Methoxyl,

— X ist ein Aminoacylrest aus der folgenden Gruppe : L-Alanyl, L-Arginyl, L-Asparaginyl, L-Aspartyl, L-Cysteinyl, L-Glutaminyl, L-Glutamyl, Glycyl, L-Histidyl, L-Hydroxy-prolyl, L-Isoleucyl, L-Leucyl, L-Lysyl, L-Methionyl, L-Ornithyl, L-Phenylalanyl, L-Prolyl, L-Seryl, L-Threonyl, L-Tryptophyl, L-Tyrosyl und L-Valyl,

— Y ist —OH, ein Alkoxyradical mit 1 bis 10 Kohlenstoffatomen, oder —$NH_2$, wobei die Wasserstoffatome der Aminogruppe durch Alkylreste mit 1 bis 10 Kohlenstoffatomen substituiert sein können, oder ein Aminoacylrest,

— A ist ein Aminoacylrest aus der oben für X angegebenen Gruppe, oder als letztes Glied der Peptidkette ein Rest eines diesen Aminoacylen (—NH—CH—CO—) entsprechenden Aminoalkohols (—NH—CH—$CH_2$—O—), wobei die in der gleichen Verbindung vorhandenen Gruppen A gleich oder verschieden sein können, und n nur die Gesamtzahl der Gruppen A in der Verbindung angibt,

— n ist 0, 1, 2 oder 3,

— Z ist eine Gruppe —OR', —NHR', —$OCH_2$—$CH_2O$—COR' oder —$OCH_2$—CHOH—$CH_2O$—COR', wenn der letzte Rest der Peptidkette ein Aminoacyl ist, oder eine Gruppe COR' wenn der letzte Rest A ein Aminoalkohol ist, worin R' ein geradkettiges oder verzweigtes, gesättigtes oder ungesättigtes Alkyl ist, das die funktionellen Gruppen Hydroxyl, Carbonyl, Carboxyl, Cyclopropan und Aryl enthält, wobei letztere gegebenenfalls substituiert ist, und die Gruppe mindestens 4 Kohlenstoffatome und bis zu 90 Kohlenstoffatome enthalten kann, mit der Ausnahme, daß wenn R Wasserstoff ist, die Gruppierung $(A)_n$—Z eine Gruppe —NH—CH($CH_3$)—COO—$CH_2$—CHOH—$CH_2O$—R° ist, worin R° einer Mycolsäure mit 80 bis 90 Kohlenstoffatomen entspricht.

2. Verbindung nach Anspruch 1, dadurch gekennzeichnet, daß X ein Aminoacylrest aus der Gruppe L-Alanyl, L-Seryl, Glycyl, L-Prolyl, L-Threonyl und L-Valyl ist.

3. Verbindung nach einem der vorhergehenden Ansprüche, dadurch gekennzeichnet, daß die Gruppe oder die Gruppen A Aminoacylreste aus der Gruppe Alanyl, Leucyl, Lysyl, Glycyl, Valyl und Isoleucyl sind.

4. Verbindung nach Anspruch 3, dadurch gekennzeichnet, daß das erste an die γ-Carboxylfunktion des D-Glutamylrestes fixierte Aminoacyl A der Rest L-Alanyl, L-Lysyl oder L-Glutamyl ist.

5. Verbindung nach Anspruch 3 oder 4, dadurch gekennzeichnet, daß n 1 oder 2 ist.

6. Verbindung nach einem der vorhergehenden Ansprüche, dadurch gekennzeichnet, daß Y eine —OH, —$OCH_3$, —$OC_4H_9$ oder —$NH_2$ Gruppe ist.

7. Verfahren nach einem der vorhergehenden Ansprüche, dadurch gekennzeichnet, daß $R_4$ ein Wasserstoffatom ist.

8. Verbindung nach einem der Ansprüche 1 bis 6, dadurch gekennzeichnet, daß $R_4$ eine Gruppe —CO—$(CH_2)_2$—$CO_2H$ oder —$COCH_3$ ist.

9. Verbindung nach einem der vorhergehenden Ansprüche, dadurch gekennzeichnet, daß $R_6$ Wasserstoff ist.

10. Verbindung nach einem der Ansprüche 1 bis 8, dadurch gekennzeichnet, daß $R_6$ ein Acylradikal mit 1 bis 4 Kohlenstoffatomen ist.

11. Verbindung nach Anspruch 10, dadurch gekennzeichnet, daß $R_6$ die Gruppe —$COCH_3$ oder —$CO(CH_2)_2$—$CO_2H$ ist.

12. Verbindung nach einem der Ansprüche 1 bis 8, dadurch gekennzeichnet, daß $R_6$ eine ($C_{80}$ bis $C_{90}$)-Mycoloyl- oder ($C_{32}$)-Corynomycoloyl-Gruppe ist.

13. Verbindung nach einem der vorhergehenden Ansprüche, dadurch gekennzeichnet, daß R Wasserstoff ist.

14. Verbindung nach einem der Ansprüche 1 bis 12, dadurch gekennzeichnet, daß R und $R_2$ beide $CH_3$— Gruppen sind.

15. Verbindung nach Anspruch 14, dadurch gekennzeichnet, daß R' eine Alkylgruppe mit 5 bis 90 Kohlenstoffatomen ist, die die funktionellen Gruppen Hydroxyl, Carboxyl, Carbonyl, Cyclopropan und Methoxyl tragen kann.

16. Verbindung nach Anspruch 14, dadurch gekennzeichnet, daß der Rest $(A)_n$—Z einer der folgenden ist :

—NH—CH($CH_3$)—COO—$(CH_2)_{19}$—$CH_3$

—NH—CH($CH_3$)—CONH—$(CH_2)_9$—$CH_3$

$-NH-CH(CH_3)-COO-(CH_2)_9-CH_3$
$-NH-CH(CH_3)-COO-(CH_2)_{14}-CH_3$
$-NH-CH(CH_3)-COO-(CH_2)_3-CH_3$
$-NH-CH(CH_3)-COO-CH_2-C_6H_5$
$-NH-CH(CH_3)-COO-CH_2-CHOH-CH_2-O-R°$, worin R° einer Mycolsäure mit 80 bis 90 Kohlenstoffatomen entspricht,
$-NH-CH\ [(CH_2)_4-NH_2]-COO-(CH_2)_9-CH_3$
$-O-(CH_2)_3-CH_3$
$-O-(CH_2)_9-CH_3$

17. Verbindung nach Anspruch 1, dargestellt durch eine der folgenden Produkte :

— N-Acetyl-muramyl-L-alanyl-D-isoglutaminyl-decyl-ester der Formel

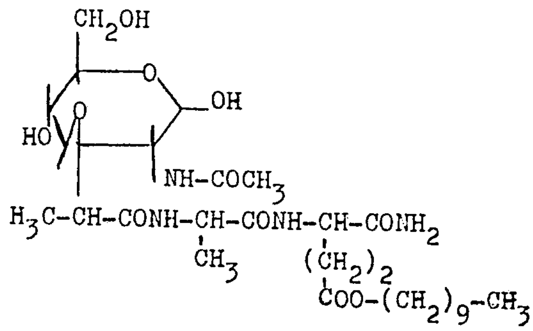

— β-D-p-Aminophenyl-N-acetyl-muramyl-L-alanyl-D-isoglutaminyl-L-alanyl-decyl-ester der Formel

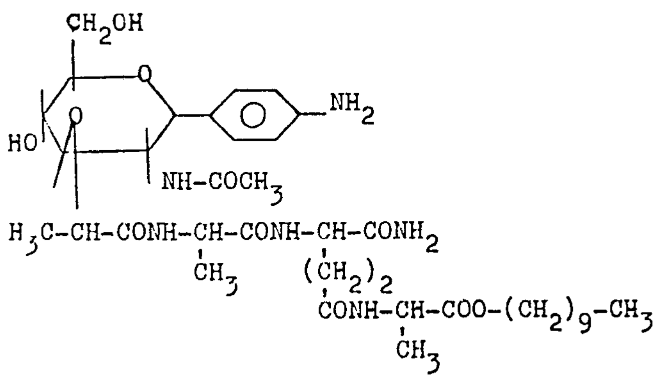

— N-Acetyl-muramyl-L-alanyl-D-isoglutaminyl-L-alanyl-pentadecyl-ester der Formel

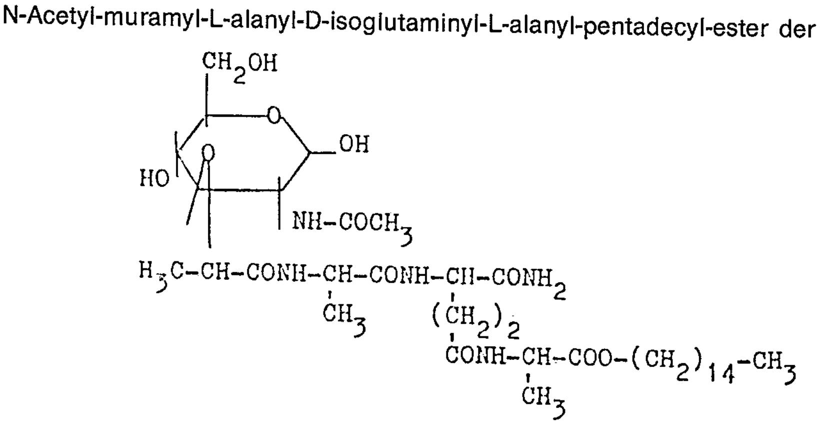

— N-Acetyl-muramyl-L-alanyl-D-isoglutaminyl-L-alanylbenzyl-ester der Formel

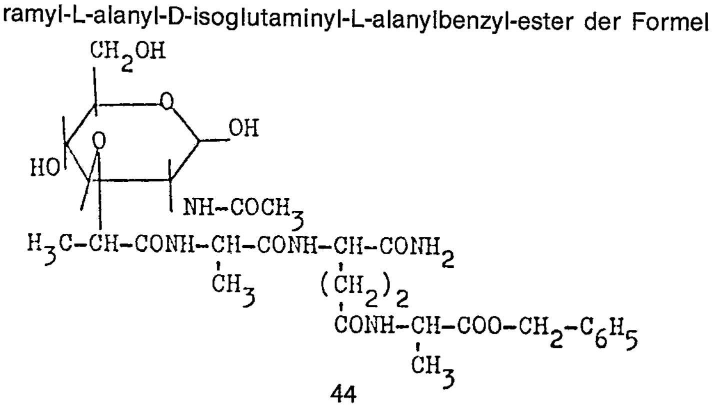

— N-Acetyl-muramyl-L-alanyl-D-isoglutaminyl-L-alanyl-D-glyceryl-mycolat der Formel

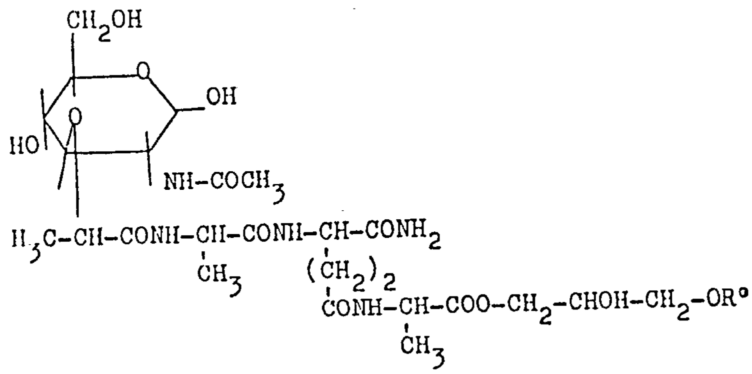

worin

R° ein Mycolsäureradical mit 80 bis 90 Kohlenstoffatomen ist ;

— N-Acetyl-muramyl-L-alanyl-D-isoglutaminyl-L-lysindecyl-ester der Formel

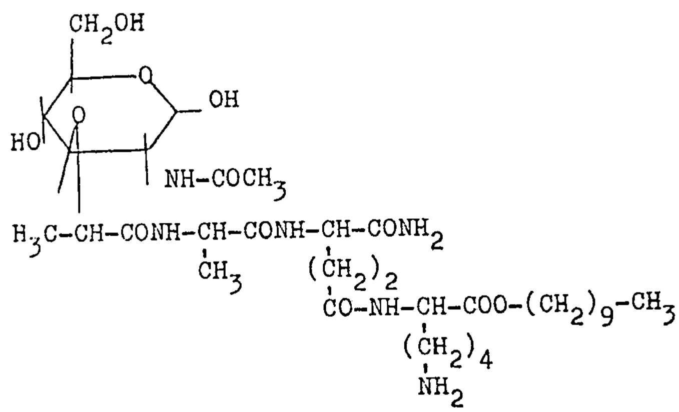

— N-Acetyl-muramyl-L-alanyl-D-isoglutaminyl-butyl-ester der Formel

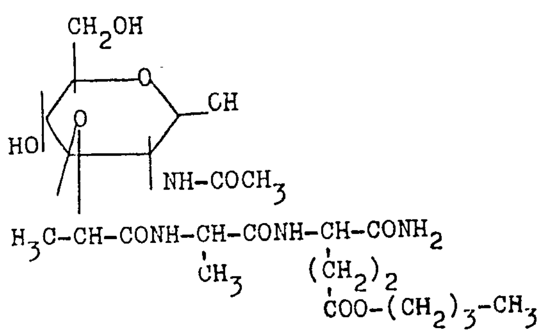

— N-Acetyl-muramyl-L-alanyl-D-glutamyl-α-methyl-ester-γ-butyl-ester der Formel

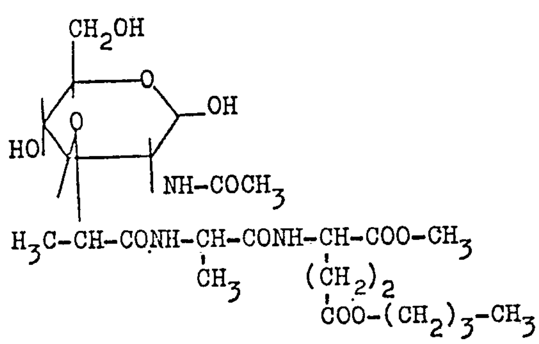

— N-Acetyl-muramyl-L-alanyl-D-glutamyl-α-methyl-ester-γ-decyl-ester der Formel

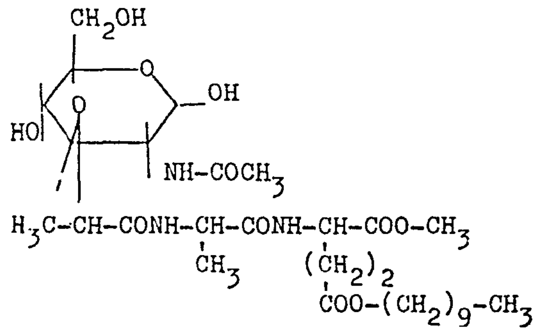

18. Pharmazeutische Zusammensetzung, dadurch gekennzeichnet, daß sie eine wirksame Dosis mindestens einer der Verbindungen der Formel (I) gemäß einem der Ansprüche 1 bis 17 enthält.

19. Pharmazeutische Zusammensetzung nach Anspruch 18, dadurch gekennzeichnet, daß sie die Verbindungen (I) zusammen mit Trägern oder pharmazeutisch annehmbaren Exzipienten enthält.

20. Pharmazeutische Zusammensetzung nach Anspruch 18 oder 19, dadurch gekennzeichnet, daß sie eine Lösung oder Suspension der Verbindung (I) in einem injizierbaren Medium enthält.

21. Pharmazeutische Zusammensetzung nach einem der Ansprüche 18 bis 20, dadurch gekennzeichnet, daß die Zusammensetzung in Form von Liposomen vorliegt.

22. Zusammensetzung nach Anspruch 21, dadurch gekennzeichnet, daß der Lipidteil der Liposomen aus Phosphorlipid gebildet wird.

23. Pharmazeutische Zusammensetzung nach Anspruch 22, dadurch gekennzeichnet, daß der Lipidteil der Liposomen durch Lecithin und Cholesterin gebildet wird.

24. Pharmazeutische Zusammensetzung nach Anspruch 23, dadurch gekennzeichnet, daß die Bestandteile der lipiden Phase im Molverhältnis Lecithin/Cholesterin zwischen 8 : 1 und 1 : 1 vorliegen.

25. Pharmazeutische Zusammensetzung nach einem der Ansprüche 21 bis 24, dadurch gekennzeichnet, daß die wäßrige Phase auf einen dem Neutralpunkt benachbarten pH-Wert gepuffert ist.

26. Pharmazeutische Zusammensetzung nach Anspruch 25, dadurch gekennzeichnet, daß die wäßrige Phase mit Phosphat gepuffert ist.

27. Pharmazeutische Zusammensetzung nach Anspruch 20, dadurch gekennzeichnet, daß das injizierbare Medium aus einer wäßrigen Lösung zur Injektion besteht, insbesondere aus einer sterilen isotonischen Salz- oder Glukoselösung.

28. Pharmazeutische Zusammensetzung nach Anspruch 18 oder 19, dadurch gekennzeichnet, daß sie die Verbindung (I) in Verbindung mit zur oralen Administration geeigneten Exzipienten enthält.

29. Pharmazeutische Zusammensetzung nach Anspruch 18 oder 19, dadurch gekennzeichnet, daß sie die Verbindung (I) in Verbindung mit Exzipienten enthält, die eine Verabreichung durch Applikation in die Augenschleimbeutel oder Luftwege, oder vaginale oder rektale Verabreichung ermöglichen.

30. Pharmazeutische Zusammensetzung nach Anspruch 20 oder 21, dadurch gekennzeichnet, daß die Einzeldosen bei der Behandlung von Infektionskrankheiten und einer parenteralen Administration 10 bis 1.000 μg der Verbindung (I) enthalten.

31. Pharmazeutische Zusammensetzung nach Anspruch 28, dadurch gekennzeichnet, daß die Einzeldosen bei einer Behandlung von Infektionskrankheiten und einer oralen Administration 200 bis 20.000 μg der Verbindung (I) enthalten.

32. Laboratoriumsreagenz enthaltend mindestens eine Verbindung gemäß einem der Ansprüche 1 bis 17.